# EUROPEAN PATENT SPECIFICATION

(11) **EP 4 431 195 B1**
(45) Date of publication and mention of the grant of the patent: **01.10.2025**
(21) Application number: 23162482.6
(22) Date of filing: 16.03.2023
(51) Int. Cl.: B09B 3/35, A61L 2/00, A61L 2/18, A61L 2/28, A61L 11/00, B02C 18/18, B02C 23/36, B02C 25/00, G01N 1/10, B02C 18/00, B09B 101/65, A61L 101/36

(54) **VALIDATION METHOD FOR A BIOHAZARDOUS WASTE TREATMENT PROCESS AND APPARATUS THEREFOR**
VALIDIERUNGSVERFAHREN FÜR EIN BEHANDLUNGSVERFAHREN FÜR BIOGEFÄHRLICHE ABFÄLLE UND VORRICHTUNG DAFÜR
PROCÉDÉ DE VALIDATION POUR UN PROCÉDÉ DE TRAITEMENT DE DÉCHETS DANGEREUX BIOLOGIQUES ET APPAREIL ASSOCIÉ

(43) Date of publication of application: 18.09.2024
(73) Proprietor: Envetec Sustainable Technologies Limited, Birdhill, Co. Tipperary V945K02 (IE)
(72) Inventor: BELL, Malcolm, Nenagh (IE); McCRAITH, Alan, Killaloe (IE)
(74) Representative: Edson, Russell Gregory

(56) References cited:
- CN-A- 113 522 470
- US-A- 5 340 536
- US-A1- 2022 250 127

## Description

### Technical Field

The present disclosure relates to the processing of biohazardous waste. In particular, the disclosure relates to the treatment of waste to prepare it to enter a recycling process. More particularly, the disclosure relates to validation testing of such processes, to verify their efficacy.

### Background of the Invention

Currently, biohazardous waste generated from various different sources is typically autoclaved, incinerated and ultimately sent to landfill. As a result, there is currently little to no known recycling of biohazardous waste globally, due to a lack of availability of suitable treatment processes.

More generally, treatment of waste from various sources requires different levels of treatment in order for it to be further processed in any one of various known recycling processes.

Generally, recycling processes are sensitive to contaminants and some degree of cleaning or decontamination, or disinfection of waste materials may be necessary prior to entering a recycling process. Recycling processes may require relatively small particles of waste materials in order to process them effectively. While processes for processing waste exist, methods for validating the efficacy of such processes may be improved upon.

There exists a need for improved methods and apparatus for validating the efficacy of a waste treatment and processing method.

CN113522470 describes a stirring device for food testing, which includes a blade 7 that is supported in a rotatable cylinder 6. Food waste can be introduced into the cylinder 6 and fall therethrough passing via blade 7. Water can be introduced into the device via a nozzle 21. Waste from the device can fall into a sample receiving tube 15 for removal. US2022/250127 describes a smart portable device and system for disposal of sanitary waste. US5340536 describes a method and apparatus for neutralization of biohazardous waste.

With reference to GB2627983A, (earlier application No. GB2303520.7 in the United Kingdom), the applicant has limited the scope of the patent for contracting state GB by submitting separate claims for this state.

### Summary of the Invention

The invention relates to an apparatus according to claim 1 and to a method according to claim 9.

One aspect of improvement identified is a waste processing apparatus in which a test sample can be subjected to the process in a manner which gives a more accurate illustration of the exposure to treatment fluids experienced by the waste in the live waste processing process.

In one aspect a waste processing apparatus is provided, comprising one or more of: a waste processing vessel for holding waste and treatment fluid during processing; a blade arrangement for shredding the waste provided in the waste processing vessel; a waste processing zone in which the waste can be shredded while immersed in a treatment liquid; and at least one process validation module arranged such that it can hold a process validation sample in fluid communication with the treatment liquid in the waste processing zone during operation of the blade arrangement to shred the waste in the waste processing zone while immersed in the treatment liquid.

The at least one process validation module may comprise a sample holding portion for holding a validation sample; and may further comprise a fixing portion, configured to retain the process validation module to the waste processing vessel, which may maintain the sample holding portion in fluid communication with the waste processing zone.

The sample holding portion may be arranged to be detached from the fixing portion and may be arranged to be reattached to the fixing portion. The sample holding portion may be arranged to form a cavity between the sample holding portion and the fixing portion in which a process validation sample can be received.

The waste processing apparatus may further comprise one or more openings through a wall of the cavity of the sample holding portion. The opening or openings may be provided such that fluid from the waste processing zone can pass into the cavity. The at least one process validation module may comprises a plurality of sample holding portions, which may be arranged to be able to hold a plurality of process validation samples in fluid communication with the waste processing zone of the waste processing apparatus.

The waste processing vessel may comprises at least one port providing a passage through a wall of the waste processing vessel. The port may be arranged to facilitate fluid communication between a sample holding portion of the process validation module and the waste processing zone of the waste processing vessel.

The waste processing apparatus may further comprise a fluid passage extending outwardly from the wall of the waste processing vessel. The passage may be arranged to facilitate communication of fluid from the waste processing zone to the sample holding portion of the process validation module. The passage may be arranged to allow at least a portion of the validation module to enter the waste processing zone.

The at least one process validation module may comprise a plurality of sample holding portions. The plurality of sample holding portions may be arranged to be attached to a fixing portion to provide a plurality of sample holding cavities. The cavities may have openings arranged to expose one or more interiors of the cavities to waste treatment fluid from the waste processing zone.

The sample holding portion may project from the fixing portion toward an interior of the waste processing vessel when installed on the waste processing vessel. This may maintain the sample holding portion within or adjacent to the waste processing zone.

The waste processing apparatus may comprise a plurality of process validation ports. The plurality of process validation ports may be arranged to receive at least one process validation module or modules. The process validation module or modules may be arranged to retain a plurality of sample holding cavities of the process validation modules in fluid communication with the waste processing zone of the waste processing vessel.

The at least one process validation module may be arranged to seal a process validation port of the waste processing vessel. This may be done while retaining a sample holding portion of the process validation module in fluid communication with the waste processing zone of the waste processing vessel.

One aspect of improvement identified is a validation method in which a test sample is subject to the process in a manner which gives a more accurate illustration of the exposure to treatment fluids experienced by the waste in the live process.

In one aspect, a validation method is provided, comprising one or more of the following steps:
shredding waste using a blade arrangement in a waste processing vessel in which the waste is shredded and exposed to a waste treatment fluid during a waste processing cycle time; and
exposing a process validation sample comprising a process efficacy indicator to the waste treatment fluid, to which the waste is exposed, during the waste processing cycle time; and
analysing the effect of the waste treatment fluid on the process efficacy indicator to determine an efficacy of the waste processing and liquid treatment process.

The validation process of the present disclosure provides improved means for testing the efficacy of a waste shredding and liquid treatment process. In particular, improved ways of ensuring a validation test sample is exposed to the treatment fluid used in the process in a way which is more representative of the exposure of the waste being processed to the treatment liquid.

As used herein, cycle time is the time elapsed between the beginning of the mechanical processing, or shredding, of the waste in the presence of the treatment fluid, and the end of the cycle. It therefore begins when the waste and the treatment fluid are present in the waste processing vessel together, and the blade arrangement begins shredding of the waste. It ends when the cycle ends and the treated waste and treatment fluid are emptied out of the waste processing vessel 120.

It may be the case that the waste being shredded and the process validation sample are immersed in treatment fluid for the duration of the cycle time.

It may be the case that the process efficacy indicator comprises a biological indicator. As used herein, the term "biological indicator" relates to microorganisms which are suitable for determining the efficacy of the waste shredding and liquid treatment process and includes spores produced by such microorganisms. As used herein, the term "spore" takes its usual meaning in the art and relates to dormant structures that certain fungi, plants, and bacteria produce in response to unfavourable environmental conditions.

The biological indicator may comprise one or more bacterial spores, a virus, vegetative bacteria, or a combination thereof. It may be the case that the one or more bacterial spores is derived from *Geobacillus* spp., *Alicyclobacillus* spp., *Bacillus* spp., or combinations thereof. It may be the case that the one or more bacterial spores is derived from *Geobacillus stearothermophilus.*

The biological indicator may be secured to a substrate, which may be comprised in the process validation sample(s) 701. The substrate may comprise a material which is compatible with and does not influence the liquid treatment process. The substrate may comprise paper, glass fibers, plastics, ceramics, stainless steel, and metal oxides. It may be the case that the substrate comprises stainless steel. The substrate may be in the form of a strip or a disc.

It may be the case that the biological indicator is secured to the substrate by the use of an inert adhesive. It may be the case that the inert adhesive comprises a polymeric adhesive. It may be the case that the inert adhesive comprises a hydrogel. As used herein, the term "hydrogel" takes its usual meaning and relates to a gel composed of one or more polymers suspended in water. The hydrogel may comprise a natural polymer, a synthetic polymer, or a combination thereof.

The waste processing apparatus may be arranged to be configurable in any one of:
a first configuration to receive waste through the waste receiving opening of the waste processing vessel;
a second configuration to close the waste receiving opening of the waste processing vessel, to shred the waste in the waste processing vessel while the waste is immersed in a treatment fluid; and
a third configuration to release the waste and treatment fluid from the waste processing vessel to a conveyor for removal of the waste and treatment fluid from the apparatus.

The waste processing apparatus may be arranged to permit user access to the validation sample receiver when configured in a validation sample changing configuration; and may be arranged to prevent user access to the validation sample receiver in the second configuration. The validation sample changing configuration may be achieved in any one of the above first, second or third configurations.

### Brief Description of the Drawings

Further features and advantages of the present invention will become apparent from the following description of embodiments thereof, presented by way of example only, and by reference to the drawings, in which:
Figure 1 shows a waste processing apparatus according to one example;
Figure 2A shows detail of a locking pin arrangement for the apparatus of Figure 1;
Figure 2B shows the waste processing apparatus from an opposite angle;
Figure 3 shows a waste processing vessel of the disclosure;
Figure 4 shows an exploded view of the waste processing vessel of Figure 3;
Figures 5A and 5B show a fluid delivery arrangement for the apparatus of Figures 1 and 2;
Figures 6A to 6C show the waste processing apparatus in different operating configurations;
Figures 7A and 7B show a process validation module for use in the apparatus;
Figure 8 is a perspective view of a shredding assembly;
Figure 9 is an exploded view of a shredding assembly;
Figure 10 is a perspective view of a rotatable blade array;
Figure 11 is an end view of a rotatable blade array;
Figure 12 is a perspective view of a rotatable blade;
Figure 13 is a perspective view of a lower fixed blade array;
Figure 14 is a perspective view of a fixed upper blade; and
Figure 15 is a bottom perspective view of an upper fixed blade;
Figure 16 shows a schematic diagram of a waste processing apparatus;
Figure 17 shows an example of a method of controlling a waste processing apparatus;
Figures 18A and 18B show an example of a waste cycle process;
Figures 19A and 19B show an example of a validation cycle process; and
Figure 20 shows an example of a reject cycle process.

### Detailed Description

A large amount of biologically hazardous waste is generated from different sources, including clinical facilities, diagnostic laboratories, and research institutes. This waste requires specialised treatment. Such treatment typically occurs off-site and therefore also requires specialised transportation and handling ahead of treatment, which can increase costs, poses a contamination risk, and increases the carbon footprint. Treatment of hazardous waste, biohazardous medical waste, or other regulated waste in particular, often requires energy intensive processes, such as transport for off-site processing, along with autoclaving or incineration, whose emissions, water and energy usage, and environmental impact can be improved upon.

A particular aspect of biologically hazardous (biohazardous) waste is that the risks relating to contaminants present in or on the waste need to be handled throughout its processing, until such time as it is considered safe for contact with humans or the environment.

There is therefore a need to monitor and confirm the efficacy of the treatment process used to mitigate the risks associated with waste that has not been effectively disinfected.

The inventors have identified particular areas for improvement in the processing of waste, in particular contaminated, hazardous, or biohazardous waste.

In particular, the inventors have devised improved methods for testing the efficacy of treatment of waste during a shredding process and apparatus therefore.

As used herein, the term "biohazardous waste" (also commonly referred to as "infectious waste") takes its usual meaning in the art and relates to waste that has been contaminated with potentially infectious agents, or other materials that are deemed to be a threat to either public health or to the environment. For example, biohazardous waste includes medical waste generated in laboratory or clinical settings (such as hospitals), which has been contaminated with blood, body fluids, and/or cell lines from humans or animals. Such waste can also sometimes fall within the terms medical waste or regulated waste, depending on the different national regulatory definitions. While aspects of the presently disclosed methods and apparatus have been developed with biohazardous waste in mind, aspects of the methods can be applied to validation of waste processing methods for other applications and types of waste, whilst still giving an indication of the efficacy of the waste processing process.

### Waste Processing Apparatus

The waste processing apparatus described herein provides a number of advantageous features and aspects. One advantageous feature is the system being able to perform shredding of waste while the waste is submersed or immersed in a reservoir of the treatment fluid, which provides simultaneous treatment, such as sterilisation or disinfection, simultaneously with the shredding of the waste. This can reduce overall processing times and improve the reliability of the shredding and disinfection or sterilisation, or other cleansing process. The treatment fluid may comprise liquid, such as water, and may comprise a treatment agent. The treatment fluid may be in the form of a homogenous liquid mixture or a solution. The treatment agent may be provided in solid, liquid or gel form and may be combined with a diluent, which may be a liquid diluent such as water.

One aspect of improvement identified is the combined shredding and treatment of waste in a closed-bottomed receiver, in which a treatment fluid such as a liquid treatment of the types described herein is present, over an extended period, such that shredding and cleaning and/or disinfection of the waste takes place simultaneously. In one aspect, a waste processing apparatus is provided, comprising one or more of the following features: a waste processing vessel for holding waste and treatment fluid during processing; and a blade arrangement provided in the waste processing vessel, for shredding the waste; the waste processing vessel comprising a waste processing zone, in which fluid can be retained, such that waste provided to the waste processing vessel can be shredded by the blade arrangement while immersed in treatment fluid in the waste processing zone.

The waste processing apparatus of the invention provides an advantageous arrangement in which during the shredding operation, the waste being shredded is immersed in treatment fluid. This is an improvement compared to prior art systems in which, in order for repeated passes through a standard vertical shredding device to take place, waste has to be drawn out of treatment fluids and re-passed through a vertical shredder such that treatment fluid generally runs off the waste being shredded and treated while it passes once more through the shredding arrangement.

The blade arrangement may comprise a fixed blade array and a rotatable blade array, at least a portion of the fixed blade array preferably being disposed in the waste processing zone, such that waste material can be shredded against the fixed blade array, by the rotatable blade array, under the surface of treatment fluid retained in the waste processing zone. The waste processing vessel may comprise a substantially fluid-tight closed-bottomed container, configured such that a treatment fluid can be retained in the waste processing zone during waste processing, optionally at a substantially constant volume. The waste processing apparatus may further comprise a waste receiving opening, and a closure arrangement for closing the waste receiving opening. The closure may be arranged to retain treatment fluid and waste in the waste processing vessel during the simultaneous shredding and fluid treatment of the waste in the vessel.

The waste processing vessel may be movable to and from, and retainable in, at least one of: a first position in which the waste processing vessel is oriented towards a waste filling position to facilitate loading of waste into the waste processing vessel; a second position, in which waste treatment fluid can be provided to, and remains in, the waste processing vessel; a third position, in which waste and/or treatment fluid can be tipped out of the waste processing vessel. The waste processing apparatus may further comprise positional locking means configured to lock the waste processing vessel in at least one of the first, second or third positions. The positional locking means may comprise one or more locking mechanisms comprising an actuator and a pin. The actuator may be configured to move the pin to engage with the waste processing vessel so that the waste processing vessel is locked or lockable in one or more of the first, second and third positions. The waste processing vessel may comprise a vessel rotation axis about which the vessel may be rotated to occupy one or more of the first, second or third positions. The waste processing apparatus may further comprise a conveying means to which treated and shredded waste may be tipped from the waste processing vessel. The conveying means may be configured to perform conveying of the waste away from the waste processing vessel. The blade arrangement may comprise a rotatable blade array rotatable about a first axis. The apparatus may comprise one or more of: first drive means for driving the rotatable blade array about the first axis; and second drive means configured to drive the waste processing vessel about the vessel rotation axis. The first axis may be parallel to the vessel rotation axis. The apparatus may comprise treatment agent delivery means configured to deliver a treatment agent to the waste processing vessel. The treatment agent delivery means may be comprised in the closure arrangement.

The treatment agent delivery means may comprise first delivery means configured to deliver a treatment agent to the waste processing vessel. The treatment agent delivery means may comprise second delivery means configured to deliver a diluent to the processing vessel. The first delivery means may comprise a liquid delivery system, comprising pumping means and flow control means configured to deliver treatment liquid from a treatment liquid container to the waste processing vessel. The second delivery means may comprise a liquid delivery system comprising flow control means, and may be arranged to be connected to an external fluid supply, such as a mains water supply.

The waste processing apparatus may further comprise a chassis comprising outer walls which define an outer enclosure. The waste processing vessel, the waste receiving opening and the closure arrangement may be enclosed within the outer walls. The outer walls may comprise a loading hatch, which may be arranged to be allowed to be opened to load the waste processing vessel and which may be arranged to be kept closed during waste processing in the waste processing vessel.

A further aspect provides a method of treating waste, the method comprising one or more of the following steps: addition of waste and a treatment fluid to a waste processing vessel of a waste processing apparatus, wherein the waste processing vessel comprises a waste processing zone, and wherein the treatment fluid is retained in the waste processing zone; activation of a blade arrangement, such that the waste in the waste processing vessel can be shredded by the blade arrangement while in contact with or while immersed in the treatment fluid retained in the waste processing zone of the waste processing vessel; deactivation of the blade arrangement after a pre-determined cycle time. As used herein, cycle time is the time elapsed between the beginning of the mechanical processing, or shredding, of the waste in the presence of the treatment fluid, and the end of the cycle. It therefore begins when the waste and the treatment fluid are present in the waste processing vessel together, and the blade arrangement begins shredding of the waste. It ends when the cycle ends and the treated waste and treatment fluid are emptied out of the waste processing vessel. After shredding by the blade arrangement, the method may further comprise addition of a neutralising agent after shredding by the blade arrangement. Addition of a neutralising agent may be carried out after a predefined period of shredding.

A further aspect provides use of a solution comprising a disinfectant and a diluent in the treatment of biohazardous waste, wherein the disinfectant comprises peracetic acid and hydrogen peroxide. The disinfectant may comprise peracetic acid in the range of from 10% to 20% based on the total weight of the disinfectant and hydrogen peroxide in the range of from 15% to 30% based on the total weight of the disinfectant. The diluent may comprise water. The solution may be of a concentration in the range of from 0.5% (v/v) to 5% (v/v). As used herein, the term "solution" relates to liquid mixture in which the minor component(s) (i.e., the treatment agent) is uniformly distributed throughout the major component (i.e., the diluent). The treatment agent in the form of a solution may also be referred to as the treatment solution. The diluent may comprise, consist essentially of or preferably consist of, water. Advantageously, the process may use peracetic acid (CH₃CO₃H, also commonly referred to as peroxyacetic acid) and hydrogen peroxide in the waste shredding and disinfecting process. This and other such fluids as described herein can improve the efficiency and efficacy of the treatment of the waste simultaneously with the shredding process. The design of the waste processing apparatus is particularly suited to the treatment of hazardous waste, such as biohazardous waste or other waste containing harmful or toxic substances or infectious agents, from which a user must be protected before during and after processing of the waste. Advantageous aspects of the design of the blade arrangement used in the process for improved shredding efficiency and flexibility are also described. Advantageous control schemes and methods of operation of the process are also described, which improve the efficacy, user experience and user safety and overall process efficiency of the device.

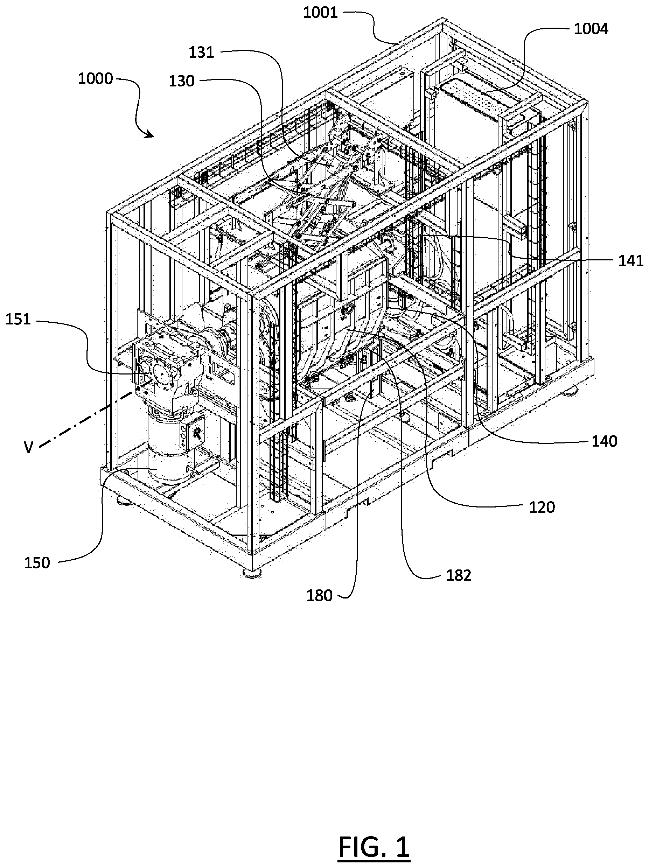

With reference to Figure 1, there is shown a waste processing apparatus 1000 according to the disclosure. The apparatus comprises a chassis 1001, comprising a series of struts and beams arranged to mechanically support and surround the working components of the waste processing apparatus. A plurality of wall portions, such as panels, doors or plates may be arranged on or around the chassis. In this manner an enclosure for the working components of the apparatus can be provided. A waste processing vessel 120 may be provided within an enclosure, such as that provided by the chassis 1000. There may further be provided a control system enclosure 1004, which may include power electronics, signal and power distribution modules and computing and/or processing devices for the control of the apparatus. A closure mechanism 130 may be provided for moving the closure arrangement, such as a lid, which may be termed a press-plate, for closing a waste receiving opening provided in the waste processing vessel 120. The closure mechanism may comprise a closure mechanism actuator 131, which may be a linear or rotational actuator. It may further comprise a closure mechanism sensor for sensing a position of the closure mechanism. This can enable the detection of a position of the lid or press plate 170 to verify whether it is in an open or a closed position. The waste processing vessel 120 may be rotatable about a vessel rotation axis V. Vessel displacement means may be provided to displace vessel 120 between different operational positions. Vessel rotation drive means 140 may be provided to drive the vessel 120 in rotation about an axis V. The vessel 120 may be moved between different configurations to enable different operations, such as loading of the vessel, closure of the vessel for processing, and emptying of the vessel of processed waste and treatment fluid. This movement between different configurations may be provided by rotation about the axis V. However, other displacement means and types of displacement of the vessel between the different positions may be envisaged, such as linear, translational, a combination of rotation and translation among others within the knowledge of the skilled reader. The vessel drive means 140, which may be rotational drive means, may be mounted to a part of the chassis 1000 and may drive the vessel via a gearbox 141, which may also be mounted to a part of the chassis 1000. When rotatable, the axis of rotation of the vessel may also be the same axis about which a drive input for a blade arrangement provided in the vessel is provided, as will be described in relation to later figures. Blade rotation drive means 150 may be provided in the form of a motor. The motor 150 may drive the blades in rotation via a gearbox 151. One or more of motors 140 and 151 may be mounted with its axis of rotation perpendicular to the axis of rotation of the waste processing vessel 120 and/or the axis of rotation of the blade arrangement. This can enable a more compact overall arrangement of the waste processing apparatus within its enclosure.

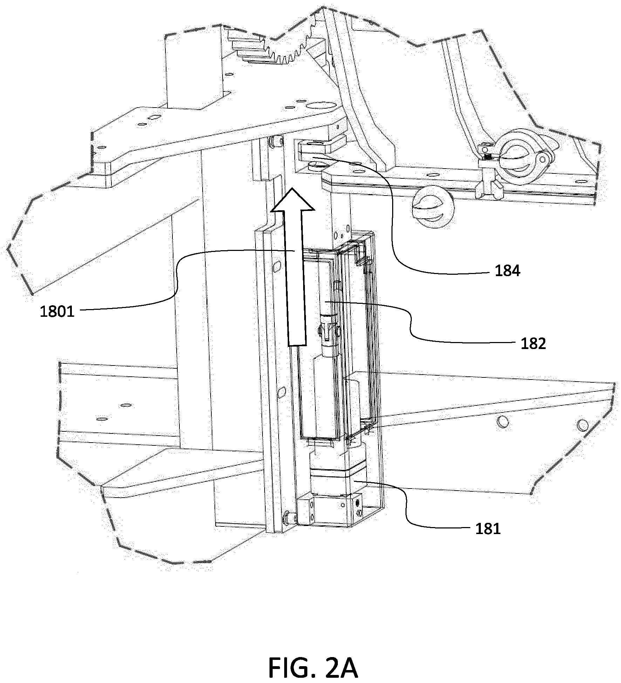

Whether rotationally mounted and rotationally displaceable or not, it can be advantageous to lock the waste processing vessel 120 in position during the waste processing cycle. This inhibits vibration of the waste processing vessel 120 which may be caused by rotation of the blades. To this end, positional locking means may be provided to the vessel to retain the vessel in place within the chassis 1001 and prevented generally from rotational and/or translational movement during the processing cycle. Examples of positional locking means may include friction brakes, bolts clamps or other means for retaining the vessel in place. In the present example, positional locking means are provided by locking mechanisms 180. Each locking mechanism 180 includes a linear actuator 181 and a pin 182, as illustrated in Figure 2A. The linear actuator 181 is configured to move the pin 182 towards the vessel 120 as shown by the arrow 1801, so that the pin 182 extends from a chassis 183 of the locking mechanism 180. A corresponding opening 184 can be provided in the vessel 120, such that the pin 182, when extended, extends into the opening 184 to engage with the vessel 120 and thereby lock the vessel 120 in place relative to the chassis 1001. One, two or more locking mechanisms 180 may be provided to the apparatus 1000 for improved stability, and may be arranged below the vessel 120, such as is illustrated in Figure 2A, and may secure the vessel by engaging with openings in the vessel provided at or adjacent the closed bottom side of the vessel 120.

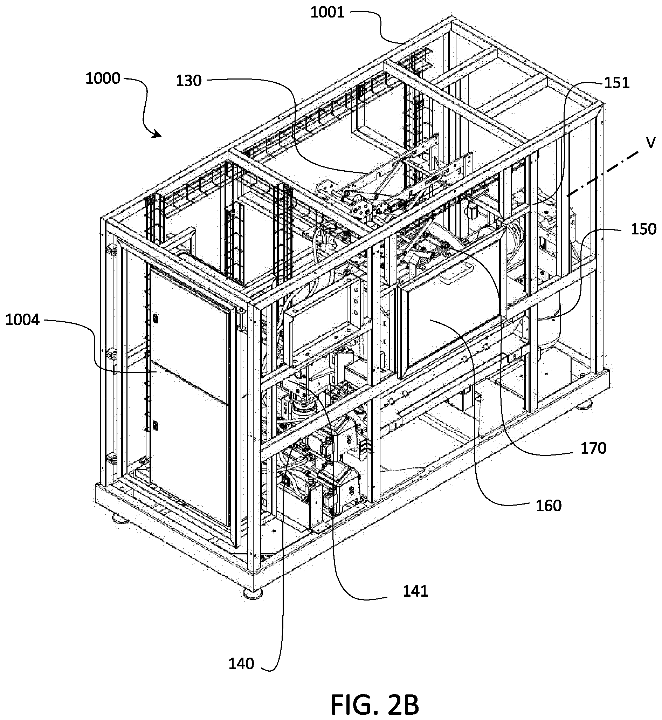

Turning to Figure 2B, a waste loading hatch 160 may be provided which can be opened for loading of the waste processing vessel 120 with waste and which may be closed in order to enclose the waste processing vessel during a waste processing cycle of the apparatus. This can help to protect users from leakage or spillage of contaminants or treatment fluids during the processing cycle. In the present example, a handle 161 is provided on the waste loading hatch 160 to assist a user with manually opening the hatch 160. In other examples, the opening of the hatch may be automated e.g. to allow a robot or other automated systems or handlers to load the waste processing vessel 120.

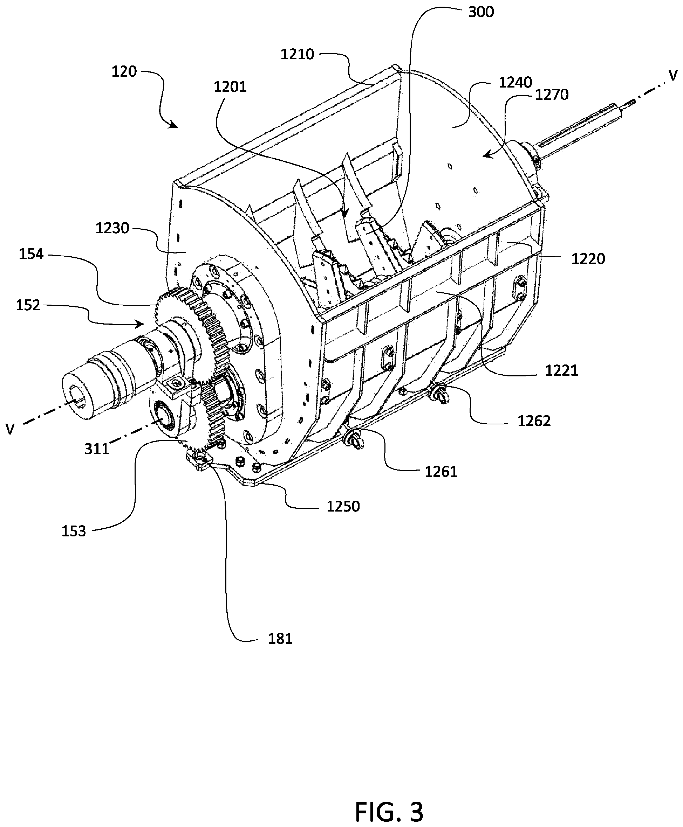

Figure 3 illustrates a waste processing vessel 120 outside of the enclosure illustrated in figures 1 and 2. The waste processing vessel may comprise at least one wall 1210, 1220, arranged to form a waste processing chamber 1201 into which waste can be placed for shredding and fluid treatment by the apparatus. The at least one wall may comprise a first side wall 1210, a second side wall 1220, a first end wall 1230 and a second end wall 1240. One or more of the at least one walls may comprise one or more strengthening ribs 1221 to provide strength to the wall to resist shredding and compression forces which may be applied to the wall during rotation of a blade arrangement 300 located in the waste processing vessel 120. A bottom wall 1250 may be provided. The waste processing chamber 1201 may be provided in the form of a closed-bottomed container, in which an amount of fluid may be retained such that waste residing in a bottom of the fluid can be maintained in an immersed state under an amount of fluid provided to the waste processing vessel 120.

The waste processing vessel 120 may be provided with one or more ports 1261, 1262 for receiving process validation samples. The one or more ports 1261, 1262 may be arranged to be externally accessible with respect to the waste processing vessel, so that the user can access the ports to install and/or remove process validation samples from the waste processing vessel. It can be advantageous to have the one or more ports 1261, 1262 located in a waste processing zone of the waste processing vessel 120. The one or more validation ports 1261, 1262 may be arranged to provide an opening through one or more walls of the waste processing vessel, such as wall 1220, and may be provided in a lower region of such wall so as to be located in a region of the waste processing vessel in the waste processing zone Z (Figure 3), which may be a lower region of the waste processing vessel 120 when it is oriented in its waste processing position. The one or more validation ports 1261, 1262 may be provided with blanking plates when the validation process is not being carried out and the vessel 120 is being used for waste processing. Such blanking plates may be replaced with validation sample holders when a validation cycle is to be carried out. A validation cycle is a process in which a normal shredding and treatment cycle is run in the machine, and validation samples are held in the waste processing zone of the waste processing vessel, such that the validation sample contained in a validation sample holder is exposed to the same degree of treatment fluid as the waste in the waste processing zone. This can provide a reliable validation of the waste processing cycle, as will be described in more detail in later sections.

The blade arrangement 300 may be driven in rotation about a blade rotation axis 311, optionally via a gear arrangement 152. Gear arrangement 152 may comprise first 153 and second 154 gears arranged to transfer a drive from the drive source such as the drive source 152 the blade arrangement 300. In one arrangement as shown, the vessel rotation axis V may be substantially parallel to the blade rotation axis 311. The gear arrangement 154 may be arranged to transfer a drive between the vessel rotation axis V and the blade rotation axis B. As can be seen, drive for both the blade arrangement 300 and rotation of the waste processing vessel 120 may be provided on the same axis, such that the waste processing vessel is arranged to rotate around a same axis V along which the drive provided for driving the blade arrangement 300.

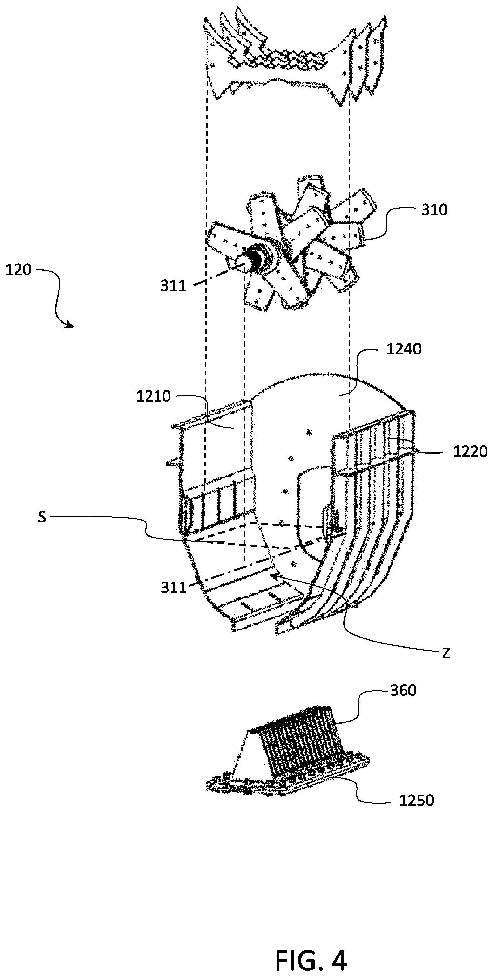

An exploded view of the waste processing vessel 120 can be seen in Figure 4. As can be seen, a base plate 1250 may comprise an array of fixed blades, which will be described in greater detail in later sections. The base plate 1250 may cooperate with the at least one wall 1210, 1220 and at least one end wall 1230, 1240, to form a vessel capable of containing a fluid.

The waste processing vessel 120 comprises a waste processing zone Z. When the vessel is in its operating state, the rotational axis 311 will be at the lower 311 position as shown in Figure 4, with the base plate 1250 installed. The vessel 120 is arranged such that when a predetermined amount of liquid is provided to the vessel, at least a portion of the fixed blade array 360 and the rotatable blade array 310 is in the waste processing zone Z. The zone Z may, for example, be filled with treatment liquid to a level S. As will become apparent, therefore, when the rotatable blade array 310 is rotated, waste will be shredded in the zone Z whilst immersed in treatment fluid. It is beneficial if at least a portion of a region of overlap between the rotatable blade array 310 and the fixed blade array 360 is in the Zone Z and so can be under the surface of the treatment fluid during processing.

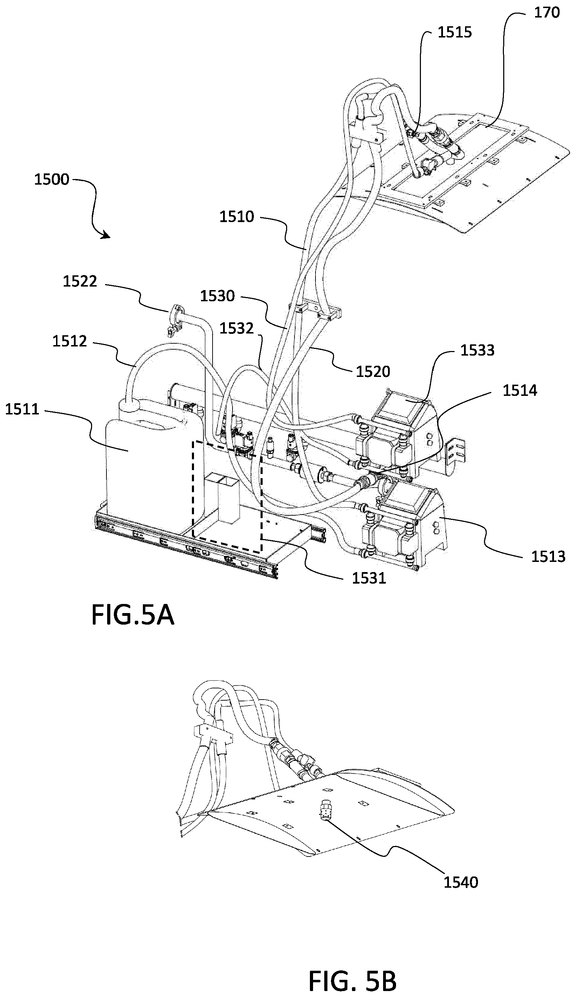

Figure 5A illustrates a fluid delivery apparatus 1500, which may be integrated into the waste processing apparatus 1000. The delivery apparatus 1500 may comprise first fluid delivery means 1510 and second fluid delivery means 1520. A third fluid delivery means 1530 may be included. First fluid delivery means 1510 may be for delivering a treatment fluid, which may be any treatment fluid discussed herein, such as chemical cleaning fluid, disinfectant, sterilisation fluid, a detergent or any other such treatment fluid. The second fluid delivery means 1520 may be for delivering a diluent. The third fluid delivery means 1530 may be for delivering a neutralising agent. As used herein, a "diluent" relates to a substance that can have a diluting effect on the treatment fluid. The diluent may comprise, consist essentially of, or preferably consist of water. First fluid delivery means may comprise a treatment fluid container 1511 and a treatment fluid delivery line 1512. The treatment fluid delivery conduit 1512 may be arranged to transfer treatment fluid from the fluid container 1511 to a pumping means 1513. A suitable fluid delivery conduit may be a flexible or solid pipe or tube, for example. Treatment fluid pumping means 1513 may be any suitable form of pump, but is advantageously a diaphragm pump. A first fluid control means 1514, such as a valve, which may be controlled by a solenoid, may be provided in the first 1510 and/or second 1520 fluid delivery means. A flowmeter 1515 may be provided in the first fluid delivery means 1510, and/or may also be provided in the second fluid delivery means 1520. The first and/or second fluid delivery means can deliver treatment fluid and/or diluent to the closure arrangement, such as lid 170, which is arranged to close the waste processing vessel 120. This delivery may occur via a spray nozzle 1540 provided on an inside surface of the lid 170. The second fluid delivery means 1520 may comprise an inlet 1522. The inlet 1522 may be configured for connection to a diluent source. The diluent source may be from a container, or may be from an external water supply such as a mains water supply. The inlet 1522 may pass via a switching means such as the valve 1514 or a further separate valve provided for this function, and may be subsequently connected to the closure arrangement such as lid 170, to deliver diluent into the waste processing vessel 120. The third fluid delivery means 1530 may be configured similarly to the first fluid delivery means, having features equivalent to one or more, or all of, the features of the first fluid delivery means 1510. In particular, a flowmeter may be provided in the third fluid delivery means 1530. The third fluid delivery means may deliver a fluid provided in a container 1531, similarly configured to the container 1511 of figure 5A. The container 1531 is represented schematically by dashed lines in figure 5A to enable features behind it in that view to be seen. Fluid may be delivered from the container 1531 via a fluid delivery conduit 1532. The fluid delivery conduit 1532 may pass via a pumping means 1533. The pumping means 1533 may have similar, or same features, as those of the pumping means 1513. Fluid delivery conduit 1532 is connected to the closure arrangement 172 deliver fluid therethrough. Passing via the lid is of course optional and the treatment fluid and/or diluent could be delivered to the waste processing vessel by other paths, such as through walls or a base of the vessel. While one means of delivering treatment fluid, treatment agents, or neutralisation fluids or agents as disclosed herein to the vessel 120 is via the described fluid delivery means, it will be understood that treatment agents can be provided in any suitable manner, in liquid, gel, solid or powdered form.

It may be the case that the temperature of the diluent is in the range of from about 1 °C to about 40 °C. It may be the case that the temperature of the diluent is in the range of from about 10 °C to about 30 °C.

### Operating Sequence

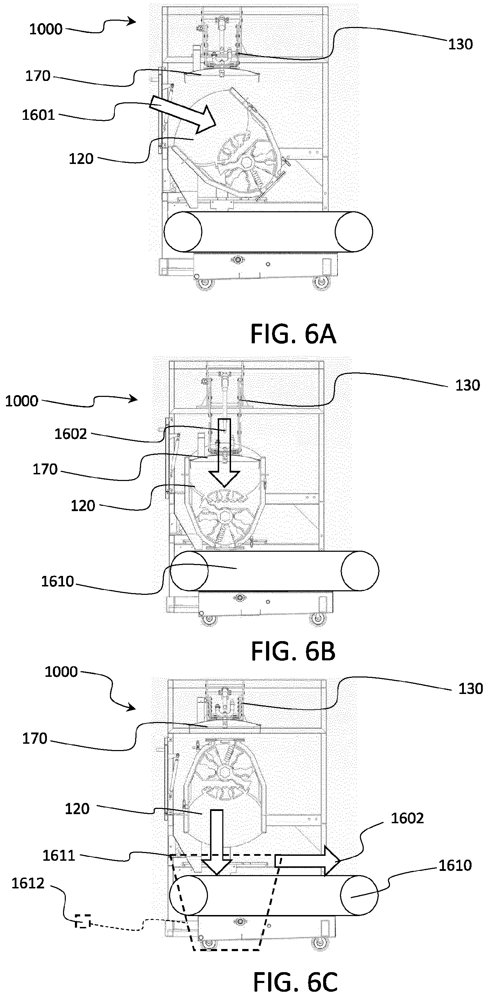

Operation of the waste processing apparatus will now be described with reference to Figures 6A to 6C. As will be understood, the waste processing vessel can be arranged in a plurality of different positions for carrying out different stages of the operation. A first position of the waste processing vessel may be a waste loading position. An example of such a waste loading position is shown in Figure 6A. In the waste loading position, the closure arrangement 170 is in an open configuration. Closure arrangement 170 may be mounted to the chassis or enclosure within which the waste processing vessel 120 is disposed. The closure arrangement 170 may therefore not be directly connected to the waste processing vessel. In the example illustrated, the closure arrangement 170 is therefore retracted from and distanced from the waste processing vessel.

An operating sequence may start by orienting the waste processing vessel 120 in the first position, in which waste may be loaded into the waste processing vessel 120. This may include orienting the waste receiving opening 1270 of the waste processing vessel 120 towards a loading position, and may include arranging the waste receiving opening 1270 towards the loading hatch 160 to enable waste to be loaded through the loading hatch 160 into the waste processing vessel 120 in the direction of arrow 1601 as shown in Figure 6A.

The waste processing vessel 120 may be moved to a second position. The second position can involve orienting the waste receiving opening 1270 towards a more upward or more vertical orientation relative to the first position. An example of such a second position is shown in Figure 6B. The waste receiving opening is oriented upwardly so that fluids added to the vessel remain in the vessel due to gravity and do not tend to pour or spill from the vessel 120. In this orientation, the closure arrangement 170 may be advanced towards the waste processing vessel 120 to close or substantially close the waste receiving opening, such as by advancing the closure arrangement 170 in the direction of arrow 1602 in Figure 6B. This may help prevent waste and/or treatment fluid and/or diluent and/or vapour from escaping from the waste processing vessel 120. An amount of treatment fluid and/or diluent may then be added to the waste processing vessel via first 1510 and/or second 1520 fluid delivery means. With the waste processing vessel in a closed configuration, the rotatable blade array is rotated so as to carry out a combined shredding and mixing operation, to shred waste between the rotatable blade array and the fixed blade array(s) and also to mix the waste with the treatment fluid in the bottom of the waste processing vessel 120.

When the waste processing operation is complete, the closure mechanism 130 may be actuated to open the closure arrangement 170 and the waste processing vessel 120 may be moved to a third position, in which the opening 1270 faces at least partially, or fully, downwards. In this orientation, waste and/or treatment fluid may be tipped out of the waste processing vessel 120 through the opening 1270. An example of such a third position is shown in Figure 6C. This tipping action may deliver the waste and/or treatment fluid to a conveying means. Such conveying means may include a linear conveyor 1610, or could alternatively be a rotational conveying means such as an auger. Fluid may be able to drain from the waste onto, and drain off or out of, the conveying means. The waste may then be conveyed, such as in the direction of arrow 1603 in Figure 6C, for discharge from the waste processing apparatus 1000. The waste may be delivered to a further conveying means, for example, for conveying to a further location, such as in an upward direction, to allow the waste to be delivered to a receiving vessel such as a waste bin or other container. If a shredding process is aborted, the waste may instead be ejected into a reject bin as shown by dashed lines 1611, which may carry a reject bin identifier plug 1612, for plugging into a reject bin identifier socket, to indicate to the controller 410 of the apparatus 1000 that the reject bin 1611 is in place.

### Chemical Process

The treatment fluid may comprise liquid, such as water, and may comprise a treatment agent. The treatment fluid may be in the form of a homogenous liquid mixture or a solution. The treatment agent may be provided in solid, liquid or gel form and may be combined with a diluent, which may be a liquid diluent such as water. The treatment fluid may comprise a disinfectant. The treatment fluid may comprise a disinfectant and a diluent. The treatment fluid may comprise a solution comprising a disinfectant and a diluent.

As used herein, the term "solution" relates to liquid mixtures in which the minor component(s) (i.e., the disinfectant) is uniformly distributed throughout the major component (i.e., a diluent as described above with respect to the waste processing apparatus). The diluent may comprise, consist essentially of or preferably consist of water. The solution as described herein may also be referred to as the "disinfectant solution".

The disinfectant solution may be provided via the first 1510 and/or second 1520 fluid delivery means. It may be the case that at least some of the total volume of the diluent is provided to the vessel 120 via the second fluid delivery means. The disinfectant solution may therefore be provided in a 'pre-mixed' state via only one fluid delivery means into the waste processing vessel 120. Alternatively, different components of the disinfectant solution may be delivered separately into the waste processing vessel via different fluid delivery means. The diluent may comprise, consist essentially of or preferably consist of water.

In one example, in a first step, a first component, such as the disinfectant, is provided to the waste processing vessel 120 via the first fluid delivery means 1510 in a desired amount. A second component such as the diluent may be delivered to the waste processing vessel in a second amount, to achieve a desired mixture, with desired relative concentrations, of the first and second components. In a first example, the diluent and disinfectant or other component(s) are added to the vessel simultaneously. However, it will be appreciated that in other examples, the diluent may be added to the waste processing vessel in a first step and followed by the disinfectant or other component, or vice versa. Adding the diluent first may avoid high concentrations of disinfectant in the vessel 120, for example.

As used herein, the term "disinfectant" relates to a chemical agent, which is able to inactivate viruses, bacteria, and other microorganisms that can cause infection and disease.

The disinfectant may comprise peracetic acid and hydrogen peroxide. As used herein, peracetic acid may also be referred to as peroxyacetic acid.

Without being bound by theory, peracetic acid and hydrogen peroxide demonstrate a synergistic effect when used in combination against infectious agents (such as microorganisms) present in biohazardous waste. For example, hydrogen peroxide can erode the outer layer of the microorganism, allowing peracetic acid to more efficiently degrade the proteins and nucleic acids contained within.

A combination of peracetic acid and hydrogen peroxide results in an equilibrium reaction as outlined below in Equation (I) below:

Equation (I): CH₃C(=O)OH + H₂O₂ 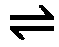 CH₃C(=O)OOH + H₂O

The term "equilibrium reaction" takes its usual meaning and relates to a chemical reaction in which two opposing chemical reactions are occurring simultaneously. The equilibrium may be shifted by changing various parameters, such as the reaction temperature, pressure, incorporation of a catalyst, or the composition of the reactants/products.

In Equation (I) Acetic acid and hydrogen peroxide react to generate peracetic acid and water. Peracetic acid in the presence of water will degrade to hydrogen peroxide and acetic acid over time.

Peracetic acid may be present in the range of from about 10% to about 20% based on the total weight of the disinfectant. It may be the case that peracetic acid is present in the range of from about 12% to about 17% based on the total weight of the disinfectant. At least 13% peracetic acid may be present based on the total weigh of the disinfectant. At least 14% peracetic acid may be present based on the total weigh of the disinfectant. At least 15% peracetic acid may be present based on the total weigh of the disinfectant. At least 16% peracetic acid may be present based on the total weigh of the disinfectant.

Hydrogen peroxide may be present in the range of from about 15% to about 30% based on the total weight of the disinfectant. It may be the case that hydrogen peroxide is present in the range of from about 18% to about 25% based on the total weight of the disinfectant. At least 20% hydrogen peroxide may be present based on the total weigh of the disinfectant. At least 21% hydrogen peroxide may be present based on the total weigh of the disinfectant. At least 22% hydrogen peroxide may be present based on the total weigh of the disinfectant. At least 23% hydrogen peroxide may be present based on the total weigh of the disinfectant.

Peracetic acid and hydrogen peroxide present at the abovementioned weight percentages drives the equilibrium reaction shown in Equation (I) towards the formation of peracetic acid and water, without the need for the addition of a strong acid catalyst, such as sulfuric acid. It is desirable to avoid the need for addition of strong acid catalysts, as they are corrosive and difficult to handle.

Moreover, without being bound by theory, driving the equilibrium reaction towards the formation of peracetic acid provides greater disinfectant efficacy in a shorter space of time compared to known disinfectants. For instance, the disinfectant according to the disclosure gives rise to a log reduction over a short cycle time. As used herein, the term "log reduction" takes its usual meaning in the art and relates to the mathematical term used to express the relative number of microorganisms (such as bacteria and viruses, or bacterial or fungal spores) that are inactivated by a disinfectant. The Log reduction value indicates the percentage kill rate of microorganisms. Log reduction can be measured using any known technique. For instance, a biological indicator as described herein below can be exposed to the treatment fluid from the waste processing zone of the waste processing vessel during a waste processing cycle of the waste processing apparatus. The biological indicator can then be extracted following completion of the processing cycle and placed in a culture medium and incubated for a pre-determined time (depending on the nature of the biological indicator in question and its respective desired culture conditions). Following incubation, absence of growth of microorganisms in the culture medium may indicate an efficient disinfection process. For example, an absence of growth may indicate a 99.9999% reduction in the number of active microorganisms and/or a log reduction in the number of active microorganisms. In instances where the biological indicator comprises about 10⁴ microorganisms prior to the treatment process, a log 4 reduction following treatment would be indicative of an efficient disinfection process. In instances where the biological indicator comprises about 10⁵ microorganisms prior to the treatment process, a log 5 reduction following treatment would be indicative of an efficient disinfection process. In instances where the biological indicator comprises about 10⁶ microorganisms prior to the treatment process, a log 6 reduction following treatment would be indicative of an efficient disinfection process. In instances where the biological indicator comprises about 10⁷ microorganisms prior to the treatment process, a log 7 reduction following treatment would be indicative of an efficient disinfection process. In instances where the biological indicator comprises about 10⁸ microorganisms prior to the treatment process, a log 8 reduction following treatment would be indicative of an efficient disinfection process. In instances where the biological indicator comprises about 10⁹ microorganisms prior to the treatment process, a log 9 reduction following treatment would be indicative of an efficient disinfection process. In instances where the biological indicator comprises about 10¹⁰ microorganisms prior to the treatment process, a log 10 reduction following treatment would be indicative of an efficient disinfection process.

The cycle time for achieving a log reduction during the waste treatment process may be 20 minutes or less, 18 minutes or less, 16 minutes or less, 14 minutes or less, 12 minutes or less, 11 minutes or less, 10 minutes or less, 9 minutes or less, 8 minutes or less, 7 minutes or less, 6 minutes or less, 5 minutes or less, 4 minutes or less, or 3 minutes or less. In certain examples, the cycle time may be as little as 2 minutes. Factors influencing the effectiveness of different cycle times include the efficiency of the shredding arrangement in shredding solid materials and/or closed containers, so that contaminated surfaces are exposed to the treatment fluid, combined with the chemical efficacy of the treatment fluid comprising a solution comprising a disinfectant and a diluent (also referred to as the disinfectant solution). Therefore, particularly short cycle times may be effective when using the treatment fluids described herein, or the shredding arrangements described herein, and particularly when using those in combination.

It may be the case that the disinfectant solution comprising a diluent and a disinfectant comprising peracetic acid and hydrogen peroxide is of a concentration in the range of from about 0.5% (v/v) to about 5% (v/v). It may be the case that the disinfectant solution comprising a diluent and a disinfectant comprising peracetic acid and hydrogen peroxide is of a concentration in the range of from about 1% (v/v) to about 3% (v/v). It may be the case that the concentration is at least 1% (v/v). It may be the case that the concentration is at least 2% (v/v). It may be the case that the concentration is at least 3% (v/v). As used herein, the term "v/v" denotes volume per volume for concentration of a solution wherein the components within the solution are liquid. The diluent may comprise, consist essentially of or preferably consist of, water.

It may be the case that the disinfectant comprising peracetic acid and hydrogen peroxide is present in the range of from about 100 millilitres to about 1000 millilitres. It may be the case that the disinfectant comprising peracetic acid and hydrogen peroxide is present in the range of from about 300 millilitres to about 800 millilitres. It may be the case that at least 600 millilitres of disinfectant comprising peracetic acid and hydrogen peroxide is present. It may be the case that at least 700 millilitres of disinfectant comprising peracetic acid and hydrogen peroxide is present.

It may be the case that the diluent is present in the range of from about 10 litres to about 30 litres. At least 15 litres of diluent may be present. At least 20 litres of diluent may be present. At least 25 litres of diluent may be present.

It may be the case that the disinfectant solution comprises a diluent and a disinfectant comprising peracetic acid in the range of from about 10% to about 20% based on the total weight of the disinfectant and hydrogen peroxide in the range of from about 15% to about 30% based on the total weight of the disinfectant; and wherein the solution is of a concentration in the range of from about 0.5% (v/v) to about 5% (v/v). The diluent may comprise, consist essentially of or preferably consist of water.

It may be the case that the solution comprises a disinfectant and a diluent, wherein the disinfectant comprises peracetic acid in the range of from about 12% to about 17% based on the total weight of the disinfectant and hydrogen peroxide in the range of from 18% to 25% based on the total weight of the disinfectant; and wherein the solution is of a concentration in the range of from about 1% (v/v) to about 3% (v/v). The diluent may comprise, consist essentially of or preferably consist of water.

It may be the case that the disinfectant solution further comprises an antifoaming agent. The antifoaming agent may be a silicone-based antifoaming agent, a non-silicone based antifoaming agent, or a combination thereof.

As used herein, the term "antifoaming agent" takes its usual meaning in the art and relates to chemical agents which can control the formation of foam.

A silicone based antifoaming agent relates to antifoaming agents comprising polymers with silicon backbones that are either oil or water based. Non-silicone-based antifoaming agents include mineral and organic based products, such as hydrocarbon based antifoaming agents.

Examples of silicone-based antifoaming agents include, but are not limited to, organopolysiloxane oils, such as poly(dimethylsiloxane), poly(methylphenylsiloxane), poly(methylethylsiloxane), poly(diethylsiloxane), poly (ethylphenylsiloxane), or combinations thereof.

Examples of non-silicone based antifoaming agents include, but are not limited to, hydrocarbon-based antifoaming agents. For example, hydrocarbon based antifoaming agents may comprise an aliphatic hydrocarbon oil, an aromatic hydrocarbon oil, an acyclic hydrocarbon oil, or combinations thereof. Additionally, or alternatively, the antifoaming agent may be selected from the foam control agents in the Foamdoctor range from PennWhite^{®} Global chemical solutions (e.g., FoamDoctor^{®} G2777E, FoamDoctor^{®} G2005, FoamDoctor^{®} G2020, FoamDoctor^{®} G2030, or FoamDoctor^{®} G2898).

The antifoaming agent may be present at a volume in the range of from about 5 millilitres to about 50 millilitres. It may be the case that the antifoaming agent is present at a volume of from about 10 millilitres to about 30 millilitres. It may be the case that at least 50 millilitres of antifoaming agent are present. It may be the case that at least 40 millilitres of antifoaming agent are present. It may be the case that at least 30 millilitres of antifoaming agent is present. It may be the case that at least 20 millilitres of antifoaming agent is present.

The antifoaming agent may be provided together with the disinfectant solution. It may be the case that the antifoaming agent is provided via the first 1510 and/or second 1520 fluid delivery means together with the disinfectant and/or diluent. It may be the case that at least some of the total volume of the antifoaming agent is provided to the vessel 120 via the second fluid delivery means.

The antifoaming agent may be added to the waste processing vessel at the same time as the biohazardous waste to be treated. It may be the case that the antifoaming agent is housed within a container. The container may be flexible or solid. The container may be tubular, conical, cubic, or have a bottle-like shape with a neck. and may be substantially cylindrical or may be a sealed bag. The container may comprise a material which is compatible with and does not influence the liquid treatment process. The container may comprise glass, glass fibers, plastic, ceramics, stainless steel, and metal oxides. It may be the case that the container comprises plastic.

Addition of the antifoaming agent in a container as described herein delays contact of the antifoaming agent contained within the biohazardous waste and treatment fluid, as the antifoaming agent is only released upon shredding of the container. Upon simultaneous shredding and treatment of the biohazardous waste, the level of foaming is often more pronounced. Therefore, addition of an antifoaming agent in a container will allow for optimum defoaming of the waste material and treatment fluid within the waste processing vessel.

The disinfectant solution may further comprise a fragrance additive. As used herein, the term "fragrance additive" takes its usual meaning in the art and relates to fragrance additives that are used to mask any unwanted odours of the treatment fluid. The fragrance additive may comprise an aldehyde (such as an aromatic aldehyde or fatty aldehyde comprising between 8 and 13 carbon atoms), a ketone, an ester (such as an aliphatic ester), an ether, an acetate, a nitrile, a terpene hydrocarbon, or an essential oil (such as natural essential oils or synthetic essential oils). An example of an aromatic aldehyde includes, but is not limited to, cinnamic aldehyde. It may be the case that the fragrance additive is RESAFRESH RIO^{®} by MACNAB SALES.

The fragrance additive may be present at a volume in the range of from about 5 millilitres to about 50 millilitres. It may be the case that the antifoaming agent is present at a volume of from about 10 millilitres to about 30 millilitres.

According to another aspect of the disclosure, there is provided a method of processing waste, the method comprising the following steps:
(I) addition of waste and a treatment fluid to a waste processing vessel of a waste processing apparatus, wherein the waste processing vessel comprises a waste processing zone, and wherein the treatment fluid is retained in the waste processing zone; and
(II) activation of a blade arrangement, such that the waste in the waste processing vessel can be shredded by the blade arrangement while immersed in the treatment fluid retained in the waste processing zone of the waste processing vessel; and
(III) deactivation of the blade arrangement after a pre-determined cycle time.

The waste, treatment fluid, and waste processing apparatus can be provided as described hereinabove.

As used herein, the cycle time relates to the time elapsed between the beginning of the mechanical processing, or shredding, of the waste in the presence of the treatment fluid. It therefore begins when the waste and the treatment fluid are present in the waste processing vessel together, and the blade arrangement begins shredding of the waste. It ends when the shredding and mixing of the waste in the vessel 120 stops. When the treatment fluid comprises a disinfectant solution as described herein, the cycle time may be the time taken, or the time sufficient, to reach a log reduction.

The method for treating waste may take place at a temperature in the range of from about 1 °C to about 40 °C, optionally in the range of from about 5 °C to about 30 °C, optionally in the range of from about 10 °C to about 26 °C.

According to another aspect of the disclosure, there is provided the use of a solution comprising a disinfectant and a diluent (also referred to as a disinfectant solution) in the treatment of biohazardous waste.

The disinfectant may comprise peracetic acid and hydrogen peroxide. The diluent may comprise, consist essentially of or preferably consist of water. The disinfectant may comprise peracetic acid in the range of from about 10% to about 20% based on the total weight of the disinfectant. The disinfectant may comprise peracetic acid in the range of from about 12% to about 17% based on the total weight of the disinfectant.

The disinfectant may comprise hydrogen peroxide in the range of from about 15% to about 30% based on the total weight of the disinfectant. The disinfectant may comprise hydrogen peroxide in the range of from about 18% to about 25% based on the total weight of the disinfectant. The solution may be of a concentration in the range of from about 0.5% (v/v) to about 5% (v/v). The solution may be of a concentration in the range of from about 1% (v/v) to about 3% (v/v).

According to another aspect of the disclosure, there is provided a use of a solution comprising a disinfectant and a diluent comprising water in the treatment of biohazardous waste, wherein the disinfectant comprises peracetic acid in the range of from about 10% to about 20% based on the total weight of the disinfectant and hydrogen peroxide in the range of from about 15% to about 30% based on the total weight of the disinfectant.

According to another aspect of the disclosure, there is provided a use of a solution comprising a disinfectant and a diluent comprising water in the treatment of biohazardous waste, wherein the disinfectant comprises peracetic acid in the range of from about 10% to about 20% based on the total weight of the disinfectant and hydrogen peroxide in the range of from about 15% to about 30% based on the total weight of the disinfectant; and wherein the solution is of a concentration in the range of from about 0.5% (v/v) to about 5% (v/v).

According to another aspect of the disclosure, there is provided a use of a solution comprising a disinfectant and a diluent comprising water in the treatment of biohazardous waste, wherein the disinfectant comprises peracetic acid in the range of from about 12% to about 17% based on the total weight of the disinfectant and hydrogen peroxide in the range of from about 18% to about 25% based on the total weight of the disinfectant, and wherein the solution is of a concentration in the range of from about 1% (v/v) to about 3% (v/v), in the treatment of biohazardous waste.

It is desirable to disinfect biohazardous waste, such that it can be disposed of safely or recycled. Moreover, there is a desire to disinfect biohazardous waste as quickly as possible. Use of a solution comprising a disinfectant comprising peracetic acid and hydrogen peroxide in the treatment of biohazardous waste as described above provides for effective chemical treatment of biohazardous waste, wherein a log reduction is achieved over a short cycle time. Moreover, use of a disinfectant solution described herein in combination with aspects of the waste processing apparatus described herein, whereby waste being shredded is immersed in a disinfectant solution during shredding, not only results in a log reduction over a short cycle time, but overcomes the setbacks associated with prior art systems in which, in order for repeated passes through a standard vertical shredding device to take place, waste must be drawn out of treatment fluids and re-passed vertically through a shredder arrangement, such as a conventional two-shaft shredding arrangement in which waste passes vertically downwards through and between the shafts and their associated shredding blades such that a disinfectant solution would generally run off the waste being shredded and treated while it passes once more through the shredding arrangement.

### Validation Process

The validation process of the present disclosure provides improved means for testing the efficacy of a waste shredding and liquid treatment process. In particular, improved ways of ensuring a validation test sample is exposed to the treatment fluid used in the process in a way which is more representative of the exposure of the waste being processed to the treatment liquid.

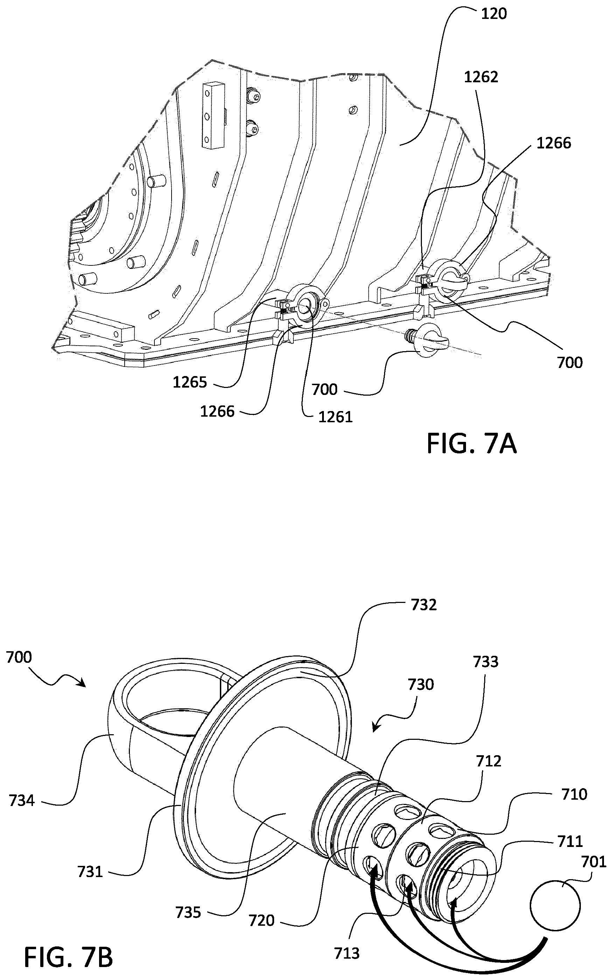

Figures 7A and 7B show in greater detail a process validation apparatus which is suitable for use with a waste processing apparatus. The process validation apparatus permits connection of a process validation module to the waste processing vessel. The process validation module, and any validation sample which it may carry, can be exposed to treatment fluid from the waste processing zone of the waste processing vessel during a waste processing cycle of the waste processing apparatus. This can be advantageous, since it can cause a validation sample to be exposed to the treatment fluid to a same or very similar degree to the exposure experienced by the waste being processed. This can give a more accurate and reliable indication of the degree of treatment to which the waste has been exposed and can permit more reliable validation of the performance of the waste treatment aspect of the waste processing method.

Figure 7A shows in greater detail the provision of one or more ports 1261, 1262 for fluidly connecting one or more process validation modules to the waste processing vessel 120 and in particular to the waste processing zone Z of the waste processing vessel 120. Alternatives can be envisaged in which the process validation module is located directly within the waste processing vessel 120. The process validation module can fulfil several functions, including holding and protecting a process validation sample from physical impact from items being shredded in the waste processing zone Z, whilst permitting treatment fluid from the waste processing zone Z to contact the process validation sample to enable the efficacy of the process to be validated. An example of a process validation module 700 is shown in figure 7B. The process validation module 700 can be provided with a sample holding portion 710 and may be equipped with a plurality of sample holding portions 710, 720. Providing a plurality of ports 1261, 1262 for receiving a process validation module at different respective locations can be advantageous, since this enables validation that adequate exposure to treatment fluid has happened at different locations within the waste processing vessel. Providing a plurality of sample holding portions on one or more process validation modules 700 can be advantageous since it allows a plurality of process validation samples 701 to be provided in a sample validation module 700, enabling dual samples to be tested from a single port location, increasing reliability of the validation process. Process validation samples 701 may comprise a substrate, which may be in the form of a planar element, such as a disc or other form suitable for carrying biological indicators, which may be attached to it in any suitable manner, including as described later in this specification. The sample holding portion 710 may comprise an internal cavity and a body portion which surrounds the internal cavity such that a validation sample 701 placed in an internal cavity of the sample holding portion 710 is protected from impact by waste being processed in the waste processing zone Z where the sample holding portion may be located. The sample holding portion 710 may be provided with one or more openings 713 which enable liquid from the waste processing zone Z to pass into the cavity to expose the validation sample to the treatment liquid. It can be advantageous for the treatment fluid be able to enter the cavity and to remain in continuous contact so that the fluid in the cavity is representative of the fluid in the waste processing vessel at all times through the waste processing cycle. The sample holding portion can be detachable from fixing portion 730 of the process validation module. This can enable the cavity to be opened for installation or removal of a process validation sample 701.

The fixing portion 730 is arranged to retain the process validation module to the waste processing vessel 120 to hold the validation sample in a substantially fixed position during waste processing. The sample holding portion 710 may comprise one or more fixing portions 711, 712. These may comprise for example a threaded arrangement. The fixing portion 711 may be arranged such that it can engage a corresponding fixing portion 712 of an adjacent sample holding portion 720 in this way, the sample holding portions may be stacked adjacent to one another to permit multiple validation samples to be mounted to a single sample holding portion 730 of a validation module 700. Module 734 may comprise the handle portion to enable a user to carry the module 700. A shaft portion 735 may extend between the handle portion 734 and the sample holding portion(s). A flange portion 731 may be provided for sealing against or around a port provided on the vessel 120. Channels 732, 733 for o-ring seals may be provided for further sealing against the waste processing vessel 120.

Assembly of the process validation module 700 with the waste processing vessel 120 is illustrated in figure 7A. The waste processing vessel 120 be provided with one or more ports 1261, 1262, which may enable the process validation module to be placed in fluid communication with an interior of the waste processing vessel 120 from an external side of the waste processing vessel. The port may comprise an extension 1265 extending the port away from a wall of the waste processing vessel 120, to facilitate fixation of the module 700 in fluid communication with an interior of the vessel 120, away from a wall of the vessel 120. One or more of the ports 1261, 1262 of the vessel 120 may comprise a flange, arranged to provide a sealing surface against which a flange 731 of the validation module 700 may provide a seal, to prevent escape of liquid from within the vessel 120 while the sample holding portion of the process validation module 700 is in fluid communication with the interior of the vessel 120. Retaining means or a retaining device 1266 may be provided to retain the validation module 700 to the vessel 120. This may be provided in the form of the illustrated clamping ring 1266, but other means may be envisaged such as a screw thread fixing, or other clamping means, such as a circlip, for example. As will be appreciated, the arrangements described above can enable one or more process validation samples to be maintained in fluid communication with the waste processing zone during a waste processing cycle. This can be advantageous because it enables the validation of a live waste processing cycle, and facilitates easy placement and extraction of a process validation sample.

The port or ports 1261, 1262 on the vessel 120 are preferably located such that they may be easily accessed by a user at least in between waste processing cycles. In the illustrated example, placing the vessel 120 in the position illustrated in figure C can enable a user to access a bottom side of the vessel 120 to install one or more process validation modules in a lower region of the vessel 120. When the vessel 120 is placed in the position illustrated in figure 6B, the ports 1261, 1262, may be located out of reach from a user accessing the device through the loading patch 160, for example. When it is not desired to locate a process validation module 700 in one of the ports 1261, 1262, then the port may be blocked or blanked by applying the closure means such as a blanking plate or a plug to rent leakage into or out of the waste processing vessel 120 via the port or ports.

Control- and method-related aspects of the validation cycle are described separately in the relevant section of this disclosure. The practical aspects of running a validation cycle in the machine can be readily understood when reading that section in combination with the above disclosure. When inserting the process validation module, the system may place the waste processing vessel 120 in a suitable position such that a user can access the validation port or ports 1261, 1262. The user will then install one or more process validation samples in fluid communication with the waste processing zone Z by attachment of a process validation module into fluid communication with the waste processing zone Z, I connection to one or more of the validation ports 1261, 1262. A validation cycle is then run as described in the relevant control section of this disclosure.

It may be the case that the efficacy of the waste shredding and liquid treatment process is determined by measuring the number of microorganisms eliminated by the waste shredding and liquid treatment process (i.e., the log reduction value).

The process efficacy indicator, which can be used to determine the efficacy of the waste shredding and liquid treatment process, may comprise a biological indicator.

The biological indicator may be selected from a bacteria, a virus, a fungus, a protozoon, one or more bacterial spores, one or more fungal spores, or a combination thereof.

As used herein, the term "virus" takes its usual meaning in the art and relates to microorganisms which infect cells. A virus typically consists of a nucleic acid molecule in a protein coat.

As used herein, the term "fungus" takes its usual meaning in the art and relates to eukaryotic single-celled or multinucleate organisms that live by decomposing and absorbing the organic material in which they grow classified in the kingdom Fungi.

As used herein, the term "protozoon" takes its usual meaning in the art and relates to a single-celled microscopic animal of a group of phyla of the kingdom *Protista.*

The bacteria may be Gram-positive bacteria, Gram-negative bacteria, or a combination thereof. The bacteria may be Gram-positive and/or Gram-negative bacteria that are non-spore-forming. Examples of Gram-positive bacteria that are non-spore-forming may include mycobacteria such as *Mycobacterium chelonae, Mycobacterium gordonae, Mycobacterium smegmatis,* and *Mycobacterium terrae.* An example of Gram-negative bacteria that is non-spore forming may include, but is not limited to, *E. coli.* It may be the case that the Gram-positive and/or Gram-negative bacteria are in the vegetative state.

The one or more bacterial spores that may be used as the biological indicator include, but are not limited to, spores derived from Gram positive bacteria, such as one or more of *Bacillus* spp. (e.g., *Bacillus subtilis, Bacillus subtilis globigii, Bacillus circulans,* and/or *Bacillus cereus), Geobacillus* spp. (e.g., *Geobacillus stearothermophilus), Clostridium spp. (e.g., Clostridium sporogenes), Alicyclobacillus* spp. (e.g., *Alicyclobacillus acidiphilus, Alicyclobacillus acidocaldarius, Alicyclobacillus acidoterrestris, Alicyclobacillus aeris, Alicyclobacillus cellulosilyticus, Alicyclobacillus contaminans, Alicyclobacillus cycloheptanicus, Alicyclobacillus dauci, Alicyclobacillus disulfidooxidans, Alicyclobacillus fastidiosus, Alicyclobacillus ferrooxydans, Alicyclobacillus fodiniaquatilis, Alicyclobacillus herbarius, Alicyclobacillus hesperidum, Alicyclobacillus kakegawensis, Alicyclobacillus macrosporangiidus, Alicyclobacillus montanus, Alicyclobacillus pomorum, Alicyclobacillus sacchari, Alicyclobacillus sendaiensis, Alicyclobacillus shizuokensis, Alicyclobacillus tengchongensis, Alicyclobacillus tolerans,* and/or *Alicyclobacillus vulcanalis),* or combinations thereof. Bacterial spores often have a high resistance to many sterilisation and disinfection processes, such as high temperatures.

It may be the case that the biological indicator comprises one or more spores derived from *Bacillus* spp., *Geobacillus* spp., *Alicyclobacillus* spp., or combinations thereof. It may be the case that the biological indicator comprises one or more spores derived from *Geobacillus* spp., *Alicyclobacillus* spp., or combinations thereof. *Alicyclobacillus* spp. are thermotolerant and can be grown at elevated temperatures. The *Geobacillus* group include thermophilic spore formers. Thermophiles thrive at high temperatures. As such, use of one or more spores derived from *Geobacillus spp.* and/or *Alicyclobacillus* spp. as a biological indicator mean that validation assays can be run at elevated temperatures, which prevents the growth of contaminating organisms, which allows for more reliable and conclusive results on the efficacy of the waste shredding and liquid treatment process.

It may be the case that one or more spores derived from one or more of *Geobacillus stearothermophilus, Bacillus subtilis, Bacillus subtilis globigii, Clostridium sporogenes, Bacillus cereus, Bacillus circulans, Alicyclobacillus acidiphilus, Alicyclobacillus acidocaldarius, Alicyclobacillus acidoterrestris, Alicyclobacillus aeris, Alicyclobacillus cellulosilyticus, Alicyclobacillus contaminans, Alicyclobacillus cycloheptanicus, Alicyclobacillus dauci, Alicyclobacillus disulfidooxidans, Alicyclobacillus fastidiosus, Alicyclobacillus ferrooxydans, Alicyclobacillus fodiniaquatilis, Alicyclobacillus herbarius, Alicyclobacillus hesperidum, Alicyclobacillus kakegawensis, Alicyclobacillus macrosporangiidus, Alicyclobacillus montanus, Alicyclobacillus pomorum, Alicyclobacillus sacchari, Alicyclobacillus sendaiensis, Alicyclobacillus shizuokensis, Alicyclobacillus tengchongensis, Alicyclobacillus tolerans, Alicyclobacillus vulcanalis,* or combinations thereof are used as the biological indicator. Additionally, or alternatively, one or more spores derived from one or more of *Geobacillus stearothermophilus, Bacillus subtilis, Bacillus subtilis globigii, Clostridium sporogenes, Bacillus cereus, Bacillus circulans,* or a combination thereof may be used as the biological indicator.

It may be the case that the biological indicator comprises one or more spores derived from *Geobacillus stearothermophilus. Geobacillus stearothermophilus* is a thermophile, which as noted above, thrives at high temperatures. It may be the case that the strain of *Geobacillus stearothermophilus* is ATCC 7953.

Viruses that may be used as the biological indicator include one or more of bacteriophages (for example, Somatic coliphages, Male-specific (F+) coliphages, F+ bacteriophage, Staphylococcal phages, or combinations thereof), Adenoviridae (for example, mammalian adenoviruses and avian adenoviruses), Picornaviridae (for example, Enteroviruses such as poliovirus), and Caliciviridae (for example, noroviruses such as murine norovirus). The biological indicator may be a virus selected from bacteriophages selected from somatic coliphages, male-specific (F+) coliphages, F+ bacteriophage (e.g., *Emesvirus zinderi,* also known as MS2), staphylococcal phages, or combinations thereof. Bacteriophages, such as those described herein, are simple to handle, and enumerate, thereby facilitating the preparation of the validation sample (or samples) for the validation process described herein.

Fungi that may be used as the biological indicator include one or more of *Aspergillus brasiliensis, Candida albicans, Trichophyton mentagrophytes,* and *Wangiella dermatitis.* Additionally, or alternatively, one or more spores derived from one or more of *Aspergillus brasiliensis, Candida albicans, Trichophyton mentagrophytes,* and *Wangiella dermatitis* can be used as the biological indicator.

In instances where Protozoa are used as the biological indicator, said Protozoa may be in the form of a cyst or in the form of an oocyst. Protozoa that may be used as the biological indicator include one or more of *Giardia lamblia* in the form of a cyst and *Cryptosporidium parvum* in the form of an oocyst.

As mentioned above, fungal spores that may be used as the biological indicator include, but are not limited to, spores derived from one or more *of Aspergillus brasiliensis, Candida albicans, Trichophyton mentagrophytes,* and *Wangiella dermatitis.*

It may be the case that the biological indicator comprises one or more bacterial spores, a virus, vegetative bacteria, or a combination thereof. It may be the case that the biological indicator comprises one or more bacterial spores. It may be the case that the biological indicator comprises a virus.

In instances where the biological indicator comprises one or more bacterial spores, one or more fungal spores, or combinations thereof, the number of spores present in the bacterial indicator is in the range of from about 10⁴ to about 10¹⁰.

In instances where the biological indicator comprises one or more bacterial spores, one or more fungal spores, or combinations thereof, the one or more spores may be produced by growing the bacteria and/or fungus in question to a high concentration, optionally in a culture medium, followed by subjecting the vegetative bacteria and/or fungal cells in question to environmental stress (such as, elevated temperature). The vegetative cells will then subsequently sporulate. Remaining vegetative cells can be removed using any known techniques, such as for example centrifugation or chemical treatment (for example, treatment with lysozyme). The spores can then be stored or processed for use as the biological indicators.

In instances where the biological indicator comprises one or more bacterial spores, the substrate comprising the biological indicator may be disrupted to break up and/or disintegrate the substrate before or after incubation of the biological indicator in culture medium. The disruption may be mechanical disruption, such as bead beating.

Enumeration may be carried out by plating on an agar plate, such as on Tryptone Soy Agar. It may be the case that triplicate enumeration is carried out.

In instances where the biological indicator comprises one or more bacterial spores, it may be the case that the biological indicator is diluted one or more times prior to enumeration of the viable spores following cultivation. The biological indicator may be diluted at least 10 times prior to enumeration. It may be the case that the biological indicator is diluted at least 7 times prior to enumeration. It may be the case that the biological indicator is diluted at least 5 times prior to enumeration.

The biological indicator may be secured to a substrate, which may be comprised in the process validation sample(s) 701.

The substrate may comprise a material which is compatible with and does not influence the liquid treatment process. The substrate may comprise paper, glass fibers, plastics, ceramics, stainless steel, and metal oxides. It may be the case that the substrate comprises stainless steel. The substrate may be in the form of a strip or a disc.

It may be the case that the biological indicator is secured to the substrate by the use of an inert adhesive. It may be the case that the inert adhesive comprises a polymeric adhesive. It may be the case that the inert adhesive comprises a hydrogel.

The hydrogel may comprise a natural polymer, a synthetic polymer, or a combination thereof.

Examples of natural polymer include, but are not limited to, collagen, chitosan, silk fibroin, alginate, or a combination thereof. Examples of synthetic polymers include, but are not limited to, poly(ethylene glycol) (PEG), poly(vinyl alcohol), poly(N-isopropylacrylamide) (PNIPAM), or a combination thereof.

The hydrogel may comprise a sheet of polymeric crosslinked molecules. Alternatively, the hydrogel may be free flowing. Alternatively, the hydrogel may be dispersed into an absorbent material, for example, a gauze, a sponge, a strip, or a pad.

It may be the case that the biological indicator is secured to the substrate through electrostatic forces, whereby a surface of the biological indicator and a surface of the substrate are charged prior to adhesion. Any known charging technique can be used, such as for example the coating of all or part of one or more surfaces of the biological indicator and/or substrate with polyomino acids, bovine serum albumen, multivalent glyconanoparticles, glycolipids, dendrimers or various polymers (such as, for example acrylamide derivatives).

To distribute the biological indicator on the substrate, it may be the case that a suspension of the biological indicator in a solvent selected from water, saline solution, or alcohol is prepared. As used herein, the term "saline solution" refers to a solution comprising water and sodium chloride. The concentration of saline solution may be in the range of from about 0.1 % to about 1.0 % by weight of sodium chloride. The suspension may then be placed over the substrate material (and the inert glue when present). The suspension may be placed over the substrate using a needle or a micropipette. The impregnated substrate my then be cut to the appropriate size and shape for use in the validation apparatus described herein.

After conclusion of the waste shredding and liquid treatment process, the biological indicator (and substrate if present) may then be removed, and then subsequently incubated in a culture medium for a pre-determined period of time (depending on the desired culture conditions of the biological indicator selected) to promote growth of any remaining viable microorganisms. Subsequent microbial growth will provide an indication that the liquid treatment process was ineffective.

In instances where the biological indicator comprises bacteriophages, the bacteriophage in question may be mixed with a culture of the appropriate host bacterium at an appropriate concentration for a specific period of time. The skilled person will appreciate that the concentration and time period will depend on the bacteriophage in question. Following this, a bacterial lawn may be generated from the mixture, and the bacterial lawn may then be evaluated to determine whether there is any indication of bacterial lysis. In instances where the biological indicator comprises a virus, such as an animal virus, a cell lysis process as above for bacteriophages may be implemented using animal cell lines.

Removal and/or subsequent incubation of the biological indicator may be handled using standard aseptic techniques. Standard aseptic techniques prevent the introduction of contamination, which would potentially lead to inaccurate results about the efficacy of the liquid treatment process.

The validation sample comprising a biological indicator (and substrate if present) may be handled using sterile forceps.

The culture medium may comprise any suitable medium for the biological indicator depending on the desired culture conditions for said biological indicator.

Where the biological indicator comprises one or more spores derived from *Geobacillus* spp. (such as *Geobacillus stearothermophilus), Bacillus* spp., *Alicyclobacillus* spp., or combinations thereof, the culture medium may be selected from trypticase soy agar, tryptone soya agar, trypticase soy broth, nutrient broth, nutrient broth number 2, or Mueller-Hinton broth. The culture medium for the majority of spore-forming organisms such as those from the genera *Bacillus* and *Geobacillus* may have a pH in the range from about 6 to about 8 which provides optimal culture conditions. For *Alicyclobacillus* spp. the culture medium may have a pH in the range of about 4 to about 5.5 depending on the strain to be used. In instances where the treatment fluid described herein comprises a disinfectant comprising peracetic acid and hydrogen peroxide, the culture medium may further comprise a peracetic acid neutraliser. A peracetic acid neutraliser ensures that any peracetic acid remaining on the biological indicator matrix does not continue to act on the biological entity. The peracetic acid neutraliser may be selected from sodium thiosulphate, sodium metabisulphite, or a combination thereof. The incubation temperature may be in the range of from about 50 °C to about 65 °C. The incubation time may be in the range of from about 5 days to about 10 days. Incubation times in these ranges provide for sufficient time to bring about a conclusive result as to the presence of any microorganism growth within the validation sample, depending on the method utilised for detecting microbial growth. For instance, the method for detecting microbial growth may include monitoring consumption of oxygen by viable bacteria in a sample. The changes in oxygen concentration would allow for the concentration of bacteria to be determined. For instance, methods such as those described in EP2541234B1 may be used to determine microbial growth.

### Examples

Biohazardous waste was provided containing a combination of solid waste and of whole human blood (WHB).

Four cycles were carried out using a waste processing apparatus 1000 on two separate days with varying assortments of biohazardous waste (detailed below).

For each treatment cycle, the treatment fluid was a 3% (v/v) concentration of peracetic acid and hydrogen peroxide solution consisting of 600 ml VigorOx 15/23^{®} (PeroxyChem) and 20 litres of water. VigorOx 15/23^{®} contains peracetic acid at 15 wt.% and hydrogen peroxide at 23 wt. %.

For each cycle, the solid waste and whole human blood were loaded into the waste processing vessel 120. Subsequently, 600 ml VigorOx 15/23^{®} was added to the waste processing vessel, together with 20 litres of water and 50 ml Foam Doctor^{®} G2777E (a hydrocarbon based antifoaming agent). Foam Doctor^{®} G2777E was added in a plastic vial container.

For each cycle, the apparatus was started, and the waste and the treatment fluid were in contact for a specific time (i.e., the cycle time). The cycle times of each cycle is detailed below.

To evaluate the efficacy of the process under standard operating conditions the biological Indicator *Geobacillus stearothermophilus* (ATCC Strain 7953) on spore strips made of paper was placed in a process validation module 700, into a sampling port 1261, 1262, in contact with the same liquid mixture as the shredded waste and peracetic acid and hydrogen peroxide aqueous solution. The biological indicator *Geobacillus stearothermophilus* (ATCC 7953) was prepared by BIoCI labs, Raleigh, North Carolina in the form of biological indicator spore strips (average > 4 log₁₀ and 6 log₁₀ spores/strip). After each cycle was completed, the validation module 700 was removed, and the efficacy of each cycle was determined as detailed below.

### Validation Protocol

The following protocols were applied for each cycle:
1. The system cycle for "Validation Test" on the touch screen display was selected.
2. The biological indicators were prepared for use in the waste processing vessel.
3. The biological indicators were placed into the individual validation modules 700.
4. The assembled biological validation modules were inserted into validation ports 1261, 1262 and secured in place.
5. Once the biological indicator carriers were attached to the waste processing vessel 120, their attachment was confirmed by selecting "Manifold Fitted" on the touch screen display.
6. The waste was loaded into the waste processing vessel 120 and a waste processing cycle was run.
7. On completion of each processing cycle, the processing chamber was returned to the position for removal of the biological indicator carriers.

### Processing of the spore strips

Individual spore strips were handled using aseptic techniques, whereby forceps used were cleaned using 70% ethanol wipes between retrieval of each sample.

Following removal from the instrument, the spore strips were placed into a tube containing 10 ml of tryptic soy broth and 10mM sodium thiosulphate to neutralise the disinfectant. Each tube was vortexed vigorously to ensure effective contact between the spore strip and the neutraliser. The spore strips were subsequently incubated. To ensure integrity of data, spores were enumerated on both days of the experimental runs.

The incubator unit was calibrated for 55 °C to 60 °C for on-site incubation of *Geobacillus stearothermophilus.*

For samples with spore strips containing 10⁴ spores, the Tryptic Soy Broth /sodium thiosulphate containing the treated strips were incubated directly at 55°C for seven days without further treatment.

For samples with spore strips containing 10⁶ spores, glass beads were added to the tubes and all tubes shaken until the strips had completely disintegrated. Serial tenfold dilutions were then performed, and any viable spores were then enumerated by plating on Tryptone Soy Agar (in triplicate) and incubating at 55°C for seven days.

### Results

### Day 1

(i) *Run 1 - Efficacy cycle run with 10⁴ spores per strip*
   - Waste weight/volume: 10 to 12 kg solid waste and 4.0 L of WHB (Total load: 13 kg)
   - Cycle Time: 19 minutes
   - Nature of biohazardous waste: empty and gelatine filled blood collection tubes (various sizes), empty syringes, empty urine collection cups, blood RNA tubes, EDTA blood collection tubes and Teruflex dry collection bags with whole human blood.
   - Status of recovered biological indicators: not affected
   - Average pH value of effluent: 4.48
(ii) *Run 2 - Efficacy cycle run with 10⁴ spores per strip*
   - Waste weight/volume: 10 to 12 kg solid waste and 4.0 L of WHB (Total load: 16 kg)
   - Cycle Time: 19 minutes
   - Nature of biohazardous waste: empty and gelatine filled blood collection tubes (various sizes), empty syringes, empty urine collection cups, blood RNA tubes, EDTA blood collection tubes and Teruflex dry collection bags with whole human blood
   - Status of recovered biological indicators: not affected
   - Average pH of effluent: 4.37
(iii) *Run 3 - Efficacy cycle run with 10⁴ spores per strip*
   - Waste weight/volume: 10 to 12 kg solid waste and 4.0 L of WHB (Total load: 16 kg)
   - Cycle Time: 19 minutes
   - Nature of biohazardous waste: empty and gelatine filled blood collection tubes (various sizes), empty syringes, empty urine collection cups, blood RNA tubes, EDTA blood collection tubes and Teruflex dry collection bags with whole human blood.
   - Status of recovered biological indicators: not affected
   - Average pH of effluent: 5.09
(iv) *Run 4 - Efficacy cycle run with 10⁶ spores per strip*
   - Waste weight/volume: 10 to 12 kg solid waste and 4.0 L of WHB (Total Load: 16 kg)
   - Cycle Time: 19 minutes
   - Nature of biohazardous waste: empty and gelatine filled blood collection tubes (various sizes), empty syringes, empty urine collection cups, blood RNA tubes, EDTA blood collection tubes and Teruflex dry collection bags with whole human blood.
   - Status of recovered biological indicators: not affected
   - Average pH of effluent: 4.50

### Day 2

(v) *Run 1 - Efficacy cycle run with 10⁴ (Qualitative analysis) and 10⁶ (Quantitative analysis) spores per strip*
   - Waste weight/volume: 6.0 kg solid waste and 4.0 L of WHB (Total load: 10 kg)
   - Cycle Time: 19 minutes
   - Nature of biohazardous waste: empty and gelatine filled blood collection tubes (various sizes), empty syringes, empty urine collection cups, blood RNA tubes, EDTA blood collection tubes and Teruflex dry collection bags with whole human blood.
   - Status of recovered biological indicators: not affected
   - Average pH value of effluent: 4.26
(vi) *Run 2 - Efficacy cycle run with 10⁴ (Qualitative analysis) and 10⁶ (Quantitative analysis) spores per strip*
   - Waste weight/volume: 7.5 kg solid waste and 4.0 L of WHB (Total load: 11.5 kg)
   - Cycle Time: 19 minutes
   - Nature of biohazardous waste: empty and gelatine filled blood collection tubes (various sizes), empty syringes, empty urine collection cups, blood RNA tubes, EDTA blood collection tubes and Teruflex dry collection bags with whole human blood.
   - Status of recovered biological indicators: not affected
   - Average pH of effluent: 4.25
(vii) *Run 3 - Efficacy cycle run with 10⁴ (Qualitative analysis) and 10⁶ (Quantitative analysis) spores per strip*
   - Waste weight/volume: 7.5 kg solid waste and 4.0 L of WHB (Total load: 11.5 kg)
   - Cycle Time: 19 minutes
   - Nature of biohazardous waste: empty and gelatine filled blood collection tubes (various sizes), empty syringes, empty urine collection cups, blood RNA tubes, EDTA blood collection tubes and Teruflex dry collection bags with whole human blood.
   - Status of recovered biological indicators: not affected
   - Average pH of effluent: 4.25
(viii) *Run 4 - Efficacy cycle run with 10⁴ (Qualitative analysis) and 10⁶ (Quantitative analysis) spores per strip* Run 4 was conducted with 3.0% added disinfectant.
   - Waste weight/volume: 7.7 kg solid waste and 4.0 L of WHB (Total Load: 11.7 kg)
   - Cycle Time: 19 minutes
   - Nature of biohazardous waste: empty and gelatine filled blood collection tubes (various sizes), empty syringes, empty urine collection cups, blood RNA tubes, EDTA blood collection tubes and Teruflex dry collection bags with whole human blood.
   - Status of recovered biological indicators: not affected
   - Average pH of effluent: 4.24

### Conclusion

On Day 1 and Day 2 when experimental runs were carried out, the disinfectant performed as expected and achieved greater than 4 log inactivation of *Geobacillus stearothermophilus* where 10⁴ spores were used. The runs performed on Day 2 that used 10⁶ spores showed complete inactivation (i.e., greater than six log).

### Controls

The following controls were run:
- A negative control consisting of Tryptic Soy Broth containing sodium thiosulphate
- A positive control consisting of Tryptic Soy Broth containing sodium thiosulphate and an unexposed spore strip (>10⁴ spores)
- A neutraliser control consisting of 10 ml Tryptic Soy Broth and 5 ml of 3% Vigorox. After vortexing for 15 seconds an unexposed spore strip (>10⁴ spores) was added to the mixture

All controls were incubated at 55 °C for seven days. The results were as expected, and there was no growth in the negative control and normal growth in both the positive and neutralizer effectiveness control.

### Blade arrangement

One aspect of improvement identified is a novel blade arrangement which can process a vast array of waste streams that differ in size, composition, malleability, strength and composition more reliably and efficiently. The blade arrangement can be termed a shredding assembly. According to an aspect, there is provided a shredding assembly comprising any or all of the following features: a fixed blade array; a rotatable blade array configured to rotate relative to the fixed blade array about a rotational axis. The rotatable blade array may comprise a plurality of rotatable blades. A rotatable blade of the plurality of rotatable blades may comprise any of: a main body extending in an outwardly radial direction with respect to the rotational axis; a first cutting face configured for cutting in a first rotational direction of the rotatable blade array; and a second cutting face configured for cutting in a second rotational direction of the rotatable blade array. The plurality of rotatable blades may be interspersed with the fixed blade array such that, during rotation of the rotatable blade array, at least a portion of the radial extent of the main body passes through a gap between adjacent fixed blades of the fixed blade array. The shredding assembly of the present disclosure provides a device which can shred waste more flexibly and efficiently. Such a shredding assembly can be incorporated into a waste processing vessel for receiving waste to be shredded and treated. The fixed blade array may be a lower fixed blade array, such that it can be positioned at the bottom of the waste processing vessel where waste is collected in the shredding assembly. This can correspond to a waste processing zone of the waste processing vessel in which waste is continuously shredded for a set period of time. By providing a rotatable blade array configured to rotate relative to the fixed blade array, the advantageous shredding assembly can be provided, for example, by a single rotatable shaft, in contrast to arrangements having a twin shaft of interspersing rotatable blades. An advantageous arrangement may therefore provide a rotatable blade array which has blades arranged to interleave with one or more adjacent fixed blade arrays. By providing a first cutting face and a second cutting face, the shredding assembly can be operated such that the rotatable blade array can rotate in a first direction or a second, opposite direction in order to shred waste between the rotatable blades and the fixed blades when the rotatable blade array rotates in either direction. Such bidirectionality of the rotatable blade array is advantageous because it allows for an improved shredding process in which the direction of rotation can be reversed periodically, and/or in response to reaching a threshold torque of the rotatable blade array. The radial extent of the main body can be defined as the maximum radial distance of a radial extremity of the main body from the rotational axis. A maximum width of the main body between the first and second cutting faces may be less than three quarters, preferably less than seven tenths, preferably less than two thirds of the radial extent of the main body. A minimum width of the main body between the first and second cutting faces may be less than one half of the radial extent of the main body. This provides an advantageously dimensioned blade arrangement that efficiently shreds various types of waste. The shredding assembly may be configured such that at least half, preferably at least three quarters, preferably at least eight tenths of the radial extent of the main body passes through the gap between adjacent fixed blades during rotation of the rotatable blade array. By interspersing the fixed blades with the rotatable blades such that a major portion of the radial extent of the main body passes through a gap between adjacent fixed blades of the fixed blade array, the shredding assembly can be suitable for shredding waste having different dimensions while reducing the chance that the shredding assembly becomes jammed. Two or more rotatable blades may extend from the rotational axis at different angles relative to one another. The plurality of rotatable blades may comprise a first rotatable blade and a second rotatable blade, which may be located at a position adjacent along the rotational axis to the first rotatable blade. An angular offset between the first rotatable blade and the second rotatable blade may be at least 15 degrees, preferably at least 30 degrees. The angular offset may be an integer multiple of an angle between 15 and 45 degrees. The angular offset may be an integer multiple of 30 degrees. The plurality of rotatable blades may comprise a group of up to twelve rotatable blades. The shredding assembly may comprise any number between 5 and 50 rotatable blades in different implementations. One or more, or each, of the plurality of rotatable blades in the group may be angularly offset from the other rotatable blades. The group may comprise two, preferably four, preferably eight, preferably twelve rotatable blades. For different implementations, any integer number between 3 and 50 may be suitable. The angular offset between select pairs of blades in the array, or between each blade in the array may be an integer multiple of an angle between 5 and 45 degrees, or between 15 and 45 degrees, and may be 30 degrees in certain examples. In this way, the rotatable blade array can be configured such that, during rotation thereof, the passage of rotatable blades through the gaps in the fixed blade array can be staggered. This can be advantageous in reducing peaks in the maximum torque of the rotatable blade array and can help in reducing overall levels of vibration. However, different implementations may require certain blades in the array or group to be in rotational alignment, i.e., having no angular offset with respect to one another. In certain implementations, plural arrays may be provided, each array comprising blades which are in rotational alignment with each other with the respective array being offset from other arrays by the offsets described herein.

A centreline of the main body may be defined along the radial extent thereof. The first cutting face may extend along the main body at a first angle to the centreline. The second cutting face may extend along the main body at a second angle to the centreline. At least one of the first angle and the second angle may be between 1 and 10 degrees. At least one of the first angle and the second angle may be approximately 5 degrees. A plane defined by the centreline and the rotational axis may be a plane of symmetry between the first and second cutting faces. At least one of the first and second cutting faces may comprise a plurality of serrations. The plurality of serrations may comprise a first set of serrations on a first side of the rotatable blade. The plurality of serrations may further comprise a second set of serrations on a second side of the rotatable blade. The first side and the second side may define opposite sides of the rotatable blade at different positions along the rotational axis. The first side and the second side may comprise planar side faces of the main body. The planar side faces may be normal to a direction parallel to the rotational axis. The serrations of the first and/or second set of serrations may be substantially pyramidal.

The shredding assembly may further comprise a recess extending at least partially along the radial extent of the first and/or second cutting face. The recess may be located between the first set and second set of serrations. A blade height may be defined as the difference between the smallest radius of the main body about the rotational axis, which may be where the main body is connected to a shaft and the largest radius of the main body, which may be the maximum radial extent of the main body. The blade height may be larger than the lateral distance between the opposing cutting faces. The blade height may be larger than a maximum lateral distance between the opposing cutting faces by at least a quarter, preferably by at least a third, preferably by at least four tenths. The blade height may be larger than a minimum lateral distance between the opposing cutting faces by at least a third, preferably at least four tenths, preferably by at least a half. The minimum lateral distance may be at least half the maximum lateral distance between the opposing cutting faces, preferably at least six tenths, preferably at least three quarters.

The width of the main body may taper away from the rotational axis of the rotatable blade. The width of the main body may increase linearly with increased radial distance from the rotational axis. The angle between the first tangent and the second tangent may be approximately ten degrees. The angle between the centreline and at least one of the first and second tangents may be between four and six degrees; preferably, this angle is approximately five degrees. A first width (perpendicular to the centreline) of the main body between the first and second cutting faces may be defined at a first radial position, and a second width may be defined at a second radial position further from the rotational axis than the first radial position, and the first width may be greater than the second width. The first width may be measured at a base of the main body, that is, proximate to the rotational axis, and the second width may be measured at an end of the main body, that is, distal from the rotational axis. The rotatable blade may be configured such that its centre of mass is offset from the rotational axis. Alternatively or additionally, a maximum order of rotational symmetry of the rotatable blade with respect to the rotational axis may be one.

A fixed blade of the shredding assembly may comprise first and second end regions. The first end region and second end region may have mounting portions for mounting to a fixed body of the shredding assembly, which may be part of a waste processing vessel. The fixed blade may extend between the mounting portions. The fixed blade may extend across the rotatable blade array. The fixed blade may extend transverse to the rotational axis. The fixed blade array may comprise two, preferably three fixed blades. The fixed blade may further comprise an intermediate portion provided between the first and second end region. The intermediate portion may comprise a cutting face. The cutting face may extend between the first and second end regions. The fixed blade may further comprise end cutting portions. The end cutting portions may be oriented toward a centreline of the intermediate portion. The end cutting portions may be provided on the first and second end regions. The fixed blade may lie in a plane normal to the rotational axis.

The cutting face may be an upper cutting face. The upper cutting face may be oriented away from the rotational axis. The fixed blade may further comprise a lower cutting face. The lower cutting face may extend between the first and second end regions. The lower cutting face may be oriented toward the rotational axis. The lower cutting face may have end cutting portions oriented toward a centreline of the intermediate portion. In this way, the fixed blade can have cutting means on opposite sides thereof such that, when a rotatable blade rotates relative to the fixed blade, waste can be shredded against the upper cutting face on a downward swing of the rotatable blade, and can be shredded against the lower cutting face on an upward swing of the rotatable blade.

The fixed blade may comprise a further face. The further face may be oriented toward the intermediate portion. The further face may be provided between an end region and the upper cutting face. The further face may face toward the upper cutting face. The further face may form a wall of a cut-out portion provided between an end region and the upper cutting face. The cut-out portion may be configured to align with an outer radial edge of the rotatable blade when the rotatable blade rotates relative to the fixed blade.

The cutting face may comprise at least a first spike and a second spike adjacent the first spike. The fixed blade may comprise a first side face and a second side face on the opposite side of the fixed blade to the first side face. The first spike may have a surface that is coplanar with the first side face. The second spike may have a surface that is coplanar with the second side face. The first and/or second spike may be substantially pyramidal. An apex of the first spike may be in the plane of the first side face. An apex of the second spike may be in the plane of the second side face.

The fixed blade array may be a first fixed blade array of the shredding assembly, which may be an upper fixed blade array. The first fixed blade array may be provided above and across the rotational axis. The shredding assembly may further comprise a second fixed blade array. The second fixed blade array may extend from the fixed body of the shredding assembly radially toward the rotational axis. The second fixed blade array may be a lower fixed blade array. The second fixed blade array may be provided below the rotational axis.

The second fixed blade array may be arranged in a linear array. At least one of the plurality of fixed blades may comprise a substantially triangular portion, which may be truncated in proximity to the rotatable blade array. A fixed blade of the second fixed blade array may comprise a first cutting face and a second cutting face arranged in a similar manner to the first and second cutting faces on the rotatable blade. A first cutting face of the fixed blade may be configured for cutting when the rotatable blade array rotates in its first rotational direction. A second cutting face of the fixed blade may be configured for cutting when the rotatable blade array rotates in its second rotational direction. A main body of the fixed blade may extend from a fixed body of the shredding assembly toward the rotational axis.

At least one of the first and second cutting faces of the fixed blade may comprise a plurality of serrations. The plurality of serrations may comprise a first set of serrations on a first side of the fixed blade. The plurality of serrations may further comprise a second set of serrations on a second side of the fixed blade. The first side and the second side may define opposite sides of the fixed blade at different positions along the rotational axis. The first side and the second side may comprise planar side faces of the main body. The planar side faces may be normal to a direction parallel to the rotational axis. The rotatable blade array may be configured to only have a single blade in a given plane perpendicular to the rotational axis. In other words, the rotatable blade array may be arranged to consist of one rotatable blade at a given position along the rotational axis. This is in contrast to arrangements having multiple blades extending in different directions from a single position along the rotational axis. This arrangement provides more efficient shredding which can be less likely to jam and may reduce vibrations.

The rotatable blade array may be provided on a shaft. The rotatable blade may comprise a fixing portion configured to engage with, or surround a major portion or the entirety of the angular extent of, the shaft. The fixing portion may comprise an aperture configured to receive a shaft. The aperture may be configured to engage a shaft and prevent rotation between rotatable blade and a shaft to which the rotatable blade is mounted. The fixing portion may comprise a non-circular aperture. The non-circular aperture may define a spline, or may be polygonal in form. The aperture may comprise a plurality of corners, curves and/or flat faces, configured to engage corresponding faces on a shaft to prevent relative rotation therebetween. The aperture may be any polygon, for engaging any corresponding polygonal shaft and in one example, may be a hexagonal aperture, which may be configured to receive a hexagonal shaft. This provides the advantage of increasing the manufacturability of the shredding assembly. The first cutting face may extend at a first tangent from the fixing portion. The second cutting face may extend at a second tangent from the fixing portion.

The shredding assembly may be configured such that, when a rotatable blade passes through the gap between adjacent fixed blades, the separation between the rotatable blade and a fixed blade is no more than 1 mm with respect to a direction parallel to the rotational axis. The separation may additionally or alternatively be no more than two tenths, preferably no more than one tenth, preferably no more than one twentieth of the thickness of the rotatable blade with respect to a direction parallel to the rotational axis. A non-zero and non-negligible separation is provided, so that materials can pass between the adjacent blades within the gap, to reduce the risk of jamming. The separation may therefore be at least a tenth of a millimetre or at least a multiple of a tenth of a millimetre, including all integer multiples of a tenth of a millimetre up to and including the upper bounds defined above for the separation.

There is also provided a rotatable blade array for the shredding assembly as described hereinabove. In other words, there is provided a rotatable blade array separate from the shredding assembly, i.e. without including any fixed blade array. There is also provided a rotatable blade for the shredding assembly or the rotatable blade array as described hereinabove. In other words, there is provided a single rotatable blade separate from the rotatable blade array. There is also provided is a fixed blade for the shredding assembly as described hereinabove. In other words, there is provided a fixed blade separate from any rotatable blade array. The fixed blade may be a fixed blade of the first fixed blade array or the second fixed blade array, and may have any of the features described hereinabove. Blade arrangements as disclosed can be combined advantageously with any other of the aspects and features disclosed herein.

The inventors have identified particular areas for improvement in the processing of waste, in particular contaminated, hazardous or biohazardous waste. In particular, the inventors have identified improved blade arrangements which can effect both mixing and shredding of waste in a more efficient and effective manner.

The disclosed shredding assembly is particularly suited to use with the vast array of different biohazardous waste products that are produced in laboratories. Such waste streams may contain glass, paper, polymers, blood, urine, sharps, PPE, etc. and hence the blade arrangement needs to cope with a range of hard and soft materials, some being pliable and malleable others being hard and brittle. None of these materials can be recycled while they are considered to be of a biohazardous nature. For a system in which the disclosed blade arrangement is implemented to disinfect biohazardous waste, the waste needs to be shredded sufficiently to ensure that a treatment chemical comes into contact with the biohazardous materials. Once the initially biohazardous waste is sufficiently treated to be considered non-hazardous and safe for handling and transportation, it can be sent to recycling facilities for separation and pelletisation. The disclosed system facilitates recycling, as the waste processed in the system can be made safe. Combined disinfection or decontamination and shredding is an advantage to downstream recyclers as they can skip several steps in the usual known processes at recycling plants, with the output from blade arrangements and systems as disclosed herein.

Disclosed herein is a shredding assembly for the mixing and shredding of waste. The shredding assembly can be incorporated into a device for the treatment of biohazardous waste. In particular, the shredding assembly can be incorporated into a receiver, such as a waste processing vessel for waste to be treated. The shredding assembly can form part of a process for treating waste, which may additionally include treating the waste with a fluid or liquid treatment in order to clean, sterilise or disinfect the waste. Such treatment may occur in the waste processing vessel at the same time as the shredding assembly shreds the waste.

The shredding assembly disclosed herein includes a rotatable shaft having a plurality of blades configured to rotate with the shaft. The blades can be spaced at regular intervals along a rotational axis of the shaft and can extend from the shaft at a variety of different angles. Each rotatable blade on the rotatable shaft has a cutting face on opposite sides of the blade such that the cutting faces are substantially perpendicular to the path of rotation of the rotatable blade. The shredding assembly also includes a lower fixed blade array, which can be fixed relative to the waste processing vessel. The lower fixed blade array has a plurality of lower fixed blades configured to intersperse with the rotatable blades so that the rotatable blades will pass through gaps between the lower fixed blades during rotation of the rotatable blades about the rotational axis. The lower fixed blades can have cutting faces on opposite sides thereof in order to co-operate with the cutting faces of the rotatable blades. The rotatable shaft can be configured to rotate in a clockwise or an anti-clockwise direction. In this way, waste in the bottom of the waste processing vessel can be shredded by the action of the rotatable blades passing through the lower fixed blades in either a clockwise or an anti-clockwise direction.

The shredding assembly can also include one or more upper fixed blades. The upper fixed blades can be provided on a top side of the rotatable shaft, that is, on the opposite side of the rotatable shaft to the lower fixed blade array. The upper fixed blades can be configured to facilitate the breaking down and cutting of larger items of waste before such items are further shredded between the rotatable blades and the lower fixed blade array. Therefore, when a bag of hazardous waste - which may include various items of waste made from different materials, such as glass beakers, plastic tubes, fabric laboratory coats, and so on - is placed into the waste processing vessel, rotation of the rotatable blades can begin to break down the waste by crushing and cutting the waste between the cutting faces of the rotatable blade and the upper fixed blade, until the waste is small enough to fall to the bottom of the waste processing vessel, where the waste can be further shredded between the cutting faces of the rotatable blades and the lower fixed blades.

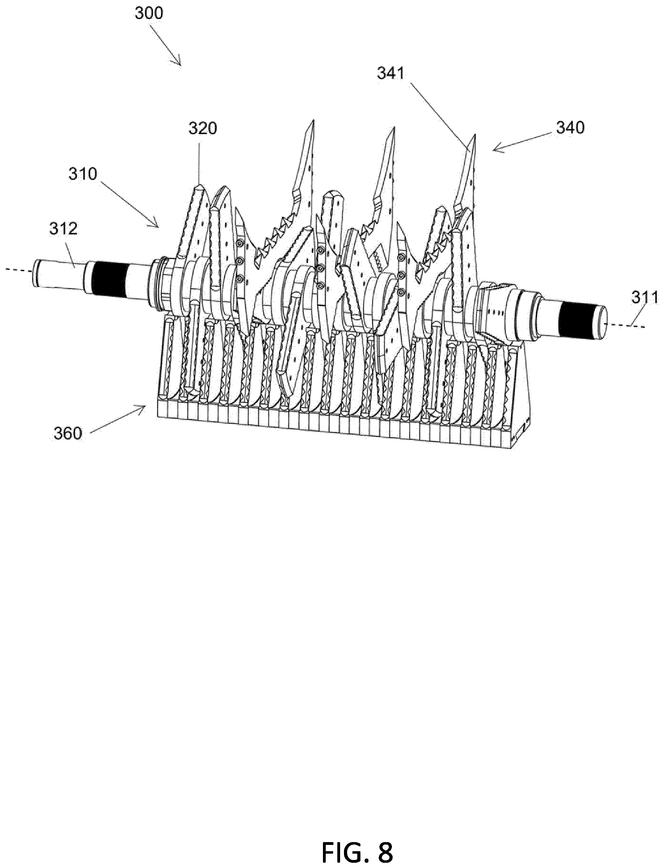

Figure 8 illustrates a shredding assembly 300 according to the present disclosure. The shredding assembly 300 comprises a lower fixed blade array 360 comprising a plurality of lower fixed blades. The shredding assembly 300 further comprises a rotatable blade array 310 configured to rotate relative to the lower fixed blade array 360. The rotatable blade array 310 is configured for rotation about a rotational axis 311 and can be provided on a shaft 312. The rotatable blade array 310 comprises a plurality of rotatable blades 320. In the arrangement shown, the rotatable blade array 310 is fixed to the shaft 312 so as to be rotatable with the shaft 312 about the rotational axis 311.

The plurality of rotatable blades 320 are interspersed with the lower fixed blade array 360 such that, during rotation of the rotatable blade arrangement 310, at least a portion of the radial extent of a rotatable blade 320 passes through a gap between adjacent lower fixed blades of the lower fixed blade array 360. As shown in Figure 8, the lower fixed blade array 360 is provided below the rotational axis 311 and may extend toward the rotational axis 311. The shredding assembly 300 may also comprise an upper fixed blade array 340, which may comprise one or more upper fixed blades 341. The upper fixed blade array 340 may be provided above the rotatable blade array 310 such that the rotatable blade array 310 is located between the upper fixed blade array 340 and the lower fixed blade array 360. At least one upper fixed blade 341 may extend laterally to the rotational axis 311.

The upper fixed blade array 340 can comprise three upper fixed blades 341 distributed along the rotational axis 311. The upper fixed blade array may be configured such that two, preferably three, more preferably four or more rotatable blades 320 are received in the gap provided between an adjacent pair of upper fixed blades 341. In the arrangement shown, four rotatable blades 320 are positioned along the rotational axis 311 between a first upper fixed blade and a second, adjacent upper fixed blade. The blades can be advantageously arranged to provide a scissor-type action between cutting faces of the rotatable blades and the fixed blades. When one or more of the rotatable blades passes adjacent one or more lower blades, an acute angle (i.e. between 0 and 90 degrees) may be formed between their oppositely oriented cutting faces. Providing such a scissor action using an acute angle between opposing cutting faces can improve the cutting action. Providing such acute angles between cutting faces on both sides of the rotatable blades and preferably on both sides of the lower fixed blades, can help to facilitate effective cutting in both directions of rotation of the rotatable blade array. Multiple cutting and shredding locations can be provided by providing such acute angles between one or two or more faces of at least one rotatable blade and any cutting face of the different fixed blades. This can include cutting faces of the upper fixed blades 340 which either face towards or away from the rotational axis 311, and in particular cutting faces 362 of the lower fixed blades oriented in either direction, or preferably two opposing directions, relative to the direction of rotation of the rotatable blade array. The interfaces can all contribute to more effective and efficient shredding, particularly in combination.

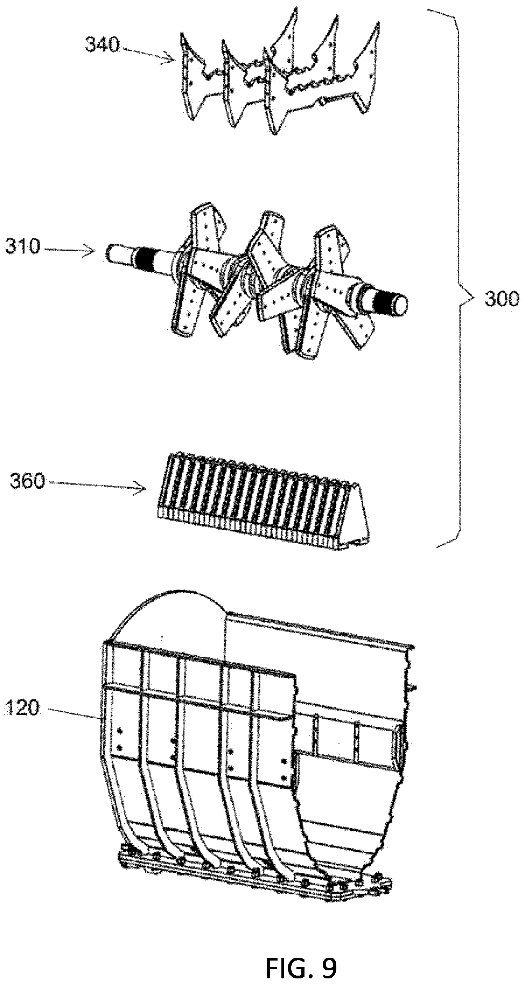

Figure 9 illustrates an exploded view of the shredding assembly 300 and a receiver or waste processing vessel 120. The waste processing vessel 120 is configured to receive waste to be shredded by the shredding assembly 300. As such, the waste processing vessel 120 may be rotatable in order to permit a user to load the waste processing vessel 120 with waste to be shredded, and to dispense the shredded waste after processing. Figure 9 illustrates a position of the waste processing vessel 120 in a shredding position. Therefore, the waste processing vessel 120 is configured such that waste to be shredded collects at the bottom of the waste processing vessel 120, such that the waste collects in the vicinity of the lower fixed blade array 360 provided in fixed relation to the waste processing vessel 120. The rotatable blade array 310 may be rotatably mounted to the waste processing vessel 120 so that the rotatable blades 320 can rotate relative to the waste processing vessel 120. The upper fixed blade array 340 may be provided in fixed relation to the waste processing vessel 120.

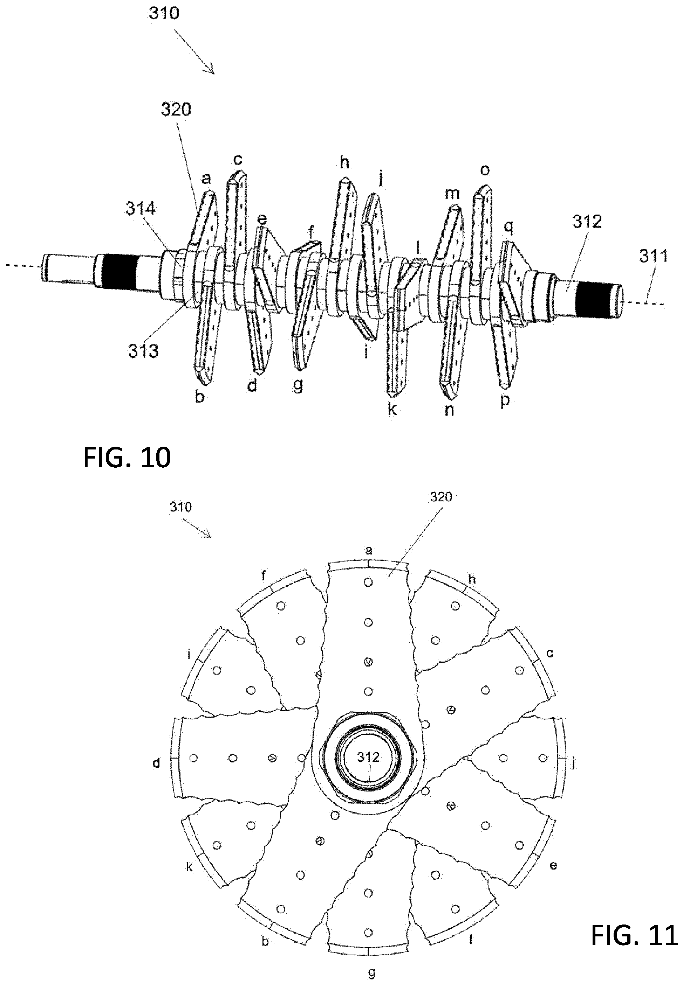

Figure 10 illustrates the rotatable blade array 310. The rotatable blade array 310 comprises a plurality of rotatable blades 320 which can be fixed to a shaft 312. The rotatable blade array 310 may comprise a plurality, such as at least four, rotatable blades 320, preferably at least eight, preferably at least twelve, preferably at least 16 rotatable blades 320. In the arrangement shown, the rotatable blade array 310 comprises 17 rotatable blades 320 fixed to the shaft 312. The rotatable blades 320 can be spaced at regular intervals along the rotational axis 311. In the arrangement shown, a locking nut 314 is provided between an end of the shaft 312 and a first rotatable blade 320a. The rotatable blade array 310 can be configured such that the plurality of rotatable blades 320 extend from the shaft 312 at a variety of angles relative to one another. In other words, at least some of the plurality of rotatable blades 320 are offset from each other with respect to a circumferential direction of the rotational axis 311.

The rotatable blade array 310 may comprise a first rotatable blade 320a and a second rotatable blade 320b adjacent to the first rotatable blade 320a along the rotational axis 311. Each of the first rotatable blade 320a and the second rotatable blade 320b comprises a main body extending from the shaft 312 in a radial direction with respect to the rotational axis 311, and a first and a second cutting face configured to cut waste in a circumferential direction with respect to the rotational axis 311. In this respect, the first and second cutting faces can be substantially perpendicular to a path of rotation of the rotatable blade 320, for example between 80 and 100 degrees to the path of rotation.

At least one adjacent pair of rotatable blades 320 may be separated by a spacer 313 provided around the shaft 312. The main body of each rotatable blade 320 may be connected to a fixing portion which surrounds the rotatable shaft 312 in order to facilitate fixing thereto. As will be described later, the main body of the rotatable blade 320 can be substantially planar such that the main body is normal to a direction parallel to the rotational axis 311. The first and second cutting faces of a rotatable blade 320 can be symmetrical with each other with respect to a centreline of the main body.

An angular offset between the first rotatable blade 320a and the second rotatable blade 320b may be a fraction of a turn wherein the fraction is at least one twenty-fourth, or at least one twelfth, one tenth, one eighth, one sixth, or one quarter. The angular offset may be at least a quarter radian, or a half radian, three quarter radians, one radian, or three half radians. An angle between the centreline of the first rotatable blade 320a and the second rotatable blade 320b may be at least 30 degrees, and may be an integer multiple of 30 degrees.

Figure 11 shows an end view of the rotatable blade array 310 as viewed from the left-hand end of Figure 10. With reference to Figures 10 and 11, the rotatable blade array 310 may be arranged such that an angle between the centrelines of any pair of rotatable blades is an integer multiple of 30 degrees. The rotatable blade array 310 may be arranged such that the centrelines of each adjacent pair of rotatable blades are offset by an angle of at least 30 degrees, preferably at least 60 degrees, preferably at least 90 degrees. In this way, the rotatable blades 320 of the rotatable blade array 310 can be arranged such that each rotatable blade 320 in a group of up to twelve successive rotatable blades 320 is orientated at a different angle to the remaining rotatable blades 320 in the group.

In the arrangement shown, the rotatable blade array 310 comprises a group of twelve rotatable blades 320, labelled 320a to 320l, wherein each of the group of rotatable blades is orientated at a different angle with respect to the rest of the group. The rotatable blade array 310 includes 17 rotatable blades 320a to 320q. Since there are only twelve distinct orientations of rotatable blades 320 under the constraint that each rotatable blade 320 is offset by an integer multiple of 30 degrees in the circumferential direction, the remaining five rotatable blades 320m to 320q may repeat the angular sequence of the rotatable blades 320a to 320e. The relative angle of each of the rotatable blades 320a to 320q with respect to the rotatable blade 320a is provided in the following sequence: 0, 210, 60, 270, 120, 330, 180, 30, 300, 90, 240, 150, 0, 210, 60, 270, 120. It will be appreciated that each of these corresponds to a clockwise direction, for example '0 degrees' corresponds to 12 o'clock, and 210 degrees corresponds to 7 o'clock, and so on.

It will be appreciated that the above sequence illustrates just one arrangement for organising the rotatable blades 320a-320q along the shaft 312, but that many other combinations of angles exist. In the arrangement shown, a first rotatable blade 320a is offset from a second rotatable blade 320b by an angle of 150 degrees. The rotatable blade array 310 may comprise a third rotatable blade 320c, arranged such that the second rotatable blade 320b is provided between the first rotatable blade 320a and the third rotatable blade 320c. The third rotatable blade 320c may be offset from the first rotatable blade 320a by 60 degrees. The rotatable blade array 310 can comprise further rotatable blades provided sequentially in order to provide a fourth rotatable blade 320d, a fifth rotatable blade 320e, a sixth rotatable blade 320f and so on.

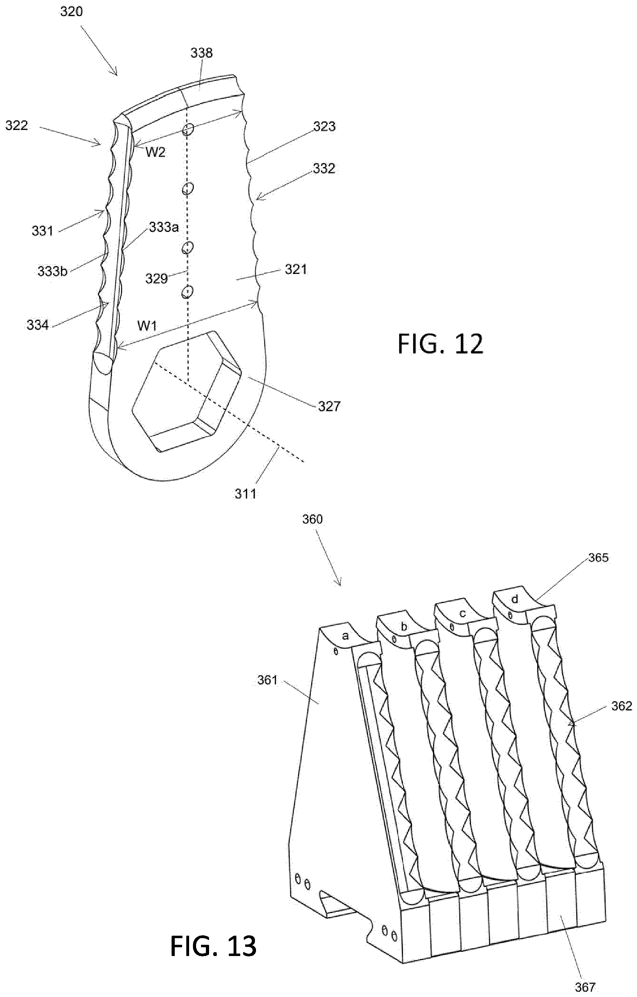

Figure 12 illustrates a rotatable blade 320. The rotatable blade 320 has a first cutting face 331 and a second cutting face 332 which can define opposite sides of a main body 323. The rotatable blade 320 is configured to be fixed to the shaft 312 (see Figure 10) such that the rotatable blade 320 can rotate about a rotational axis 311. The rotatable blade 320 may comprise a fixing portion 327 configured to fix the rotatable blade 320 to the shaft 312. It will be appreciated that in other arrangements, the rotatable blade 320 may be integral with the shaft 312.

In the arrangement shown, the fixing portion 327 comprises a hexagonal aperture configured to receive a corresponding hexagonal shaft 312 (not shown). The hexagonal aperture is shaped as a regular hexagon, that is, each internal angle being 120 degrees, and is oriented such that the centreline perpendicularly bisects two opposite edges of the hexagon. It will be appreciated that six different orientations of the rotatable blade can be achieved on a single hexagonal shaft using the rotatable blade 320 of Figure 12. In order to provide the remaining six orientations to permit angular offsets of 30 degrees, the rotatable blade array 310 may comprise an alternative rotatable blade identical in all respects except that the hexagonal aperture is rotated by 30 degrees relative to the main body 323. This alternative rotatable blade (not shown) comprises a hexagonal aperture oriented such that the centreline bisects two opposite angles of the hexagon. As has been described above, it will be appreciated that other forms of shaft and aperture can be used for securing the rotatable blades 320 to a shaft.

The rotatable blade 320 may comprise a first side face 321 and a second side face 322. The first and second side faces can define opposite sides of the main body 323, which may be perpendicular to the first and second cutting faces 331, 332. The first and second side faces 321, 322 may be arranged normal to a direction parallel to the rotational axis 311. At least one of the first and second cutting faces 331, 332 may comprise a recess 334 extending at least partially along the radial extent of the cutting face. At least one cutting face 331, 332 may comprise a plurality of serrations comprising a first set of serrations 333a which may define part of the first side face 321 and a second set of serrations 333b which may define part of the second side face 322. The first and second sets of serrations 333a, 333b can be disposed on opposite sides of a cutting face 331, 332. In this way, the sets of serrations 333a, 333b can be provided on the rotatable blade 320 at different positions along the rotational axis.

In the arrangement shown, the recess 334 provides a part-cylindrical surface which extends between the first set of serrations 333a and the second set of serrations 333b. The first and second set of serrations 333a, 333b may be scalloped. At least one serration of the first and/or second set of serrations 333a, 333b may be substantially pyramidal. In this way, at least one serration may have a three-dimensional structure defined by four surfaces converging at a vertex. One surface of the pyramid structure may be coplanar with the first and/or second side face 321, 322.

As shown in Figure 12, the structure of the blade differs from known circular toothed blades in that the main body 323 extends in only one direction from the rotational axis 311. In this way, the rotatable blade 320 has a maximum order of rotational symmetry of one with respect to the rotational axis 311. The rotatable blade 320 can be configured such that its centre of mass is offset from the rotational axis 311. A centreline 329 can be defined between the first and second cutting faces 331, 332. In the arrangement shown, the fixing portion 327 comprises a part-annular portion disposed about the rotational axis 311. The first cutting face 331 extends from the fixing portion 327 at a first tangent and the second cutting face 332 extends from the fixing portion 327 at a second tangent, and the centreline 329 bisects an angle between the first tangent and the second tangent. The centre of mass of the rotatable blade 320 may be along the centreline 329 in a region between the first and second cutting faces 331, 332. The angle between the first tangent and the second tangent may be approximately five degrees. The main body 323 of the rotatable blade 320 may therefore have a plane of symmetry defined by the centreline 329 and the rotational axis 311.

In the illustrated arrangement, the first side face 321 defines a first width W1 perpendicular to the centreline 329 between the first cutting face 331 and the second cutting face 332. The first side face 321 also has a second width W2 perpendicular to the centreline 329 between the first cutting face 331 and the second cutting face 332 at a position further from the rotational axis 311 than the first width W1. The first width W1 may be greater than the second width W2. The width of the main body 323 may increase linearly with increased radial distance from the rotational axis 311. The rotatable blade 320 may further comprise a tip portion 338. The tip portion 338 may be arcuate between the first and second cutting faces 331, 332. The tip portion 338 may provide a substantially triangular portion, which may be truncated, between the first and second side faces 321, 322.

Figure 13 illustrates a lower fixed blade array 360. The shape of the lower fixed blade array 360 may be described as a substantially triangular prism containing slots configured to permit the passage of the rotatable blade array 310 therethrough. The lower fixed blade array 360 is configured to be fixed to the waste processing vessel 120, in particular a bottom portion thereof where waste processing occurs. The lower fixed blade array 360 comprises a plurality of lower fixed blades 361. The lower fixed blade array 360 may comprise one more lower fixed blade 361 than the number of rotatable blades 320, so that each rotatable blade 320 can be interspersed between two lower fixed blades 361. The lower fixed blade array 360 may be arranged in a linear array.

The lower fixed blade array 360 may comprise four lower fixed blades 361, preferably at least eight, preferably at least twelve, preferably at least 16 lower fixed blades 361. In the arrangement shown in Figure 9, the lower fixed blade array 360 comprises 18 lower fixed blades 361 in order to receive 17 rotatable blades 320 in the gaps between adjacent lower fixed blades 361. Figure 13 can illustrate an end portion of the lower fixed blade array 360 of Figures 8 and 9. In the illustrated example, the lower fixed blade array 360 partially shown in Figure 13 comprises a first lower fixed blade 361a and a second lower fixed blade 361b adjacent the first lower fixed blade 361a. The first rotatable blade 320a can be configured to be received in the gap provided between the first lower fixed blade 361a and the second lower fixed blade 361b. As such, the gaps between adjacent lower fixed blades 361 are configured to receive a rotatable blade 320, when the rotatable blade 320 rotates about its rotational axis 311.

The lower fixed blades 361 may comprise a cutting face 362 which may be arranged in a similar manner to the cutting faces 331, 332 of the rotatable blade 320 and may have a similar arrangement of serrations. In the arrangement shown, the first lower fixed blade 361a defines an end of the lower fixed blade array 360 and its cutting faces may only define a first set of serrations, configured to co-operate with a set of serrations on the first rotatable blade 320a during rotation thereof. However, the other lower fixed blades 361b-361d may comprise two sets of serrations similarly to the rotatable blades 320.

The lower fixed blade array 360 may comprise a support structure at a base thereof configured to be connected to the waste processing vessel 120, and a support structure may include spacers 367 configured to separate adjacent lower fixed blades 361. The lower fixed blades 361 may comprise a substantially triangular portion, which may be truncated by a tip portion 365. The tip portion 365 may be provided on an upper end of a lower fixed blade 361 and may comprise a concave shape corresponding to an outer surface of the shaft 312. Although not shown in Figure 13, it will be appreciated that a cutting face may be provided on both sides of a given lower fixed blade 361 such that, when a rotatable blade 320 passes through the gap in either direction, the cutting faces 362 of the lower fixed blade 361 can co-operate with the cutting faces 331, 332 of the rotatable blade 320 in order to shred waste. An angle between the cutting faces 362 may be between 30 and 45 degrees, preferably between 35 and 40 degrees.

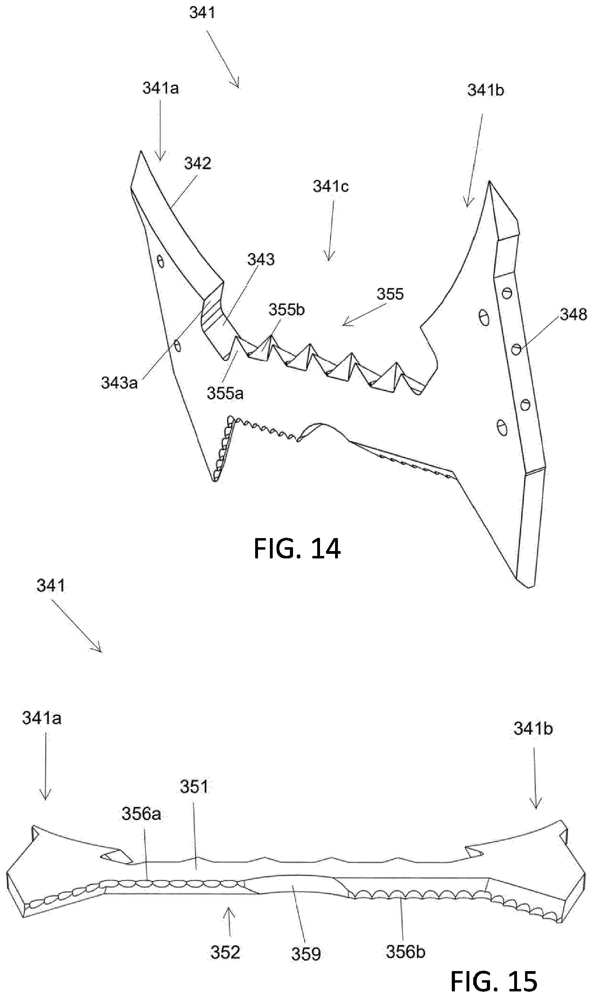

Figure 14 illustrates an upper fixed blade 341 of the upper fixed blade array 340 and Figure 15 illustrates a bottom view thereof. As shown in Figure 9, the upper fixed blade 341 is configured to be fixed to the waste processing vessel 120. In this respect, the upper fixed blade 341 can comprise mounting portions having fixing means 348, which may be threaded holes for receiving bolts. The upper fixed blade 341 may comprise an intermediate region 341c between a first end region 341a and a second end region 341b. In the arrangement shown, the upper fixed blade 341 comprises a first side face 351 and a second side face 352 opposite the first side face 351. The first and second side faces 351, 352 can be configured to be perpendicular to the rotational axis 311 of the rotatable blade array 310 when the upper fixed blade array 340 is mounted within the shredding assembly 300 of Figures 8 and 9.

At least one of the first and second end regions 341a, 341b may comprise a cutting face which may have a cutting region such as a knife edge 342. The knife edge 342 can be configured to face in a substantially upward direction with respect to the waste processing vessel 120 and can be configured to co-operate with a rotating blade 320 in order to cut waste. The knife edge 342 labelled in Figure 14 on the first end region 341a may be provided such that a surface defining the knife edge 342 is coplanar with the second side face 352. The knife edge 342 may be provided as a bevel between the first side face 351 and the second side face 352. Similarly, the second end region 341b may comprise a knife edge having a surface coplanar with the first side face 351.

As shown in Figure 14, the intermediate region 341c may comprise a serrated face 355 configured to face away from the rotatable blade array 310 when assembled therewith in the waste processing vessel 120. In the arrangement shown, the serrated face 355 comprises a plurality of spikes, including a first spike 355a and a second spike 355b. The first and/or second spikes 355a, 355b may be substantially pyramidal. The serrated face 355 may be configured such that the first spike 355a comprises a wall that is coplanar with the first side face 351 and the second adjacent spike 355b comprises a wall which is coplanar with the second side face 352. The upper fixed blade 341 may comprise a cut-out portion 343 which may comprise a substantially upward facing recess, which may be located between the cutting face 355 and the first and/or second end region 341a, 341b. The cut-out portion may comprise a sloped surface between the first side face 351 and the second side face 352. A further face 343a may be provided on the upper fixed blade 341 between the first end region 341a and the intermediate portion 341c, and/or between the second end region 341b and the intermediate portion 341c. The further face 343a can be oriented toward the intermediate portion 341c such that it faces the serrated face 355. In the illustrated arrangement, the further face 343a forms a wall of the cut-out portion 343.

With reference to Figure 15, a bottom side of the upper fixed blade 341, that is, the side facing towards the rotatable blade array 310 and towards the lower fixed blade array 360 in a downwards direction, may comprise further cutting means. In the arrangement shown, a first portion of the upper fixed blade 341 corresponding to the first end region 341a is provided with first lower serrations 356a. The first lower serrations 356a are provided on an edge defined between a bottom surface and the first side surface 351. A second portion of the upper fixed blade 341 corresponding to the second end region 341b is provided with second lower serrations 356b. The second lower serrations 356b are provided on an edge defined between the bottom surface and the second side surface 352. A concave portion or arch 359 may be provided between the first lower serrations 356a and second lower serrations 356b and may be configured to correspond to the outer surface of the shaft 312 when assembled therewith.

### Control System

One aspect of improvement identified is controlling a shredding device to move into an operating position (which may be one of several operating positions) in which combined shredding and cleaning and/or disinfection of contaminated or biohazardous waste can take place simultaneously within the shredding device.

In one aspect, there is provided a control system for a waste processing apparatus. The control system comprises a controller configured to perform one or more of the following features: control an actuator of the waste processing apparatus to move a shredding device of the waste processing apparatus into at least a shred position in which a vessel of the shredding device is able to hold liquid; control a fluid delivery device of the waste processing apparatus to deliver a treatment fluid into the vessel of the shredding device; and control the shredding device to perform a shredding function to shred waste within the vessel of the shredding device.

The controller may be configured to receive sensor data from a sensor associated with the vessel, and determine, based on the sensor data, that the vessel is in the shred position.

The controller may be configured to, in response to determining that the vessel is in the shred position, control a closure mechanism of the waste processing apparatus to move a closure arrangement to close the vessel of the shredding device.

The controller may be configured to, in response to determining that the vessel is in the shred position, control one or more locking mechanisms of the waste processing apparatus to lock the shredding device in the shred position.

The controller may be configured to control the actuator to move the shredding device into the shred position in response to receiving an input signal from a user input device of the waste processing apparatus.

The controller may be configured to control the fluid delivery device to deliver a predefined amount of treatment fluid into the vessel of the shredding device.

The waste processing apparatus may comprise a blade arrangement motor configured to drive a blade arrangement of the shredding device to perform the shredding function. The controller may be configured to control the blade arrangement motor to control the rotation speed and/or rotation direction of the blade arrangement.

The controller may be configured to control the blade arrangement motor to alternate between different rotation directions.

The controller may be configured to: control the blade arrangement motor to drive the blade arrangement in a first mode in which the blade arrangement rotates at a first speed, and in response to determining that the torque of the blade arrangement motor has decreased below a first threshold torque value, control the blade arrangement motor to drive the blade arrangement in a second mode in which the blade arrangement motor rotates at a second speed greater than the first speed.

The controller may be configured to control the shredding device to terminate the shredding function after a predefined shredding time has elapsed and/or in response to determining that a torque value of the shredding device is below a second threshold torque value.

The controller may be configured to: control the actuator to move the shredding device into an emptying position in which waste can be emptied out of the vessel of the shredding device, and control a conveying means to move said waste into a waste bin.

The controller may be configured to control a closure mechanism of the waste processing apparatus to move a closure arrangement to close the vessel of the shredding device.

The controller may be configured to control one or more locking mechanisms of the waste processing apparatus to lock the shredding device in the shred position.

The controller may be configured to initiate a neutralisation process. The neutralisation process may include activating the waste processing apparatus to deliver a neutralisation agent to the treatment fluid. The neutralisation process may include opening the vessel to allow a neutralisation agent to be added into the vessel. The neutralisation process may include controlling a fluid delivery device so as to deliver a neutralising agent into the vessel. The controller may be configured to control the fluid delivery device to deliver the neutralising agent into the vessel after terminating the shredding function of the shredding device. The neutralisation process may include delivering a neutralising agent to the treatment fluid outside of the vessel.

The controller may be configured to permit the waste processing apparatus to perform a waste processing operation in response to verifying one or more of the following conditions: a user has entered valid credentials for operating the waste processing apparatus; a water pressure of a water supply for the waste processing apparatus is at least a minimum pressure value; and a level of treatment fluid in a treatment fluid container of the waste processing apparatus is sufficient.

The controller may be configured to control the shredding device to perform the shredding function in response to verifying one or more of the following conditions: a loading hatch of the waste processing apparatus is closed; the shredding device is in the shred position; the vessel of the shredding device is closed by a closure arrangement; the shredding device is locked in the shred position by one or more locking mechanisms; a predefined amount of treatment fluid has been added to the vessel; and a predefined amount of water has been added to the vessel.

The controller may comprise one or more processors.

In another aspect, there is provided a waste processing apparatus comprising one or more of the following features: a shredding device comprising a vessel for holding waste and treatment fluid during processing, wherein the shredding device is configured to perform a shredding function to shred waste within the vessel; an actuator configured to move the shredding device into a shred position in which the vessel is able to hold liquid; a fluid delivery device configured to deliver a treatment fluid into the vessel of the shredding device; and a control system as set out above. The controller of the control system is communicatively coupled to the shredding device, the actuator and the fluid delivery device.

The actuator may comprise a motor configured to rotate the shredding device into at least the shred position.

The fluid delivery device may comprise: a container for holding treatment fluid; and a pump configured to pump treatment fluid from the container into the vessel of the shredding device.

In another aspect, there is provided a method of controlling a waste processing apparatus. The method comprises one or more of the following features: controlling an actuator of the waste processing apparatus to move a shredding device of the waste processing apparatus into a position in which the shredding device is able to hold liquid; controlling a fluid delivery device of the waste processing apparatus to deliver a treatment fluid into the shredding device; and controlling the shredding device to perform a shredding function to shred waste within the shredding device.

In another aspect, there is provided a computer-readable medium comprising instructions which, when executed by a processor, cause the processor to perform one or more of the following features: control an actuator of a waste processing apparatus to move a shredding device of the waste processing apparatus into a position in which the shredding device is able to hold liquid; control a fluid delivery device of the waste processing apparatus to deliver a treatment fluid into the shredding device; and control the shredding device to perform a shredding function to shred waste within the shredding device.

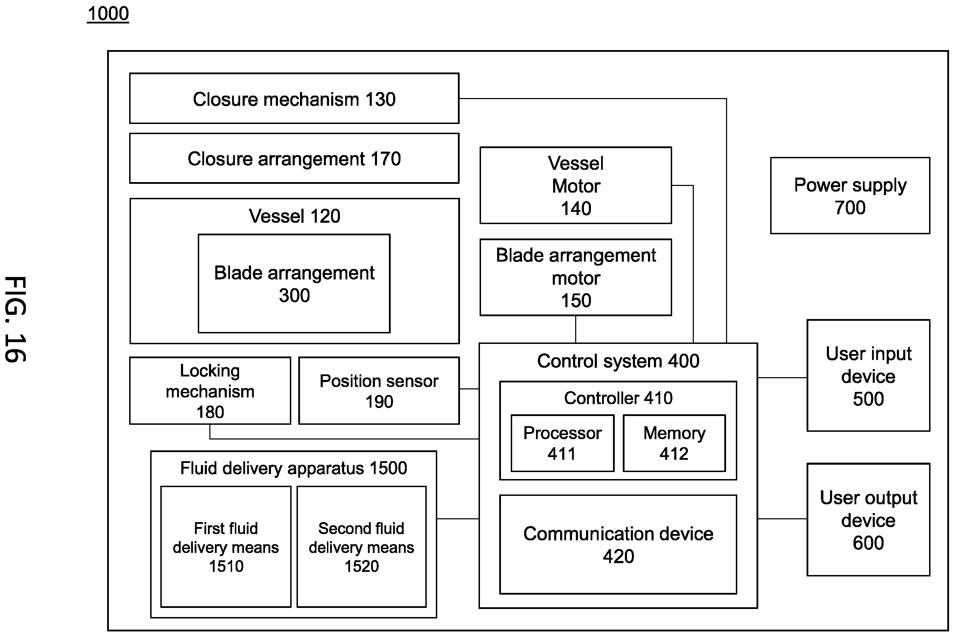

Figure 16 is a block diagram showing various components of the waste processing apparatus 1000 shown in Figure 1. The waste processing apparatus 1000 includes a control system 400 which controls the overall operation of the waste processing apparatus 1000. One or more components of the control system 400 may be disposed within the control system enclosure 1004 of the waste processing apparatus 1000. Figure 16 shows various connections between the control system 400 and other components of the waste processing apparatus 1000. However, these connections are not intended to be limiting, and other connections could be used. The control system 400 may be connected to one or more components directly (i.e. with no intervening components), or may be connected to one or more components via other components of the waste processing apparatus 1000.

The control system 400 includes a controller 410. The controller 410 may be configured to control the waste processing apparatus 1000 to perform any of the functions or processes described herein. The controller 410 is configured to control the vessel motor 140 to move the vessel 120 into various positions, such as the positions described above with reference to Figures 6A to 6C. In particular, the controller 410 is configured to control the vessel motor 140 to move the vessel into a shredding position, which may be the same as, or similar to, the second position described above in relation to Figure 6B. The controller 410 is configured to, when the vessel 120 is in the shredding position, control the fluid delivery apparatus 1500 to deliver treatment fluid into the waste processing vessel 120, and control the shredding device to perform a shredding function to shred waste within the vessel 120. This allows for simultaneous treatment and shredding of the waste.

In some examples, the controller 410 may include a memory and one or more processors configured to execute instructions stored in the memory to perform control operations, such as moving the vessel 120 into a given position. In other examples, the controller 410 may be implemented using hardware. In some cases, the controller 410 may perform control operations based on a user input. Such a user input may be received from an input device of the waste processing apparatus 1000. Alternatively, or in addition, the controller 410 may perform control operations based on input received from an external system.

In the present example, the controller 410 is a programmable logic controller (PLC). The PLC 410 includes a processor 411 and a memory 412. The processor 411 is configured to receive signals from input devices (such as sensors) and make decisions based on a program stored in the memory 412 to control devices such as the vessel motor 140. The memory 412 may store instructions that are executable by the processor 411 to perform any of the operations described herein.

The control system 400 includes a communication device 420 which enables communication between the controller 410 and various devices in the waste processing apparatus 1000, such as the vessel motor 140. In the present example, the communication device 420 is an ethernet switch. The communication device 420 may also allow for communication between the controller 410 and one or more external devices or systems. For example, the communication device 420 may be connected to a cloud data storage system via a router (not shown), and the controller 410 can send data to the cloud data storage system via the router. In some cases, the router may buffer the data locally and periodically send the data to the cloud data storage system. If an internet connection is not present, data may be buffered locally in the memory 412 until the communication device 420 can connect to the cloud data storage system again. The data can then uploaded to the cloud data storage system from the memory 412. Data stored in the cloud data storage system may be used for (for example) online dashboards, automatic reporting or automatic service ticketing.

The waste processing apparatus 1000 includes at least one user input device 500 and at least one user output device 600. The input device 500 allows the user to control various operations of the waste processing apparatus 1000. The input device may be, for example, a button (e.g. an emergency stop button) or a switch. The output device 600 provides the user with information about the state of the waste processing apparatus, e.g. the current processing cycle that is in progress. The output device 600 may be, for example, a display device. In some examples, the input device 500 and the output device 600 may be implemented together in the same device, such as a touch screen display device.

The waste processing apparatus 1000 includes a power supply 700 which is configured to supply direct current (DC) power to electrical components of the waste processing apparatus 1000, such as the vessel motor 140 and the blade arrangement motor 150.

The controller 410 may be configured to determine the current position of the vessel 120 based on data from a sensor 190 which is associated with the vessel 120. The sensor 190 may be a position sensor, or may be an image sensor such as a camera. In the present example, the sensor 190 is a rotary encoder which outputs a signal indicating the absolute angular position of the shaft of the vessel motor 140. In examples where an image sensor is used, the controller 410 may analyse images received from the image sensor to determine the position of the vessel 120.

The controller 410 can determine that the vessel 120 is in a given position (e.g. the shred position) based on the data from the sensor 190. For example, if the position signal from the rotary encoder indicates that the angular position of the shaft is 5°, then the controller 410 may determine that the vessel 120 is in the shred position. Data specifying the correspondence between different angular positions and different operational positions of the vessel 120 may be stored in the memory 412.

The controller 410 may control the closure mechanism 130 and/or the locking mechanisms 180 based on feedback from the sensor 190. In the present example, the controller 410 is configured to control the closure mechanism 130 to move the closure arrangement 170 into the closed position only when it has determined that the vessel 120 is in the shred position. Similarly, the controller 410 is configured to control the locking mechanisms 180 to lock the vessel 120 in position only when it has determined that the vessel 120 is in the shred position. This reduces the chance of errors occurring during the engagement of the closure arrangement 170 and/or the locking mechanisms 180. In other examples, the controller 410 may control the closure arrangement 170 and/or the locking mechanisms 180 without relying on feedback from the sensor 190, or the sensor 190 may be omitted altogether.

The controller 410 is configured to control the fluid delivery apparatus 1500 to deliver one or more fluids into the vessel 120. The controller 410 may be configured to control the fluid delivery apparatus 1500 to deliver predefined amounts of the one or more fluids into the vessel 120 or more generally to the treatment fluid. The amount(s) of fluid(s) delivered into the vessel 120 may be determined by the controller 410 based on feedback from a flowmeter, e.g. flowmeter 1515.

In the present example, controller 410 is configured to control the first fluid delivery means 1510 of the fluid delivery apparatus 1500 to deliver treatment fluid into the chamber 1201 of the vessel 120. In the present example, the controller 410 is configured to control the first fluid delivery means 1510 of the fluid delivery apparatus 1500 to deliver treatment fluid into the chamber 1201 of the vessel 120 only when it has determined that the vessel 120 is locked in position and the closure arrangement 170 is covering the vessel 120. In the present example, the controller 410 is configured to control the treatment fluid pumping means 1513 so as to deliver a predefined amount of the treatment fluid into the chamber 1201, based on feedback from the flowmeter 1515. The predefined amount of treatment fluid may be any of the amounts set out herein.

In the present example, the controller 410 is also configured to control the second fluid delivery means 1520 so as to deliver a predefined amount of diluent (which may be water) into the chamber 1201, based on feedback from the flowmeter 1515. The predefined amount of diluent may be any of the amounts set out herein. This results in the liquid mixture in the chamber 1201 having a particular desired concentration of treatment fluid, which may be any of the concentrations set out herein. Data defining the predefined amounts of treatment fluid and/or diluent may be stored in the memory 412 of the controller 410.

In some examples, the controller 410 may be configured to initiate a neutralisation process. The neutralisation process may include activating the waste processing apparatus 1000 to deliver a neutralisation agent to the treatment fluid. The neutralisation process may include controlling the closure mechanism 130 to open the vessel 120 to allow a neutralisation agent to be added into the chamber 1201, or alternatively may include controlling any one of the first to third fluid delivery means 1510, 1520, 1530 so as to deliver a neutralising agent into the chamber 1201. The controller 410 may be configured to control one of the first to third fluid delivery means 1510, 1520, 1530 to deliver the neutralising agent into the chamber 1201 after terminating the shredding function of the shredding device. Alternatively, or in addition, the neutralisation process may include delivering neutralising agent to the treatment fluid outside of the vessel 120. For example, the treatment fluid may be separated from the shredded waste and collected in a container or conduit. An example of such a conduit may be a pipe, tube or channel for directing fluid away from the waste processing apparatus 1000 towards, for example, a drain or other external sink. The neutralisation process may include adding the neutralisation agent to the treatment fluid in the container or conduit after it has been separated from the shredded waste. A predefined amount of neutralising agent may be used for the neutralisation process. The predefined amount may be measured based on feedback from a flowmeter. The predefined amount of neutralising agent may be any of the amounts set out herein. Data defining the predefined amount of neutralising agent may be stored in the memory 412 of the controller 410.

The controller 410 is configured to control the blade arrangement motor 150 of the shredding device to rotate the blade arrangement 300 within the vessel 120, in particular when the treatment fluid is present in the chamber 1201. In the present example, the controller 410 is configured to control the blade arrangement motor 150 to rotate the blade arrangement 300 within the vessel 120 only when it has determined that the correct amount of treatment fluid (and in the present case, the correct amount of water) has been added to the chamber 1201.

During operation of the blade arrangement motor 150, the blade arrangement motor 150 will have a given torque, related to the resistance to the blade motion of the waste it is shredding, and the controller 410 may be able to determine a value of the torque by various means. For example, the controller 410 may estimate a torque value of the blade arrangement motor 150 based on the current being used by the blade arrangement motor 150, or may receive a torque value of the blade arrangement motor 150 from a torque sensor (not shown).

The controller 410 can control the blade arrangement motor 150 so as to control, for example, the rotation speed and/or rotation direction of the blade arrangement 300. The controller 410 may select the rotation speed of the blade arrangement 300 based on the torque of the blade arrangement motor 150. For example, the torque of the blade arrangement motor 150 may be relatively high at the beginning of a waste processing cycle due to the high force required to shred the waste. At this stage, the controller 410 may control the blade arrangement motor 150 to rotate the blade arrangement 300 at a low speed, e.g. 10-20 rotations per minute (RPM). As the waste is shredded, the force required to shred the waste decreases, and so the torque of the blade arrangement motor 150 decreases. In response to detecting that the torque of the blade arrangement motor has decreased below a threshold torque value, the controller 410 may increase the rotation speed of the blade arrangement motor 150, e.g. to 30-40 RPM. For example, if the controller 410 estimates the torque of the blade arrangement motor 150 based on the current being used by the blade arrangement motor 150, the controller may increase the rotation speed of the blade arrangement motor 150 in response to the current per phase falling below 10 Amps per phase. At low rotation speeds, smaller pieces of waste may escape the cutting action of the blades, whereas this is less likely at higher speeds. Driving the blade arrangement 300 at an increased speed thus helps to ensure that the waste is shredded to a small particle size. Driving the blade arrangement 300 at an increased speed also helps to effectively mix the waste and the treatment fluid.

In some examples, the controller 410 may control the blade arrangement motor 150 to alternate between rotation in different directions, so as to rotate the blade arrangement 300 in different rotational directions. This allows the blade arrangement 300 to shred waste in both rotational directions. This reduces the force required to achieve complete shredding of the waste compared to shredding in a single rotational direction only, thereby reducing the power required to drive the blade arrangement 300. The rotation direction of the blade arrangement motor 150 may be adjusted in combination with adjusting the rotation speed. The controller 410 may control the blade arrangement motor 150 to periodically alternate between rotation in different directions. Reversing the direction of the blade arrangement 300 periodically has been found to reduce the incidence of waste collecting in a single area of the vessel 120, and may facilitate more efficient shredding. Alternatively, or in addition, the controller 410 may control the blade arrangement motor 150 to change rotation direction in response to determining that the torque value of the blade arrangement motor 150 has exceeded a maximum value (e.g. the current being used by the blade arrangement motor 150 has exceeded a maximum current value, or the torque measured by a torque sensor has exceeded a maximum value). This may, for instance, indicate that the blade arrangement 300 has encountered a hard object, e.g. a piece of steel, which it cannot immediately shred. Reversing the rotation direction may allow for the object to enter the blade arrangement 300 in a different orientation which allows for easier shredding.

In some examples, the controller 410 is configured to control the blade arrangement motor 150 to rotate the blade arrangement 300 for a predefined shredding time, i.e. the blade arrangement 300 stops rotating after the predefined shredding time has elapsed. Data defining this predefined shredding time may be stored in the memory 412 of the controller 410. Alternatively, or in addition, the controller 410 may be configured to control the blade arrangement motor 150 to rotate the blade arrangement until the controller 410 determines that the torque of the blade arrangement motor 150 has fallen below a minimum torque value (e.g. the current being used by the blade arrangement motor 150 has fallen below a minimum current value, such as 6 Amps per phase, or the torque measured by a torque sensor has fallen below a minimum value). Data defining this minimum torque value may be stored in the memory 412 of the controller 410.

In examples where a neutralising agent is added to the vessel 120, the controller 410 may be configured to control the blade arrangement motor 150 to rotate the blade arrangement 300 for a predefined period of time to mix the shredded waste and the treatment fluid with the neutralising agent, and optional diluent, in the bottom of the waste processing vessel 120. The controller 410 may control the blade arrangement motor 150 to rotate the blade arrangement 300 for a period of time in the range of from about thirty seconds to about 10 minutes, or from about 1 minute to about 8 minutes.

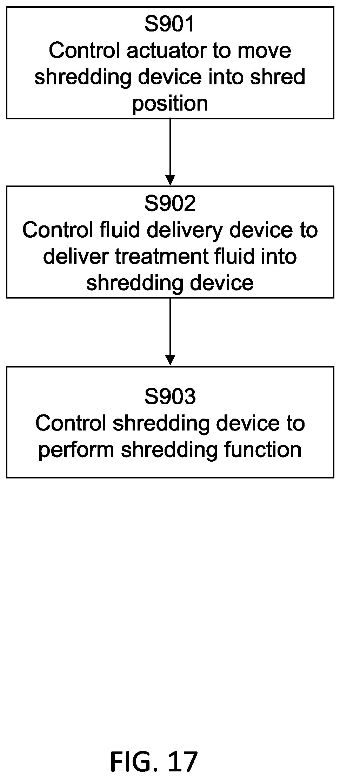

An example of a method of controlling the waste processing apparatus 1000 is shown in Figure 17. The method includes controlling an actuator of the waste processing apparatus to move a shredding device of the waste processing apparatus into a position in which the shredding device is able to hold liquid (S901). The method further includes controlling a fluid delivery device of the waste processing apparatus 1000 to deliver a treatment fluid into the shredding device (S902). The method further includes controlling the shredding device to perform a shredding function to shred waste within the shredding device (S903).

Example operating cycles of the waste processing apparatus 1000 will now be described.

In some circumstances, the controller 410 may verify that certain operating conditions are met before commencing these cycles. If at least one of these operations conditions is not met, the controller 410 will not begin processing. For example, the controller 410 may verify that the water pressure is at least a minimum pressure value (in this case, 1 bar), based on feedback from a water pressure sensor (not shown). Alternatively, or in addition, the controller 410 may verify that the level of treatment fluid in the container of the fluid delivery apparatus is at least a minimum volume (in this case, 0.1 litres), based on feedback from a level sensor (not shown). In some examples, the user may need to present valid security credentials (e.g. via the input device 500) to operate the waste processing apparatus 1000, and the controller 410 will not begin processing until such credentials are received

### Waste Cycle

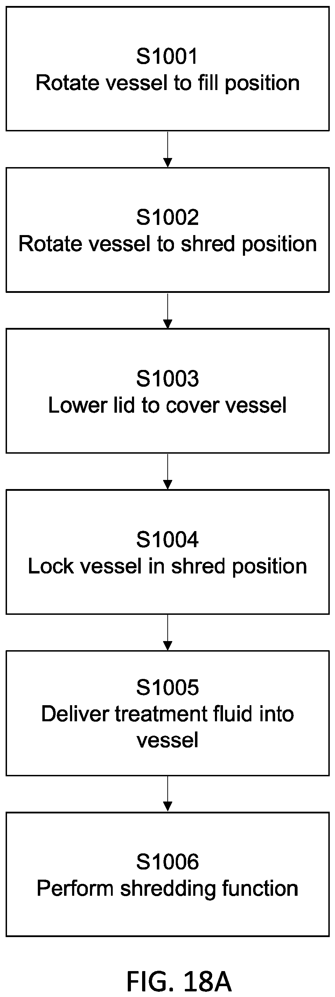

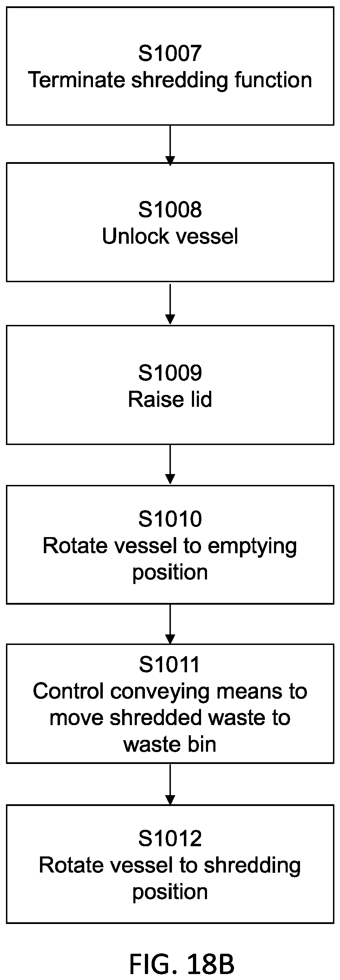

An example of a waste cycle process is shown in Figures 18A and 18B. In the present example, in response to receiving a selection of an "Enable fill position" button from a touch screen display, the controller 410 controls the vessel motor 140 to rotate the vessel 120 to the fill position (S1001) in which the waste receiving opening 1270 of the vessel 120 is presented to the loading hatch 160. In the present case, the rotational position of the vessel 120 in the fill position is 50° from vertical. The controller 410 may determine that the vessel 120 is in the fill position based on feedback from the vessel position sensor 190. In the present example, the controller 410 controls the touch screen display to make a button "Open door" appear grey, and in response to receiving a selection of the button "Open door" from the touch screen display, unlocks the loading hatch 160. The operator opens the loading hatch 160 and loads the vessel 120 with waste to be treated, then closes the loading hatch 160.

The controller 410 determines that the loading hatch 160 has been closed, and controls the touch screen display to change the colour of a button "Lock door and run" to grey. In the present example, in response to receiving a selection of the button "Lock door and run" from the touch screen display, the controller 410 locks the loading hatch 160 and controls the vessel motor 140 to rotate the vessel to the shred position (S1002). The rotational position of the vessel in the fill position may be vertical (i.e. 0 degrees), or close to vertical. In the present case, the rotational position of the vessel 120 in the shred position is 5°.

The controller 410 verifies that the vessel 120 is in the shred position based on feedback from the vessel position sensor 190. In response to verifying that the vessel 120 is in the shred position, the controller 410 controls the lid closure mechanism 130 to lower the lid 170 to cover the vessel 120 (S1003). The controller 410 also controls the linear actuators of the locking mechanisms 180 to move the locking pins 182 into their locking positions, so as to lock the vessel 120 to the chassis 1001 (S1004). In the present example, steps S403 and S404 are performed simultaneously. In other examples, these steps may be performed sequentially.

The controller 410 receives position signals from the lid actuator 130 and the locking mechanisms 180, and determines whether the lid 170 and the locking pins 182 are in the correct positions based on these signals. Once the controller 410 has verified that both the lid 170 and the locking pins 182 are in their positions, the controller 410 controls the fluid delivery apparatus 1500 to deliver treatment fluid (and in the present case, water) into the vessel (S1005). The controller 410 may verify that the correct amounts of treatment fluid and water have been delivered into the vessel 120, based on feedback from flow meters.

The controller 410 controls the blade arrangement motor 150 to rotate the blade arrangement 300 to perform the shredding function (S1006). In the present example, the controller 410 controls the blade arrangement motor 150 to rotate the blade arrangement 300 of the shredding device to perform the shredding function for a predefined shredding time (in the present case, 12 minutes). The controller 410 may control the blade arrangement motor 150 to alternately rotate in forward and reverse directions, thereby alternating the rotating direction of the shredding blades.

Once the controller 410 has determined that the shredding time has elapsed, and that the blade arrangement 300 meets little or no resistance (based on the torque of the blade arrangement motor 150), the controller 410 stops the blade arrangement motor 150, thereby terminating the shredding function (S1007). In the present example, the resistance encountered by the blade arrangement 300 is inferred from the current being consumed by the blade arrangement motor 150. As an example, the current of the blade arrangement motor 150 under load conditions may vary between a first current level, such as 6 Amps per phase and a second current level, such as 13 Amps per phase. In this case, if the shredding function has been performed for the shredding time period, such as 12 minutes and the current being consumed by the blade arrangement motor 150 is less than a first current level, such as 6 Amps per phase, then the controller 410 may stop the blade arrangement motor 150.

The controller 410 then controls the linear actuators of the locking pin mechanisms 180 to move to the unlock positions to unlock the vessel (S1008), and controls the lid actuator 130 to raise the vessel lid 170 (S1009).

The controller 410 then controls the vessel motor 140 to rotate the vessel 120 into the emptying position in which waste can be evacuated from the vessel 120 (S1010). In the present case, the rotational position of the vessel 120 in the emptying position is 180° with respect to vertical. The controller 410 can verify that the vessel 120 is in the emptying position based on feedback from the vessel position sensor 190, in the same way as for the fill position and the shred position.

Since the opening 1270 of the vessel 120 is uncovered in the emptying position, the shredded waste falls out of the vessel 120 onto a conveying means (e.g. an auger). The controller 120 may also control the blade arrangement motor 150 to rotate the blade arrangement 300 to help move the shredded waste out of the vessel 120. The controller 410 controls the conveying means to transport the shredded waste to a waste bin (S1011). Once a predefined time has elapsed, the controller 410 controls the vessel motor 140 to rotate the vessel 120 to the shredding position (S1012).

In some examples, a wash cycle is carried out once the vessel 120 has returned to the shredding position. An example of a wash cycle will now be described. With the vessel 120 in the shredding position, the controller 410 controls the locking mechanisms 180 to engage the locking pins 182 and controls the lid closure mechanism 130 to lower the lid 170 to cover the vessel 120. The controller 410 then controls the second fluid delivery means 1520 to deliver water into the vessel 120. The controller 410 also controls the blade arrangement motor 150 to rotate the blade arrangement 300 for a predefined cleaning time (in the present case, 60 seconds). This helps to clean the vessel 120. After the predefined time has elapsed, the controller 410 controls the locking mechanisms 180 to retract the locking pins 182 and controls the lid closure mechanism 130 to raise the lid 170. The controller 410 controls the vessel motor 140 to rotate the vessel 120 to the emptying position, where the water will be drained out of the vessel 120. The controller 410 controls the vessel motor 140 to return the vessel 120 to the shred position, controls the locking pin mechanisms 180 to engage the locking pins 182 and controls the lid 170 to cover the vessel 120. The ends the wash cycle.

### Validation Cycle

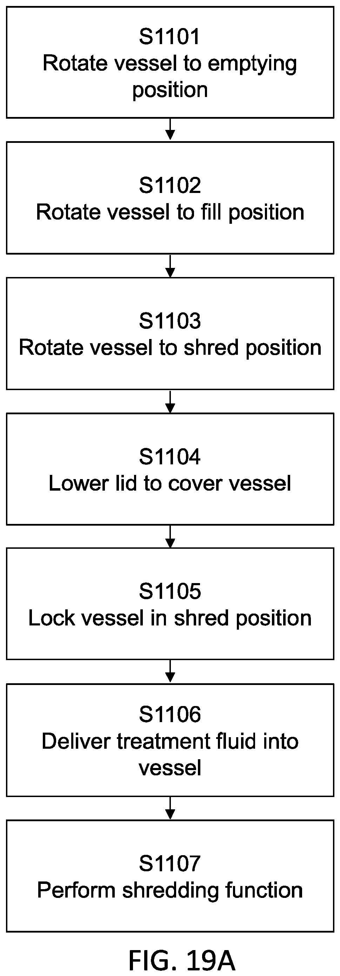

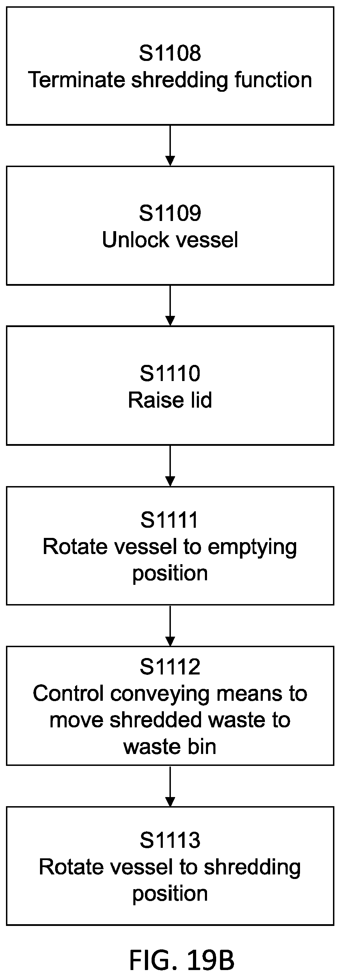

An example of a validation cycle process is shown in Figures 19A and 19B. In the present example, in response to receiving a selection of an "Enable Validation" button from the touch screen display, the controller 410 controls the vessel motor 140 to rotate the vessel 120 to the emptying position (S1101). In this present case, this emptying position is the same as the emptying position described above in relation to the waste cycle. The controller 410 can verify that the vessel 120 is in the emptying position based on feedback from the vessel position sensor 190. In the emptying position, the validation ports 1261, 1262 on the underside of the vessel 120 face the loading hatch 160 of the waste processing apparatus. In the present example, the controller 410 controls the touch screen display to make the button "Install manifold" appear grey, and in response to receiving a selection of the button "Install manifold" from the touch screen, unlocks the loading hatch 160. The operator opens the loading hatch 160 and loads the two validation plugs, with biological indicator strips installed, into the validation ports 1261, 1262. The operator then closes the loading hatch 160.

The controller 410 can determine that the loading hatch 160 has been closed and controls the touch screen display to change the button "Manifold fitted" to appear grey. In response to receiving a selection of the button "Manifold fitted" from the touch screen display, the controller 410 locks the loading hatch 160. The controller 410 controls the vessel motor 140 to rotate the vessel 120 to the fill position (S1102). The controller 410 can determine that the vessel is in the fill position based on feedback from the vessel position sensor 190. In the present example, the controller 410 controls the touch screen display to make the button "Open door" appear grey, and in response to the receiving a selection of the button "Open door" from the touch screen display, unlocks the loading hatch 160. The operator opens the loading hatch 160 and loads the vessel with the waste to be treated, then closes the loading hatch 160.

The controller 410 determines that the loading hatch 160 has been closed, and controls the touch screen display to change the colour of the button "Lock door and run" to grey. In response to receiving a selection of the button "Lock door and run" from the touch screen display, the controller 410 locks the loading hatch 160. The controller 410 controls the vessel motor 140 to rotate the vessel 120 to the shred position (S1103).

The controller 410 verifies that the vessel 120 is in the shred position based on feedback from the vessel position sensor 190. Once the controller 410 has verified that the vessel 120 is in the shred position, the controller 410 controls the lid closure mechanism 130 to lower the lid 170 to cover the vessel (S1104). The controller 410 also controls the linear actuators of the locking pin mechanisms 180 to move the locking pins 182 to lock the vessel 120 to the chassis 1001 (S1105). In the present example, these steps are performed simultaneously. In other examples, these steps may be performed sequentially.

The controller 410 receives position signals from the lid actuator and the locking pin mechanism 180, and determines whether the lid 170 and the locking pins 182 are in the correct positions based on these signals. Once the controller 410 has verified that both the lid 170 and the locking pins 182 are in their positions, the controller 410 controls the fluid delivery apparatus 1500 to deliver treatment fluid (and in the present case, water) into the vessel (S1106). The controller 410 may verify that the correct amounts of treatment fluid and water have been delivered into the vessel 120, based on feedback from flow meters.

The controller 410 controls the blade arrangement motor 150 to rotate the blade arrangement 300 to perform the shredding function (S1107). In the present example, the controller 410 controls the blade arrangement motor 150 to rotate the blade arrangement 300 to perform the shredding function for a predefined shredding time (in the present case, 12 minutes). As with the waste cycle, the controller 410 may control the blade arrangement motor 150 to alternately rotate in forward and reverse directions, thereby alternating the rotating direction of the shredding blades.

Once the controller 410 has determined that the predefined shredding time has elapsed, and that the blade arrangement 300 meets little or no resistance (based on the torque of the blade arrangement motor 150), the controller 410 stops the blade arrangement motor 150 to terminate the shredding function (S1108). In the present example, the resistance encountered by the blade arrangement 300 is inferred from the current being consumed by the blade arrangement motor 150. As an example, the current of the blade arrangement motor 150 under load conditions may vary between a first current level, such as 6 Amps and a second current level, such as 13 Amps per phase. In this case, if the shredding function has been performed for the shredding time period, such as 12 minutes, and the resistance, which may be indicated by a current being consumed by the blade arrangement motor 150, is less than a first current level, such as 6 Amps per phase, then the controller 410 may stop the blade arrangement motor 150.

The controller 410 then controls the linear actuators of the locking pin devices to move to the unlock positions to unlock the vessel 120 (S1109), and controls the lid closure mechanism 130 to raise the vessel lid 170 (S1110).

The controller 410 then controls the vessel motor 140 to rotate the vessel 120 into the emptying position (S1111). The controller 410 can verify that the vessel 120 is in the emptying position based on feedback from the vessel position sensor 190, in the same way as for the fill position and the shred position. The shredded waste falls from the vessel onto the auger. The controller 410 controls the conveying means to transport the shredded waste to the waste bin (S1112).

With the vessel 120 in the emptying position, the controller 410 controls the touch screen display to change the button "Test strip removed" to grey. The controller 410 then unlocks the loading hatch 160. The operator opens the loading hatch 160 and removes the validation plugs, and replaces the validation plugs with blanking plates. In response to receiving a selection of the "Test strip removed button" from the touch screen display, the controller 410 locks the loading hatch 160. In the present example, the operator presses these buttons on the touch screen display in this sequence: "Manifolds removed", "Ports blanked", "Fill door closed" and "Finish cycle". Upon receiving these inputs from the touch screen display, the controller 410 controls the vessel motor 140 to rotate the vessel 120 to the shredding position (S1113).

In some examples, a wash cycle is carried out once the vessel 120 has returned to the shredding position. The wash cycle may be as described above in relation to the waste cycle.

### Reject Cycle

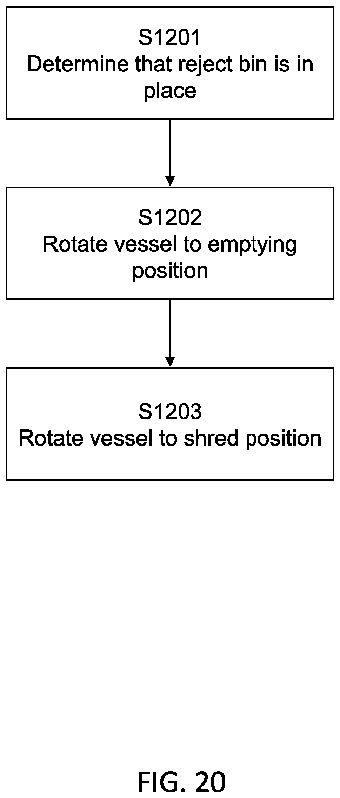

An example of a reject cycle process is shown in Figure 20. The option to start a reject cycle may be enabled when the controller 410 determines that there is an issue with shredding the waste within the vessel 120. For example, if the controller 410 determines that the torque of the blade arrangement motor 150 has exceeded a maximum torque value a predefined number of times, the controller 410 may enable the reject cycle.

In the present example, in response to receiving a selection of a "Reject Cycle" button from the touch screen display, the controller 410 will control the display to present a reject cycle screen. The operator puts the reject bin into position, and plugs in a reject bin identifier plug into an identifier socket. The control determines that the reject bin is in place (S1201) once it registers that the reject identifier plug is in position. This verification step ensures that waste will only be emptied from the vessel 120 when the reject bin is in place, reducing the risk of waste being released into the surrounding environment.

The controller 410 controls the touch screen display to turn the button "Reject bin in position" grey. In response to receiving a selection of the "Reject bin in position" button from the touch screen display, the controller 410 controls the touch screen display to turn the button "Reject" grey. In response to receiving a selection of the "Reject" button", the controller 410 controls the vessel motor 140 to rotate the vessel 120 to the emptying position (S1202). Waste is emptied from the vessel into the reject bin. The controller 410 may control the blade arrangement motor 150 to rotate for a predefined emptying time (e.g. 45 seconds) to help empty the vessel 120. Once the reject cycle has completed, the controller 410 will control the vessel motor 140 to rotate the vessel 120 to the shred position (S1203).

In some examples, a wash cycle is carried out once the vessel 120 has returned to the shredding position. The wash cycle may be as described above in relation to the waste cycle.

In various examples above, operation of the waste processing apparatus 1000 has been described as being controlled by a user. However, in alternative examples, the operation of the waste processing apparatus 1000 may be controlled by an external system (e.g. a testing or processing system which produces contaminated waste or biohazardous waste in use). Such automation further reduces that chances of users coming into contact with biohazardous waste, contaminants, or treatment fluids. In such cases, an automated loading device (e.g. a robotic arm, conveyor or other automated handling system or means) may remove waste from the testing system and load the waste into the waste processing vessel 120 of the waste processing apparatus 1000 without requiring human input. The waste processing apparatus 1000 may then process the waste according to a waste processing cycle and return the waste processing vessel 120 to the loading position so that it can accept another load of waste from the testing system.

Unless otherwise stated, each of the integers described may be used in combination with any other integer as would be understood by the person skilled in the art. Further, although all aspects of the disclosure preferably "comprise" the features described in relation to that aspect, it is specifically envisaged that they may "consist" or "consist essentially" of those features outlined in the claims. In addition, all terms, unless specifically defined herein, are intended to be given their commonly understood meaning in the art.

Further, in the discussion of the various examples, unless stated to the contrary, the disclosure of alternative values for the upper or lower limit of the permitted range of a parameter, is to be construed as an implied statement that each intermediate value of said parameter, lying between the smaller and greater of the alternatives, is itself also disclosed as a possible value for the parameter.

In addition, unless otherwise stated, all numerical values appearing in this application are to be understood as being modified by the term "about". Any means for providing a function in this disclosure may be provided in the form of an apparatus, device or system for performing that function, or configured to provide that function, including the examples specifically described.

Various modifications, whether by way of addition, deletion and/or substitution, may be made to all of the above-described embodiments to provide further embodiments, any and/or all of which are intended to be encompassed by the appended claims.

## Claims (Claims for the following Contracting State(s): AT,BE,CH,DE,DK,ES,FR,GR,IT,LI,LU,NL,SE,MC,PT,IE,SI,LT,LV,FI,RO,MK,CY,AL,TR,BG,CZ,EE,HU,PL,SK,HR,IS,MT,NO,RS,ME,SM)

1. A waste processing apparatus (1000), comprising:
a waste processing vessel (120) for holding waste and treatment fluid during processing;
a blade arrangement (300) for shredding the waste provided in the waste processing vessel (120);
a waste processing zone (Z) in which the waste can be shredded while immersed in a treatment liquid; and
at least one process validation module (700) arranged such that it can hold a process validation sample (701) in fluid communication with the treatment liquid in the waste processing zone (Z) during operation of the blade arrangement (300) to shred the waste in the waste processing zone (Z) while immersed in the treatment liquid.

2. The waste processing apparatus (1000) according to claim 1, wherein the at least one process validation module (700) comprises:
a sample holding portion (710, 720) for holding a validation sample (701); and
a fixing portion (730), configured to retain the process validation module (700) to the waste processing vessel (120) to maintain the sample holding portion (710, 720) in fluid communication with the waste processing zone (Z).

3. The waste processing apparatus (1000) according to claim 2, where the sample holding portion (710, 720) is arranged to be detached from the fixing portion (730) and can be reattached to the fixing portion (730) to form a cavity between the sample holding portion (710, 720) and the fixing portion (730) in which a process validation sample (701) can be received.

4. The waste processing apparatus (1000) according to claim 3, further comprising one or more openings (713) through a wall of the cavity of the sample holding portion (710, 720), such that fluid from the waste processing zone (Z) can pass into the cavity, wherein the at least one process validation module (700) preferably comprises a plurality of sample holding portions (710, 720) arranged to be able to hold a plurality of process validation samples (701) in fluid communication with the waste processing zone (Z) of the waste processing apparatus (1000).

5. The waste processing apparatus (1000) according to any of the preceding claims, wherein the waste processing vessel (120) comprises at least one port (1261, 1262) providing a passage through a wall (1220) of the waste processing vessel (120), arranged to facilitate fluid communication between a sample holding portion (710, 720) of the process validation module (700) and the waste processing zone (Z) of the waste processing vessel (120).

6. The waste processing apparatus (1000) according to claim 5, further comprising a fluid passage extending outwardly from the wall (1220) of the waste processing vessel (120) and arranged to facilitate communication of fluid from the waste processing zone (Z) to the sample holding portion (710, 720) of the process validation module (700) and/or to allow at least a portion of the validation module (700) to enter the waste processing zone (Z).

7. The waste processing apparatus (1000) according to any preceding claim, wherein the sample holding portion (710, 720) projects from the fixing portion (730) toward an interior of the waste processing vessel (120) when installed on the waste processing vessel (120) to maintain the sample holding portion (710, 720) within or adjacent to the waste processing zone (Z).

8. The waste processing apparatus (1000) according to any preceding claim, comprising a plurality of process validation ports (1261, 1262), arranged to receive at least one process validation module or modules (700), the process validation module or modules (700) arranged to retain a plurality of sample holding portions (710, 720) of the process validation modules (700) in fluid communication with the waste processing zone (Z) of the waste processing vessel (120).

9. A method for validating a waste processing and liquid treatment process, the method comprising the steps of:
shredding waste using a blade arrangement (300) in a waste processing vessel (120) in which the waste is shredded and exposed to a waste treatment fluid during a waste processing cycle time; and
exposing a process validation sample (701) comprising a process efficacy indicator to the waste treatment fluid, to which the waste is exposed, during the waste processing cycle time; and
analysing the effect of the waste treatment fluid on the process efficacy indicator to determine an efficacy of the waste processing and liquid treatment process.

10. The method according to claim 9, wherein waste being shredded and the process validation sample (701) are immersed in treatment fluid for the duration of the cycle time.

11. The method according to claim 9 or claim 10, wherein the process efficacy indicator comprises a biological indicator, optionally secured to a substrate, and optionally wherein the substrate comprises paper, glass fibers, a plastic, a ceramic, stainless steel, a metal oxide, or a combination thereof.

12. The method according to claim 11, wherein the biological indicator comprises one or more bacterial spores, a virus, vegetative bacteria, or a combination thereof.

13. The method according to claim 12, wherein the one or more bacterial spores is derived from *Geobacillus* spp., *Alicyclobacillus* spp., *Bacillus* spp., or combinations thereof.

14. The method according to claim 13, wherein the one or more bacterial spores is derived from *Geobacillus stearothermophilus.*

15. The method according to any of claims 11 to 14, wherein the biological indicator is secured to the substrate via an inert adhesive, optionally wherein the inert adhesive comprises a polymer adhesive.

## Claims (Claims for the following Contracting State(s): GB)

1. A waste processing apparatus (1000), comprising:
a waste processing vessel (120) for holding waste and treatment liquid during processing;
a blade arrangement (300) for shredding the waste provided in the waste processing vessel (120);
a waste processing zone (Z) in which the waste can be shredded while immersed in a treatment liquid; and
at least one process validation module (700) arranged such that it can hold a process validation sample (701) in fluid communication with the treatment liquid in the waste processing zone (Z) during operation of the blade arrangement (300) to shred the waste in the waste processing zone (Z) while immersed in the treatment liquid;
wherein the waste processing apparatus is configured to operate at a temperature in a range of about 1°C to about 40°C.

2. The waste processing apparatus (1000) according to claim 1, wherein the at least one process validation module (700) comprises:
a sample holding portion (710, 720) for holding a validation sample (701); and
a fixing portion (730), configured to retain the process validation module (700) to the waste processing vessel (120) to maintain the sample holding portion (710, 720) in fluid communication with the waste processing zone (Z).

3. The waste processing apparatus (1000) according to claim 2, where the sample holding portion (710, 720) is arranged to be detached from the fixing portion (730) and can be reattached to the fixing portion (730) to form a cavity between the sample holding portion (710, 720) and the fixing portion (730) in which a process validation sample (701) can be received.

4. The waste processing apparatus (1000) according to claim 3, further comprising one or more openings (713) through a wall of the cavity of the sample holding portion (710, 720), such that fluid from the waste processing zone (Z) can pass into the cavity, wherein the at least one process validation module (700) preferably comprises a plurality of sample holding portions (710, 720) arranged to be able to hold a plurality of process validation samples (701) in fluid communication with the waste processing zone (Z) of the waste processing apparatus (1000).

5. The waste processing apparatus (1000) according to any of the preceding claims, wherein the waste processing vessel (120) comprises at least one port (1261, 1262) providing a passage through a wall (1220) of the waste processing vessel (120), arranged to facilitate fluid communication between a sample holding portion (710, 720) of the process validation module (700) and the waste processing zone (Z) of the waste processing vessel (120).

6. The waste processing apparatus (1000) according to claim 5, further comprising a fluid passage extending outwardly from the wall (1220) of the waste processing vessel (120) and arranged to facilitate communication of fluid from the waste processing zone (Z) to the sample holding portion (710, 720) of the process validation module (700) and/or to allow at least a portion of the validation module (700) to enter the waste processing zone (Z).

7. The waste processing apparatus (1000) according to any preceding claim, wherein the sample holding portion (710, 720) projects from the fixing portion (730) toward an interior of the waste processing vessel (120) when installed on the waste processing vessel (120) to maintain the sample holding portion (710, 720) within or adjacent to the waste processing zone (Z).

8. The waste processing apparatus (1000) according to any preceding claim, comprising a plurality of process validation ports (1261, 1262), arranged to receive at least one process validation module or modules (700), the process validation module or modules (700) arranged to retain a plurality of sample holding portions (710, 720) of the process validation modules (700) in fluid communication with the waste processing zone (Z) of the waste processing vessel (120).

9. A method for validating a waste processing and liquid treatment process, the method comprising the steps of:
shredding waste using a blade arrangement (300) in a waste processing vessel (120) in which the waste is shredded and immersed in a waste treatment liquid during a waste processing cycle time;
exposing a process validation sample (701) comprising a process efficacy indicator to the waste treatment liquid, to which the waste is immersed, during the waste processing cycle time; and
analysing the effect of the waste treatment liquid on the process efficacy indicator to determine an efficacy of the waste processing and liquid treatment process.

10. The method according to claim 9, wherein the process validation sample (701) is immersed in treatment fluid for the duration of the cycle time.

11. The method according to claim 9 or claim 10, wherein the process efficacy indicator comprises a biological indicator, optionally secured to a substrate, and optionally wherein the substrate comprises paper, glass fibers, a plastic, a ceramic, stainless steel, a metal oxide, or a combination thereof.

12. The method according to claim 11, wherein the biological indicator comprises one or more bacterial spores, a virus, vegetative bacteria, or a combination thereof.

13. The method according to claim 12, wherein the one or more bacterial spores is derived from *Geobacillus* spp., *Alicyclobacillus* spp., *Bacillus* spp., or combinations thereof.

14. The method according to claim 13, wherein the one or more bacterial spores is derived from *Geobacillus stearothermophilus.*

15. The method according to any of claims 11 to 14, wherein the biological indicator is secured to the substrate via an inert adhesive, optionally wherein the inert adhesive comprises a polymer adhesive.

## Revendications (Revendications pour l'(les) Etat(s) contractant(s) suivant(s): AT,BE,CH,DE,DK,ES,FR,GR,IT,LI,LU,NL,SE,MC,PT,IE,SI,LT,LV,FI,RO,MK,CY,AL,TR,BG,CZ,EE,HU,PL,SK,HR,IS,MT,NO,RS,ME,SM)

1. Appareil (1000) de traitement des déchets, comprenant :
une cuve (120) de traitement des déchets destinée à contenir des déchets et un fluide de traitement pendant le traitement ;
un agencement (300) de lames destiné à déchiqueter les déchets fournis dans la cuve (120) de traitement des déchets ;
une zone de traitement des déchets (Z) dans laquelle les déchets peuvent être déchiquetés tout en étant immergés dans un liquide de traitement ; et
au moins un module (700) de validation de processus agencé de manière à pouvoir maintenir un échantillon (701) de validation de processus en communication fluidique avec le liquide de traitement dans la zone de traitement des déchets (Z) pendant le fonctionnement de l'agencement (300) de lames pour déchiqueter les déchets dans la zone de traitement des déchets (Z) tout en étant immergé dans le liquide de traitement.

2. Appareil (1000) de traitement des déchets selon la revendication 1, dans lequel l'au moins un module (700) de validation de processus comprend :
une partie de maintien d'échantillon (710, 720) destinée à maintenir un échantillon (701) de validation ; et
une partie de fixation (730), configurée pour retenir le module (700) de validation de processus sur la cuve (120) de traitement des déchets pour maintenir la partie de maintien d'échantillon (710, 720) en communication fluidique avec la zone de traitement des déchets (Z).

3. Appareil (1000) de traitement des déchets selon la revendication 2, dans lequel la partie de maintien d'échantillon (710, 720) est agencée pour être détachée de la partie de fixation (730) et peut être de nouveau attachée à la partie de fixation (730) pour former une cavité entre la partie de maintien d'échantillon (710, 720) et la partie de fixation (730) dans laquelle un échantillon (701) de validation de processus peut être reçu.

4. Appareil (1000) de traitement des déchets selon la revendication 3, comprenant en outre une ou plusieurs ouvertures (713) à travers une paroi de la cavité de la partie de maintien d'échantillon (710, 720), de sorte qu'un fluide provenant de la zone de traitement des déchets (Z) puisse passer dans la cavité, dans lequel l'au moins un module (700) de validation de processus comprend de préférence une pluralité de parties de maintien d'échantillons (710, 720) agencées pour être aptes à maintenir une pluralité d'échantillons (701) de validation de processus en communication fluidique avec la zone de traitement des déchets (Z) de l'appareil (1000) de traitement des déchets.

5. Appareil (1000) de traitement des déchets selon l'une quelconque des revendications précédentes, dans lequel la cuve (120) de traitement des déchets comprend au moins un port (1261, 1262) fournissant un passage à travers une paroi (1220) de la cuve (120) de traitement des déchets, agencé pour faciliter la communication fluidique entre une partie de maintien d'échantillon (710, 720) du module (700) de validation de processus et la zone de traitement des déchets (Z) de la cuve (120) de traitement des déchets.

6. Appareil (1000) de traitement des déchets selon la revendication 5, comprenant en outre un passage de fluide s'étendant vers l'extérieur à partir de la paroi (1220) de la cuve (120) de traitement des déchets et agencé pour faciliter la communication de fluide de la zone de traitement des déchets (Z) à la partie de maintien d'échantillon (710, 720) du module (700) de validation de processus et/ou pour permettre à au moins une partie du module (700) de validation d'entrer dans la zone de traitement des déchets (Z).

7. Appareil (1000) de traitement des déchets selon une quelconque revendication précédente, dans lequel la partie de maintien d'échantillon (710, 720) fait saillie à partir de la partie de fixation (730) en direction d'un intérieur de la cuve (120) de traitement des déchets lorsqu'elle est installée sur la cuve (120) de traitement des déchets pour maintenir la partie de maintien d'échantillon (710, 720) à l'intérieur de ou adjacente à la zone de traitement des déchets (Z).

8. Appareil (1000) de traitement des déchets selon une quelconque revendication précédente, comprenant une pluralité de ports (1261, 1262) de validation de processus, agencés pour recevoir un ou plusieurs modules (700) de validation de processus, le ou les modules (700) de validation de processus étant agencés pour retenir une pluralité de parties de maintien d'échantillons (710, 720) des modules (700) de validation de processus en communication fluidique avec la zone de traitement des déchets (Z) de la cuve (120) de traitement des déchets.

9. Procédé de validation d'un processus de traitement des déchets et de traitement des liquides, le procédé comprenant les étapes suivantes :
le déchiquetage de déchets à l'aide d'un agencement (300) de lames dans une cuve (120) de traitement des déchets dans laquelle les déchets sont déchiquetés et exposés à un fluide de traitement des déchets pendant une durée de cycle de traitement des déchets ; et
l'exposition d'un échantillon (701) de validation de processus comprenant un indicateur d'efficacité de processus au fluide de traitement des déchets, auquel les déchets sont exposés, pendant la durée du cycle de traitement des déchets ; et
l'analyse de l'effet du fluide de traitement des déchets sur l'indicateur d'efficacité de processus pour déterminer une efficacité du processus de traitement des déchets et de traitement des liquides.

10. Procédé selon la revendication 9, dans lequel les déchets en cours de déchiquetage et l'échantillon (701) de validation de processus sont immergés dans un fluide de traitement pendant la durée du cycle.

11. Procédé selon la revendication 9 ou la revendication 10, dans lequel l'indicateur d'efficacité de processus comprend un indicateur biologique, facultativement joint à un substrat, et facultativement dans lequel le substrat comprend du papier, des fibres de verre, un plastique, une céramique, de l'acier inoxydable, un oxyde métallique, ou une combinaison de ceux-ci.

12. Procédé selon la revendication 11, dans lequel l'indicateur biologique comprend une ou plusieurs spores bactériennes, un virus, des bactéries végétatives, ou une combinaison de ceux-ci.

13. Procédé selon la revendication 12, dans lequel les une ou plusieurs spores bactériennes sont dérivées de *Geobacillus* spp., *Alicyclobacillus* spp., *Bacillus* spp., ou de combinaisons de ceux-ci.

14. Procédé selon la revendication 13, dans lequel les une ou plusieurs bactériennes sont dérivées de *Geobacillus stearothermophilus.*

15. Procédé selon l'une quelconque des revendications 11 à 14, dans lequel l'indicateur biologique est joint au substrat par le biais d'un adhésif inerte, facultativement dans lequel l'adhésif inerte comprend un adhésif polymère.

## Revendications (Revendications pour l'(les) Etat(s) contractant(s) suivant(s): GB)

1. Appareil (1000) de traitement des déchets, comprenant :
une cuve (120) de traitement des déchets destinée à contenir des déchets et un liquide de traitement pendant le traitement ;
un agencement (300) de lames destiné à déchiqueter les déchets fournis dans la cuve (120) de traitement des déchets ;
une zone de traitement des déchets (Z) dans laquelle les déchets peuvent être déchiquetés tout en étant immergés dans un liquide de traitement ; et
au moins un module (700) de validation de processus agencé de manière à pouvoir maintenir un échantillon (701) de validation de processus en communication fluidique avec le liquide de traitement dans la zone de traitement des déchets (Z) pendant le fonctionnement de l'agencement (300) de lames pour déchiqueter les déchets dans la zone de traitement des déchets (Z) tout en étant immergé dans le liquide de traitement ;
dans lequel l'appareil de traitement des déchets est configuré pour fonctionner à une température comprise dans une plage d'environ 1 °C à environ 40 °C.

2. Appareil (1000) de traitement des déchets selon la revendication 1, dans lequel l'au moins un module (700) de validation de processus comprend :
une partie de maintien d'échantillon (710, 720) destinée à maintenir un échantillon (701) de validation ; et
une partie de fixation (730), configurée pour retenir le module (700) de validation de processus sur la cuve (120) de traitement des déchets pour maintenir la partie de maintien d'échantillon (710, 720) en communication fluidique avec la zone de traitement des déchets (Z).

3. Appareil (1000) de traitement des déchets selon la revendication 2, dans lequel la partie de maintien d'échantillon (710, 720) est agencée pour être détachée de la partie de fixation (730) et peut être de nouveau attachée à la partie de fixation (730) pour former une cavité entre la partie de maintien d'échantillon (710, 720) et la partie de fixation (730) dans laquelle un échantillon (701) de validation de processus peut être reçu.

4. Appareil (1000) de traitement des déchets selon la revendication 3, comprenant en outre une ou plusieurs ouvertures (713) à travers une paroi de la cavité de la partie de maintien d'échantillon (710, 720), de sorte qu'un fluide provenant de la zone de traitement des déchets (Z) puisse passer dans la cavité, dans lequel l'au moins un module (700) de validation de processus comprend de préférence une pluralité de parties de maintien d'échantillons (710, 720) agencées pour être aptes à maintenir une pluralité d'échantillons (701) de validation de processus en communication fluidique avec la zone de traitement des déchets (Z) de l'appareil (1000) de traitement des déchets.

5. Appareil (1000) de traitement des déchets selon l'une quelconque des revendications précédentes, dans lequel la cuve (120) de traitement des déchets comprend au moins un port (1261, 1262) fournissant un passage à travers une paroi (1220) de la cuve (120) de traitement des déchets, agencé pour faciliter la communication fluidique entre une partie de maintien d'échantillon (710, 720) du module (700) de validation de processus et la zone de traitement des déchets (Z) de la cuve (120) de traitement des déchets.

6. Appareil (1000) de traitement des déchets selon la revendication 5, comprenant en outre un passage de fluide s'étendant vers l'extérieur à partir de la paroi (1220) de la cuve (120) de traitement des déchets et agencé pour faciliter la communication de fluide de la zone de traitement des déchets (Z) à la partie de maintien d'échantillon (710, 720) du module (700) de validation de processus et/ou pour permettre à au moins une partie du module (700) de validation d'entrer dans la zone de traitement des déchets (Z).

7. Appareil (1000) de traitement des déchets selon une quelconque revendication précédente, dans lequel la partie de maintien d'échantillon (710, 720) fait saillie à partir de la partie de fixation (730) en direction d'un intérieur de la cuve (120) de traitement des déchets lorsqu'elle est installée sur la cuve (120) de traitement des déchets pour maintenir la partie de maintien d'échantillon (710, 720) à l'intérieur de ou adjacente à la zone de traitement des déchets (Z).

8. Appareil (1000) de traitement des déchets selon une quelconque revendication précédente, comprenant une pluralité de ports (1261, 1262) de validation de processus, agencés pour recevoir un ou plusieurs modules (700) de validation de processus, le ou les modules (700) de validation de processus étant agencés pour retenir une pluralité de parties de maintien d'échantillons (710, 720) des modules (700) de validation de processus en communication fluidique avec la zone de traitement des déchets (Z) de la cuve (120) de traitement des déchets.

9. Procédé de validation d'un processus de traitement des déchets et de traitement des liquides, le procédé comprenant les étapes suivantes :
le déchiquetage des déchets à l'aide d'un agencement (300) de lames dans une cuve (120) de traitement des déchets dans laquelle les déchets sont déchiquetés et immergés dans un liquide de traitement des déchets pendant une durée de cycle de traitement des déchets ; et
l'exposition d'un échantillon (701) de validation de processus comprenant un indicateur d'efficacité de processus au fluide de traitement des déchets, dans lequel les déchets sont immergés, pendant le temps de traitement des déchets ; et
l'analyse de l'effet du liquide de traitement des déchets sur l'indicateur d'efficacité de processus pour déterminer une efficacité du processus de traitement des déchets et de traitement des liquides.

10. Procédé selon la revendication 9, dans lequel l'échantillon (701) de validation de processus est immergé dans un fluide de traitement pendant la durée du temps de cycle.

11. Procédé selon la revendication 9 ou la revendication 10, dans lequel l'indicateur d'efficacité de processus comprend un indicateur biologique, facultativement joint à un substrat, et facultativement dans lequel le substrat comprend du papier, des fibres de verre, un plastique, une céramique, de l'acier inoxydable, un oxyde métallique, ou une combinaison de ceux-ci.

12. Procédé selon la revendication 11, dans lequel l'indicateur biologique comprend une ou plusieurs spores bactériennes, un virus, des bactéries végétatives, ou une combinaison de ceux-ci.

13. Procédé selon la revendication 12, dans lequel les une ou plusieurs spores bactériennes sont dérivées de *Geobacillus* spp., *Alicyclobacillus* spp., *Bacillus* spp., ou de combinaisons de ceux-ci.

14. Procédé selon la revendication 13, dans lequel les une ou plusieurs spores bactériennes sont dérivées de *Geobacillus stearothermophilus.*

15. Procédé selon l'une quelconque des revendications 11 à 14, dans lequel l'indicateur biologique est joint au substrat par le biais d'un adhésif inerte, facultativement dans lequel l'adhésif inerte comprend un adhésif polymère.

## Patentansprüche (Patentansprüche für folgende(n) Vertragsstaat(en): AT,BE,CH,DE,DK,ES,FR,GR,IT,LI,LU,NL,SE,MC,PT,IE,SI,LT,LV,FI,RO,MK,CY,AL,TR,BG,CZ,EE,HU,PL,SK,HR,IS,MT,NO,RS,ME,SM)

1. Abfallverarbeitungsvorrichtung (1000), umfassend:
einen Abfallverarbeitungsbehälter (120) zum Halten von Abfall und Behandlungsfluid während der Verarbeitung;
eine Klingenanordnung (300) zum Zerkleinern des im Abfallverarbeitungsbehälter (120) bereitgestellten Abfalls;
eine Abfallverarbeitungszone (Z), in der der Abfall zerkleinert werden kann, während er in eine Behandlungsflüssigkeit eingetaucht ist; und
mindestens ein Prozessvalidierungsmodul (700), das so angeordnet ist, dass es eine Prozessvalidierungsprobe (701) halten kann, die in Fluidverbindung mit der Behandlungsflüssigkeit in der Abfallverarbeitungszone (Z) steht, während des Betriebs der Klingenanordnung (300), um den Abfall in der Abfallverarbeitungszone (Z) zu zerkleinern, während er in die Behandlungsflüssigkeit eingetaucht ist.

2. Abfallverarbeitungsvorrichtung (1000) nach Anspruch 1, wobei das mindestens eine Prozessvalidierungsmodul (700) umfasst:
einen Probenhalteabschnitt (710, 720) zum Halten einer Validierungsprobe (701); und
einen Fixierungsabschnitt (730), der dazu konfiguriert ist, das Prozessvalidierungsmodul (700) an dem Abfallverarbeitungsbehälter (120) zu fixieren, um den Probenhalteabschnitt (710, 720) in Fluidverbindung mit der Abfallverarbeitungszone (Z) zu halten.

3. Abfallverarbeitungsvorrichtung (1000) nach Anspruch 2, wobei der Probenhalteabschnitt (710, 720) dazu angeordnet ist, von dem Fixierungsabschnitt (730) gelöst zu werden und wieder an dem Fixierungsabschnitt (730) angebracht zu werden, um einen Hohlraum zwischen dem Probenhalteabschnitt (710, 720) und dem Fixierungsabschnitt (730) zu bilden, in dem eine Prozessvalidierungsprobe (701) aufgenommen werden kann.

4. Abfallverarbeitungsvorrichtung (1000) nach Anspruch 3, weiter umfassend eine oder mehrere Öffnungen (713) durch eine Wand des Hohlraums des Probenhalteabschnitts (710, 720), sodass Fluid von der Abfallverarbeitungszone (Z) in den Hohlraum übergehen kann, wobei das mindestens eine Prozessvalidierungsmodul (700) vorzugsweise eine Vielzahl von Probenhalteabschnitten (710, 720) umfasst, die dazu angeordnet sind, in der Lage zu sein, eine Vielzahl von Prozessvalidierungsproben (701) in Fluidverbindung mit der Abfallverarbeitungszone (Z) der Abfallverarbeitungsvorrichtung (1000) zu halten.

5. Abfallverarbeitungsvorrichtung (1000) nach einem der vorstehenden Ansprüche, wobei der Abfallverarbeitungsbehälter (120) mindestens einen Anschluss (1261, 1262) umfasst, der einen Durchgang durch eine Wand (1220) des Abfallverarbeitungsbehälters (120) bereitstellt, der dazu angeordnet ist, eine Fluidverbindung zwischen einem Probenhalteabschnitt (710, 720) des Prozessvalidierungsmoduls (700) und der Abfallverarbeitungszone (Z) des Abfallverarbeitungsbehälters (120) zu erleichtern.

6. Abfallverarbeitungsvorrichtung (1000) nach Anspruch 5, weiter umfassend einen Fluiddurchgang, der sich von der Wand (1220) des Abfallverarbeitungsbehälters (120) nach außen erstreckt und dazu angeordnet ist, eine Fluidverbindung von der Abfallverarbeitungszone (Z) zu dem Probenhalteabschnitt (710, 720) des Prozessvalidierungsmoduls (700) zu erleichtern und/oder mindestens einem Abschnitt des Validierungsmoduls (700) zu ermöglichen, in die Abfallverarbeitungszone (Z) einzutreten.

7. Abfallverarbeitungsvorrichtung (1000) nach einem vorstehenden Anspruch, wobei der Probenhalteabschnitt (710, 720) von dem Fixierungsabschnitt (730) in Richtung eines Inneren des Abfallverarbeitungsbehälters (120) vorsteht, wenn er an dem Abfallverarbeitungsbehälter (120) installiert ist, um den Probenhalteabschnitt (710, 720) innerhalb oder benachbart zu der Abfallverarbeitungszone (Z) zu halten.

8. Abfallverarbeitungsvorrichtung (1000) nach einem vorstehenden Anspruch, umfassend eine Vielzahl von Prozessvalidierungsanschlüssen (1261, 1262), die dazu angeordnet sind, mindestens ein Prozessvalidierungsmodul oder Prozessvalidierungsmodule (700) aufzunehmen, wobei das Prozessvalidierungsmodul oder die Prozessvalidierungsmodule (700) dazu angeordnet sind, eine Vielzahl von Probenhalteabschnitten (710, 720) der Prozessvalidierungsmodule (700) in Fluidverbindung mit der Abfallverarbeitungszone (Z) des Abfallverarbeitungsbehälters (120) zu halten.

9. Verfahren zum Validieren eines Abfallverarbeitungs- und Abfallflüssigkeitsbehandlungsprozesses, das Verfahren umfassend die Schritte von:
Zerkleinern von Abfall unter Verwendung einer Klingenanordnung (300) in einem Abfallverarbeitungsbehälter (120), in dem der Abfall zerkleinert wird und während einer Abfallverarbeitungszykluszeit einem Abfallbehandlungsfluid ausgesetzt wird; und
Aussetzen einer Prozessvalidierungsprobe (701), die einen Prozesswirksamkeitsindikator umfasst, während der Abfallverarbeitungszykluszeit dem Abfallbehandlungsfluid, dem der Abfall ausgesetzt wird; und
Analysieren der Wirkung des Abfallbehandlungsfluids auf den Prozesswirksamkeitsindikator, um eine Wirksamkeit des Abfallverarbeitungs- und Abfallflüssigkeitsbehandlungsprozesses zu bestimmen.

10. Verfahren nach Anspruch 9, wobei der Abfall, der zerkleinert wird, und die Prozessvalidierungsprobe (701) für die Dauer der Zykluszeit in das Behandlungsfluid eingetaucht werden.

11. Verfahren nach Anspruch 9 oder Anspruch 10, wobei der Prozesswirksamkeitsindikator einen biologischen Indikator umfasst, der optional an einem Substrat gesichert ist, und optional, wobei das Substrat Papier, Glasfasern, einen Kunststoff, eine Keramik, rostfreien Stahl, ein Metalloxid oder eine Kombination davon umfasst.

12. Verfahren nach Anspruch 11, wobei der biologische Indikator eine oder mehrere Bakteriensporen, ein Virus, vegetative Bakterien oder eine Kombination davon umfasst.

13. Verfahren nach Anspruch 12, wobei die eine oder die mehreren Bakteriensporen von *Geobacillus* spp., *Alicyclobacillus* spp., *Bacillus* spp. oder Kombinationen davon abgeleitet sind.

14. Verfahren nach Anspruch 13, wobei die eine oder die mehreren Bakteriensporen von *Geobacillus stearothermophilus* abgeleitet sind.

15. Verfahren nach einem der Ansprüche 11 bis 14, wobei der biologische Indikator über einen inerten Klebstoff an dem Substrat gesichert ist, optional, wobei der inerte Klebstoff einen Polymerklebstoff umfasst.

## Patentansprüche (Patentansprüche für folgende(n) Vertragsstaat(en): GB)

1. Abfallverarbeitungsvorrichtung (1000), umfassend:
einen Abfallverarbeitungsbehälter (120) zum Halten von Abfall und Behandlungsflüssigkeit während der Verarbeitung;
eine Klingenanordnung (300) zum Zerkleinern des im Abfallverarbeitungsbehälter (120) bereitgestellten Abfalls;
eine Abfallverarbeitungszone (Z), in der der Abfall zerkleinert werden kann, während er in eine Behandlungsflüssigkeit eingetaucht ist; und
mindestens ein Prozessvalidierungsmodul (700), das so angeordnet ist, dass es eine Prozessvalidierungsprobe (701) halten kann, die in Fluidverbindung mit der Behandlungsflüssigkeit in der Abfallverarbeitungszone (Z) steht, während des Betriebs der Klingenanordnung (300), um den Abfall in der Abfallverarbeitungszone (Z) zu zerkleinern, während er in die Behandlungsflüssigkeit eingetaucht ist;
wobei die Abfallverarbeitungsvorrichtung dazu konfiguriert ist, bei einer Temperatur in einem Bereich von etwa 1 °C bis etwa 40 °C zu arbeiten.

2. Abfallverarbeitungsvorrichtung (1000) nach Anspruch 1, wobei das mindestens eine Prozessvalidierungsmodul (700) umfasst:
einen Probenhalteabschnitt (710, 720) zum Halten einer Validierungsprobe (701); und
einen Fixierungsabschnitt (730), der dazu konfiguriert ist, das Prozessvalidierungsmodul (700) an dem Abfallverarbeitungsbehälter (120) zu fixieren, um den Probenhalteabschnitt (710, 720) in Fluidverbindung mit der Abfallverarbeitungszone (Z) zu halten.

3. Abfallverarbeitungsvorrichtung (1000) nach Anspruch 2, wobei der Probenhalteabschnitt (710, 720) dazu angeordnet ist, von dem Fixierungsabschnitt (730) gelöst zu werden und wieder an dem Fixierungsabschnitt (730) angebracht zu werden, um einen Hohlraum zwischen dem Probenhalteabschnitt (710, 720) und dem Fixierungsabschnitt (730) zu bilden, in dem eine Prozessvalidierungsprobe (701) aufgenommen werden kann.

4. Abfallverarbeitungsvorrichtung (1000) nach Anspruch 3, weiter umfassend eine oder mehrere Öffnungen (713) durch eine Wand des Hohlraums des Probenhalteabschnitts (710, 720), sodass Fluid von der Abfallverarbeitungszone (Z) in den Hohlraum übergehen kann, wobei das mindestens eine Prozessvalidierungsmodul (700) vorzugsweise eine Vielzahl von Probenhalteabschnitten (710, 720) umfasst, die dazu angeordnet sind, in der Lage zu sein, eine Vielzahl von Prozessvalidierungsproben (701) in Fluidverbindung mit der Abfallverarbeitungszone (Z) der Abfallverarbeitungsvorrichtung (1000) zu halten.

5. Abfallverarbeitungsvorrichtung (1000) nach einem der vorstehenden Ansprüche, wobei der Abfallverarbeitungsbehälter (120) mindestens einen Anschluss (1261, 1262) umfasst, der einen Durchgang durch eine Wand (1220) des Abfallverarbeitungsbehälters (120) bereitstellt, der dazu angeordnet ist, eine Fluidverbindung zwischen einem Probenhalteabschnitt (710, 720) des Prozessvalidierungsmoduls (700) und der Abfallverarbeitungszone (Z) des Abfallverarbeitungsbehälters (120) zu erleichtern.

6. Abfallverarbeitungsvorrichtung (1000) nach Anspruch 5, weiter umfassend einen Fluiddurchgang, der sich von der Wand (1220) des Abfallverarbeitungsbehälters (120) nach außen erstreckt und dazu angeordnet ist, eine Fluidverbindung von der Abfallverarbeitungszone (Z) zu dem Probenhalteabschnitt (710, 720) des Prozessvalidierungsmoduls (700) zu erleichtern und/oder mindestens einem Abschnitt des Validierungsmoduls (700) zu ermöglichen, in die Abfallverarbeitungszone (Z) einzutreten.

7. Abfallverarbeitungsvorrichtung (1000) nach einem vorstehenden Anspruch, wobei der Probenhalteabschnitt (710, 720) von dem Fixierungsabschnitt (730) in Richtung eines Inneren des Abfallverarbeitungsbehälters (120) vorsteht, wenn er an dem Abfallverarbeitungsbehälter (120) installiert ist, um den Probenhalteabschnitt (710, 720) innerhalb oder benachbart zu der Abfallverarbeitungszone (Z) zu halten.

8. Abfallverarbeitungsvorrichtung (1000) nach einem vorstehenden Anspruch, umfassend eine Vielzahl von Prozessvalidierungsanschlüssen (1261, 1262), die dazu angeordnet sind, mindestens ein Prozessvalidierungsmodul oder Prozessvalidierungsmodule (700) aufzunehmen, wobei das Prozessvalidierungsmodul oder die Prozessvalidierungsmodule (700) dazu angeordnet sind, eine Vielzahl von Probenhalteabschnitten (710, 720) der Prozessvalidierungsmodule (700) in Fluidverbindung mit der Abfallverarbeitungszone (Z) des Abfallverarbeitungsbehälters (120) zu halten.

9. Verfahren zum Validieren eines Abfallverarbeitungs- und Abfallflüssigkeitsbehandlungsprozesses, das Verfahren umfassend die Schritte von:
Zerkleinern von Abfall unter Verwendung einer Klingenanordnung (300) in einem Abfallverarbeitungsbehälter (120), in dem der Abfall zerkleinert wird und während einer Abfallverarbeitungszykluszeit in eine Abfallbehandlungsflüssigkeit eingetaucht wird;
Aussetzen einer Prozessvalidierungsprobe (701), die einen Prozesswirksamkeitsindikator umfasst, während der Abfallverarbeitungszykluszeit der Abfallbehandlungsflüssigkeit, in die der Abfall eingetaucht wird; und
Analysieren der Wirkung der Abfallbehandlungsflüssigkeit auf den Prozesswirksamkeitsindikator, um eine Wirksamkeit des Abfallverarbeitungs- und Abfallflüssigkeitsbehandlungsprozesses zu bestimmen.

10. Verfahren nach Anspruch 9, wobei die Prozessvalidierungsprobe (701) für die Dauer der Zykluszeit in das Behandlungsfluid eingetaucht wird.

11. Verfahren nach Anspruch 9 oder Anspruch 10, wobei der Prozesswirksamkeitsindikator einen biologischen Indikator umfasst, der optional an einem Substrat gesichert ist, und optional, wobei das Substrat Papier, Glasfasern, einen Kunststoff, eine Keramik, rostfreien Stahl, ein Metalloxid oder eine Kombination davon umfasst.

12. Verfahren nach Anspruch 11, wobei der biologische Indikator eine oder mehrere Bakteriensporen, ein Virus, vegetative Bakterien oder eine Kombination davon umfasst.

13. Verfahren nach Anspruch 12, wobei die eine oder die mehreren Bakteriensporen von *Geobacillus* spp., *Alicyclobacillus* spp., *Bacillus* spp. oder Kombinationen davon abgeleitet sind.

14. Verfahren nach Anspruch 13, wobei die eine oder die mehreren Bakteriensporen von *Geobacillus stearothermophilus* abgeleitet sind.

15. Verfahren nach einem der Ansprüche 11 bis 14, wobei der biologische Indikator über einen inerten Klebstoff an dem Substrat gesichert ist, optional, wobei der inerte Klebstoff einen Polymerklebstoff umfasst.
